# EUROPEAN PATENT SPECIFICATION

(11) **EP 4 021 575 B1**
(45) Date of publication and mention of the grant of the patent: **22.05.2024**
(21) Application number: 20771408.0
(22) Date of filing: 31.08.2020
(51) Int. Cl.: A61K 38/46, A61K 9/00, A61K 9/06, A61K 9/08, A61K 9/19, A61K 38/48, A61K 45/06, A61K 47/02, A61P 25/28

(54) **METHODS FOR TREATING CLN2 DISEASE IN PEDIATRIC SUBJECTS**
VERFAHREN ZUR BEHANDLUNG DER CLN2-KRANKHEIT BEI PÄDIATRISCHEN SUBJEKTEN
MÉTHODES DE TRAITEMENT D'UNE MALADIE DE CLN2 CHEZ DES SUJETS PÉDIATRIQUES

(30) Priority: 29.08.2019 US 201962893535 P
(43) Date of publication of application: 06.07.2022
(73) Proprietor: BioMarin Pharmaceutical Inc., Novato, CA 94949 (US)
(72) Inventor: JACOBY, David, Novato, CA 94949 (US); HENSHAW, Joshua, Novato, CA 94949 (US)
(74) Representative: D Young & Co LLP
(86) International application number: PCT/US2020/048704
(87) International publication number: WO 2021/042020

(56) References cited:
- WO-A1-2017/070678
- WO-A1-2020/102369
- US-A1- 2012 014 935
- US-A1- 2019 216 905
- Biomarin Pharmacueticals ET AL: "BioMarin Announces Ongoing Study Demonstrates Durable Treatment Benefit from Brineura (cerliponase alfa) for 3 Years Reduced Rate of Decline Maintained in Children with CLN2 Disease, a Form of Batten Disease", , 7 February 2019 (2019-02-07), XP055744634, Retrieved from the Internet: URL:https://investors.biomarin.com/2019-02 -07-BioMarin-Announces-Ongoing-Study-Demon strates-Durable-Treatment-Benefit-from-Bri neura-R-cerliponase-alfa-for-3-Years [retrieved on 2020-10-28]
- Biomarin Pharmaceuticals: "ANNEX I SUMMARY OF PRODUCT CHARACTERISTICS", , 30 May 2017 (2017-05-30), XP055744833, Retrieved from the Internet: URL:https://www.ema.europa.eu/en/documents /product-information/brineura-epar-product -information_en.pdf [retrieved on 2020-10-28]
- ALFRIED KOHLSCHÜTTER ET AL: "Current and Emerging Treatment Strategies for Neuronal Ceroid Lipofuscinoses", CNS DRUGS, vol. 33, no. 4, 1 April 2019 (2019-04-01), pages 315-325, XP055694540, AUCKLAND, NZ ISSN: 1172-7047, DOI: 10.1007/s40263-019-00620-8
- Schulz Angela ET AL: "Cerliponase alfa for the treatment of CLN2 disease in an expanded patient cohort including children younger than three years: Interim results from an ongoing clinical study", Molecular Genetics and Metabolism, vol. 129, no. 2, 1 February 2020 (2020-02-01), page S145, XP093105682, AMSTERDAM, NL ISSN: 1096-7192, DOI: 10.1016/j.ymgme.2019.11.383

## Description

### FIELD OF THE INVENTION

The present disclosure relates to methods of treating Neuronal Ceroid Lipofuscinosis (CLN2) disease in a subject less than 3 years old and methods of delaying the onset of CLN2 or an associated physiological symptom thereof.

### BACKGROUND OF THE INVENTION

Neuronal Ceroid Lipofuscinosis (CLN2) disease is a rare genetic disease characterized by a deficiency of the lysosomal enzyme tripeptidyl peptidase-1 (TPP1) caused by mutations in the *TPP1* gene. CLN2 disease is inherited as an autosomal recessive disorder, with an estimated incidence of 0.5 per 100,000 live births. In the absence of TPP1, lysosomal storage materials normally metabolized by the enzyme accumulate in many organs, and accumulation in the central nervous system leads to the neurodegenerative symptoms typical of CLN2 disease. The untreated disease progression of CLN2 disease has been well characterized, and the natural history of the disease is remarkably consistent and predictable, as demonstrated by natural history data from independent patient populations in North America and Europe.

CLN2 disease has a predominantly 'classic' late infantile phenotype. Children with CLN2 disease typically develop normally until about 3 years of age, when first symptoms emerge. Most commonly, CLN2 patients will have a first unprovoked seizure and begin to lag with acquiring normal language milestones. By age 3, all patients exhibit one or more signs of the disease, including for example, seizures, dementia, motor loss, movement disorder, blindness, clumsiness, ataxia and cognitive decline. From the onset of clinical symptoms, the course of the disease is rapid and aggressive, generally resulting in complete loss of language, cognition, gait, fine motor, bulbar function and vision within 2 to 4 years, rendering patients immobile, mute and blind. The patient remains in a vegetative state until death, which typically occurs between 6 and 12 years of age.

Two quantitative rating scales have been developed by expert clinicians in assessing the severity of CLN2 disease, and been employed in natural history studies: (1) the Hamburg scale (Steinfeld et al., Am J Med Genet. 2002; 112(4):347-54); and (2) the Weill Cornell Medical College (WCMC) scale (Worgall et al., Neurology. 2007; 69(6):521-35). The structure and assessment methodology of the two scales is similar. Both scales measure the loss of previously attained important neurological milestones in CLN2 patients, with each unit lost in the disease rating scale representing a fundamental milestone in progressive decline.

Analysis of disease course in untreated CLN2-affected children shows that after the onset of disease, they predictably lose all language and gait in 3 years, with a loss of, on the average, 2.1 milestone events (i.e., 2.1 points lost in the disease rating scale) each year. Language decline usually precedes gait, such that the first year is characterized by loss of intelligible speech and progression to ataxic gait, the second year is characterized by loss of ambulation and intelligible language, and the third year is characterized by loss of any locomotion or communication.

Recombinant human tripeptidyl peptidase-1 (rhTPP1) is being developed as a possible treatment for CLN2 disease. The rhTPP1 protein is produced in cell culture as a zymogen (proenzyme), which does not have enzymatic activity. The proenzyme is auto-activated at acidic pH (and by lysosomal proteases) upon uptake to the lysosome. The mature native TPP1 protein is a lysosomal serine protease, and is the only known mammalian member of the sedolisin (serine-carboxyl peptidase) family characterized by a highly conserved Ser-Glu-Asp (SED) catalytic triad. The catalytic triad on rhTPP 1 is formed by S456, E253 and D341. The primary activity of the enzyme is as a tripeptidyl exopeptidase with a broad substrate specificity. Activity of the enzyme on its substrate leads to a sequential release of tripeptides from the N-terminus of the protein substrate (Oyama et al., J Biochem. 2005; 138(2): 127-34). A secondary, significantly weaker endoproteolytic activity with a pH optimum of 3 has also been reported (Lin et al., J Biol Chem. 2001; 276(3):2249-55).

The only commercially available treatments for CLN2 are symptomatic and supportive; there are currently no approved therapeutic options to slow or halt the inexorable progression of CLN2, much less reverse the deleterious effects of the disease (Mole, S.E., and Williams, R.E., 2010, GeneReviews; Chang et al., in The Neuronal Ceroid Lipofuscinoses (Batten Disease); 2011, Oxford Univ Press). Preservation of motor, language and/or vision capabilities for these children would be a clinically meaningful benefit for both patients and parents/caregivers. Thus, there is a need for new treatments for CLN2 which reduce or prevent deterioration of physiological functions associated with the disease.

### SUMMARY OF THE INVENTION

The present disclosure is directed to a composition comprising a formulation comprising recombinant human tripeptidyl peptidase-1 (rhTPP1) for use in a method of treating Neuronal Ceroid Lipofuscinosis (CLN2) disease in a subject less than 3 years old, wherein a dosage of about 300 mg or less is administered intracerebroventricularly, intrathecally, or intraocularly to the subject once every 2 weeks. In exemplary embodiments, the method comprises administering to the subject a formulation comprising recombinant human tripeptidyl peptidase-1 (rhTPP1) in an amount effective to treat the CLN2 disease in the subject. The present disclosure is also directed to a composition comprising recombinant human tripeptidyl peptidase-1 (rhTPP1) for use in a method of delaying the onset of Neuronal Ceroid Lipofuscinosis (CLN2) disease, or a symptom thereof, in a subject less than 3 years old, wherein a dosage of about 300 mg or less is administered intracerebroventricularly, intrathecally, or intraocularly to the subject once every 2 weeks. In exemplary embodiments, the method comprises administering to the subject a formulation comprising rhTPP1 in an amount effective to delay the onset of the CLN2 disease or symptom thereof in the subject. In various aspects, the formulation is administered by infusion at a rate of about 2.5 mL per hour. In exemplary aspects, the subject is greater than or about 2 years old, and, optionally, a dosage of about 300 mg rhTPP1 is administered to the subject. In various aspects, the subject is greater than or about 1 year old and less than 2 years old, and optionally, a dosage of about 200 mg rhTPP1 is administered to the subject. In exemplary aspects, each of the 1^{st}, 2^{nd}, 3^{rd} and 4^{th} dosages administered to the subject (e.g., who is greater than or about 1 year old and less than 2 years) is about 200 mg rhTPP1 and each of the 5^{th} and subsequent dosages administered to the subject is greater than about 200 mg rhTPP1. In exemplary instances, each of the 5^{th} and subsequent dosages administered to the subject is about 300 mg rhTPP1. In various aspects, the subject is greater than or about 6 months old and less than 1 year old, and, optionally, a dosage of about 150 mg rhTPP1 is administered to the subject. In various aspects, the subject is less than 6 months old, and, optionally, a dosage of about 100 mg rhTPP1 is administered to the subject. In exemplary aspects, the subject exhibits a decrease in TPP1 enzyme activity based on a blood test. In exemplary instances, the subject is a sibling of an individual diagnosed with CLN2. In exemplary aspects, the subject has a total score on the motor and language subscales of about 3 to about 6 points. In exemplary aspects, the subject has no prior treatment with a stem cell therapy, gene therapy, or enzyme supplementation therapy. In exemplary instances, the method comprises administering to the subject an antihistamine with or without an antipyretic before administration of the rhTPP1, optionally, about 30 to about 60 minutes before administration of the rhTPP1. In various aspects, the formulation comprises the rhTPP1 and at least one pharmaceutically acceptably carrier, diluent or excipient. In various instance, the formulation comprises disodium hydrogen phosphate pentahydrate, monosodium phosphate monohydrate, sodium chloride, potassium chloride, magnesium chloride, calcium chloride hydrate, water for injection, or a combination thereof. In various instances, the method comprises administering to the subject a flush solution after administering the formulation. In various aspects, the flush solution comprises disodium hydrogen phosphate pentahydrate, monosodium phosphate monohydrate, sodium chloride, potassium chloride, magnesium chloride, calcium chloride hydrate, water for injection, or a combination thereof. In various aspects, the treatment period is at least 10 weeks, at least 20 weeks, at least 40 week, at least 80 weeks, or at least 96 weeks.

The foregoing summary is not intended to define every aspect of the invention, and other features and advantages of the present disclosure will become apparent from the following detailed description, including the drawings. The present disclosure is intended to be related as a unified document, and it should be understood that all combinations of features described herein are contemplated, even if the combination of features are not found together in the same sentence, paragraph, or section of this disclosure. In addition, the disclosure includes, as an additional aspect, all embodiments of the invention narrower in scope in any way than the variations specifically mentioned above. With respect to aspects of the disclosure described or claimed with "a" or "an," it should be understood that these terms mean "one or more" unless context unambiguously requires a more restricted meaning. With respect to elements described as one or more within a set, it should be understood that all combinations within the set are contemplated. If aspects of the disclosure are described as "comprising" a feature, embodiments also are contemplated "consisting of" or "consisting essentially of' the feature. Additional features and variations of the disclosure will be apparent to those skilled in the art from the entirety of this application, and all such features are intended as aspects of the disclosure. The references to the methods of treatment by therapy or surgery of this description are to be interpreted as references to compositions, formulations, or doses comprising rhTPP 1 for use in those methods.

### BRIEF DESCRIPTION OF THE DRAWINGS

**Figure 1** depicts the amino acid sequence of rhTPP1 zymogen, lacking the associated signal peptide. The pro-segment of the enzyme is the first 176 amino acid residues, and the mature enzyme is 368 amino acids in length starting from position 177.
**Figure 2** depicts the clinical progression of untreated subjects having CLN2 disease in the natural history study and shows the 0 to 6 Hamburg motor and language composite score as a function of patient age. The median, quartile and 10%/90% distributions; in addition to the mean and 95% confidence interval, are shown.
**Figures 3A to 3F** depict the clinical assessments of 24 patients accrued over the treatment duration and show the 0 to 6 Hamburg motor and language composite score. Open circles represent CLN2 scores obtained on or before the first 300 mg infusion of rhTPP 1, while closed circles represent CLN2 scores obtained after the first 300 mg infusion of rhTPP1. Both the aggregate score (circles) and the contribution of motor/gait (squares) and language (triangles) to the aggregate score are shown. Analysis Day 1 is the date of the first infusion.
**Figures 4A to 4I** compare the change in CLN2 score from 9 patients treated with rhTPP1 to untreated natural history patients matched to the treated subjects by disease rating score (denoted with the prefix "HAM") on the 0 to 6 Hamburg motor and language aggregate scale. Results for the treated patients are shown with a solid line in each panel compared to results for matched, untreated natural history patients, which are shown with a broken line.
**Figure 5** depicts the distribution of clinical change from baseline for matched, untreated natural history patients (circles) for the treatment duration of the patient match compared to the study subjects (squares).
**Figures 6A** to **6I** depict the change in CLN2 score from 9 patients treated with rhTPP1 to untreated natural history patients matched by disease rating score on the 0 to 9 Hamburg motor/language/vision aggregate scale. Results for the treated patients are shown with a solid line in each panel compared to results for matched, untreated natural history patients, which are shown with a broken line.
**Figure 7** depicts the volume (top panel) and proportion (bottom panel) of cerebrospinal fluid for all 24 patients measured over the treatment duration. Each line represents one patient.
**Figures 8A** to **8L** depict the brain volume of 24 treated patients. The volumes (top panel) and proportions (bottom panel) of white matter are shown as the difference between whole brain volume (dash-dot line) and CSF and gray matter (dashed line) and of gray matter as the difference between CSF and gray matter (dashed line) and CSF (solid line).
**Figures 9A** and **9B** depict the average change in CLN2 score for patients treated with rhTPP 1 and untreated natural history patients. **Figure 9A** depicts the CLN2 score for 23 patients treated with 300 mg rhTPP1 for 48 weeks (dashed line) and an untreated natural history cohort of 41 subjects (solid line). **Figure 9B** depicts the change in CLN2 score from baseline for 23 patients treated with 300 mg rhTPP 1 for 48 weeks.

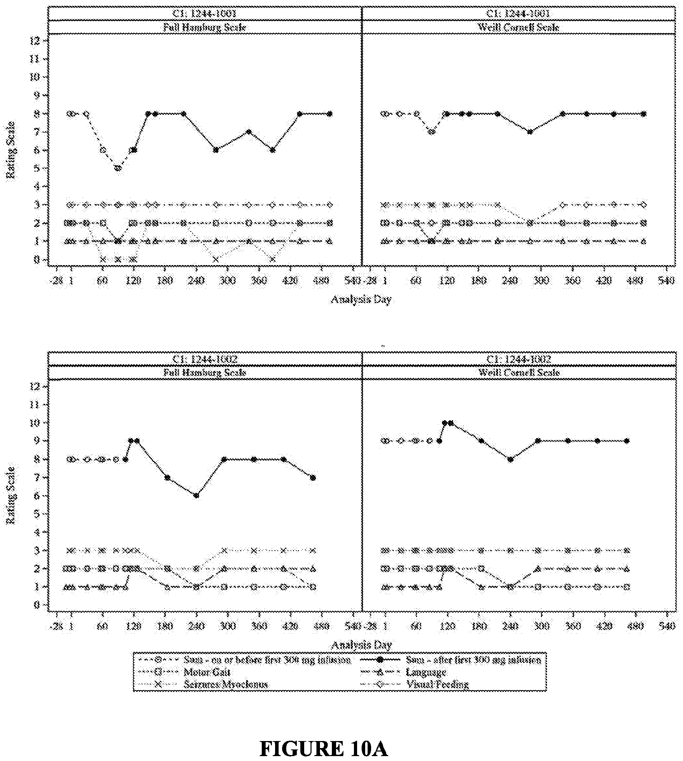

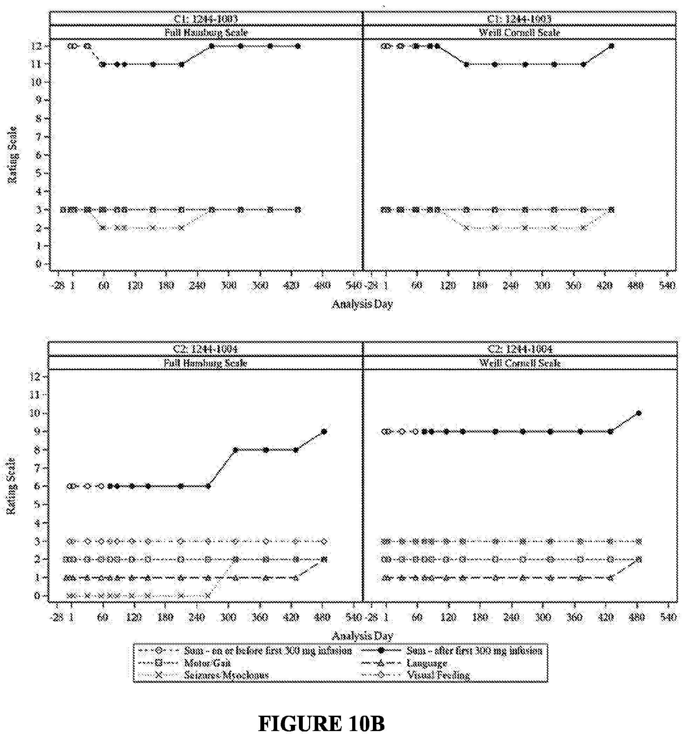

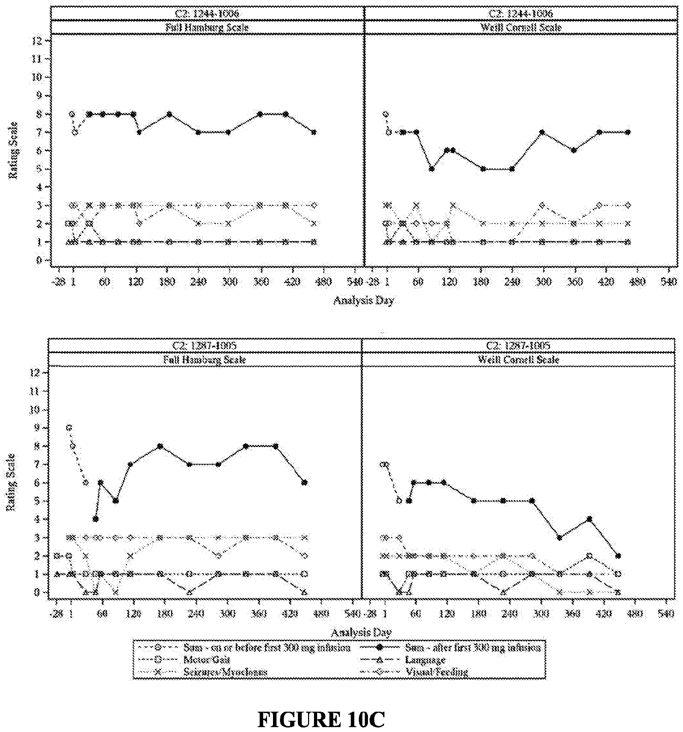

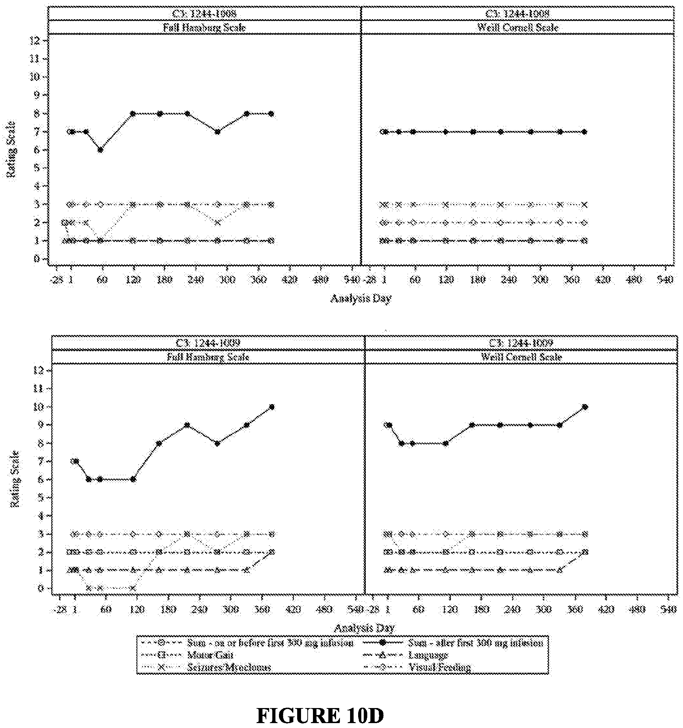

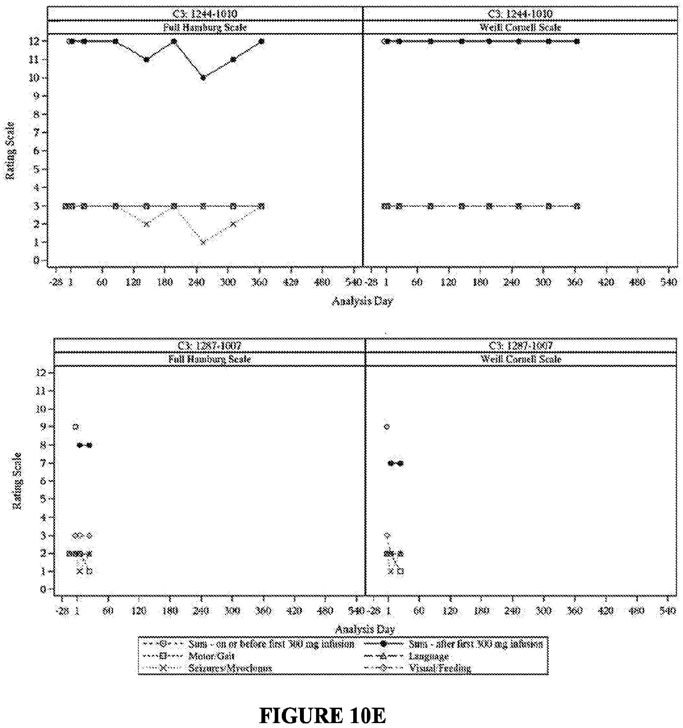

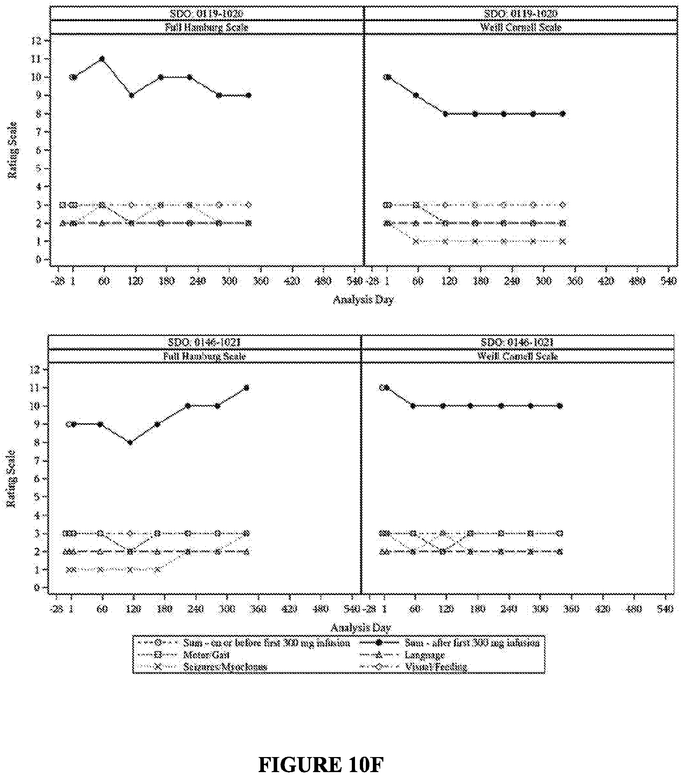

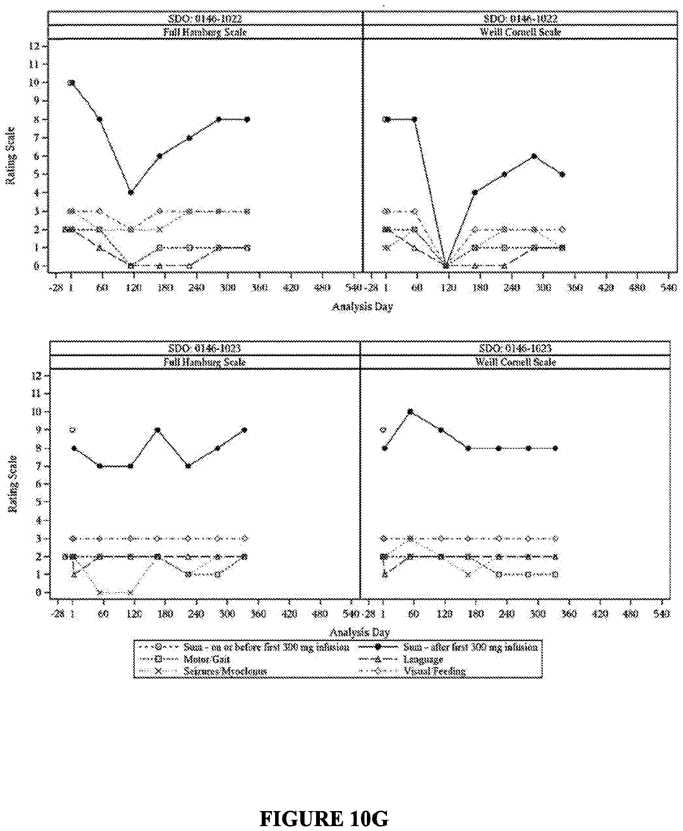

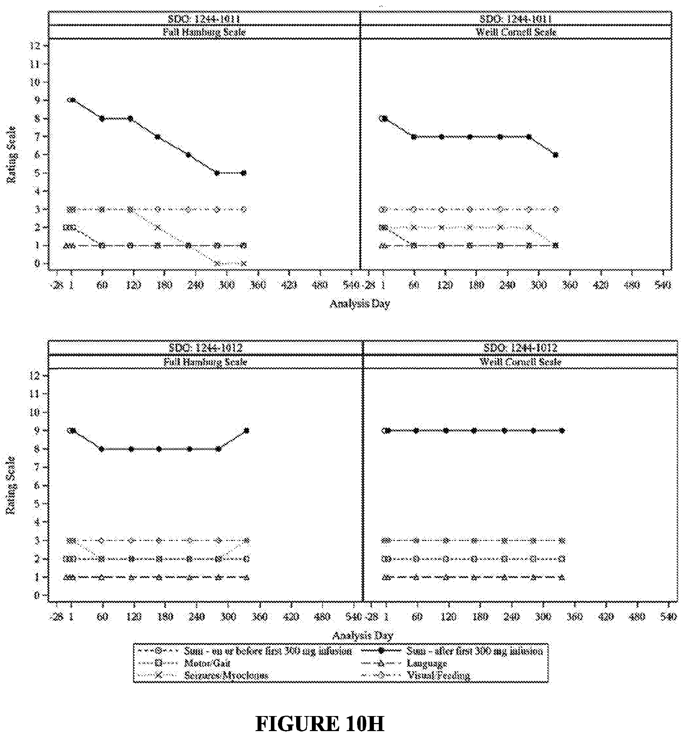

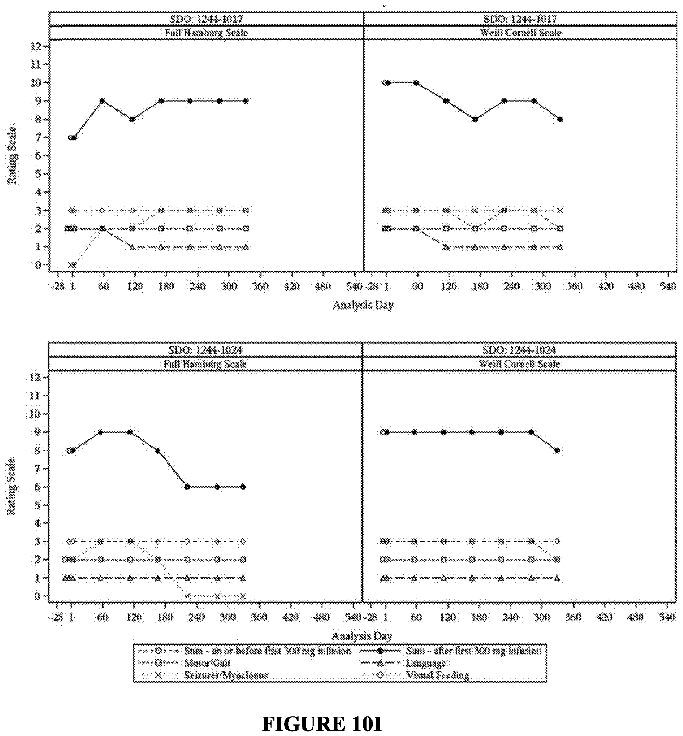

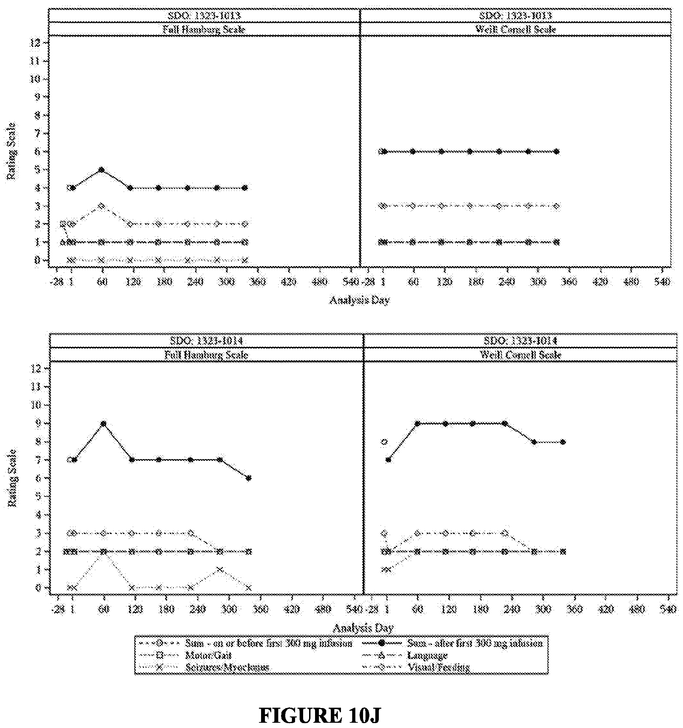

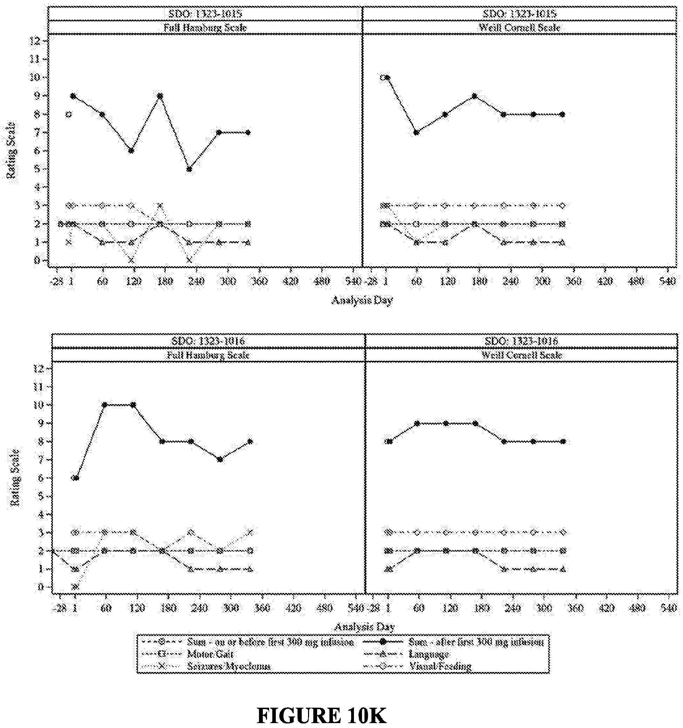

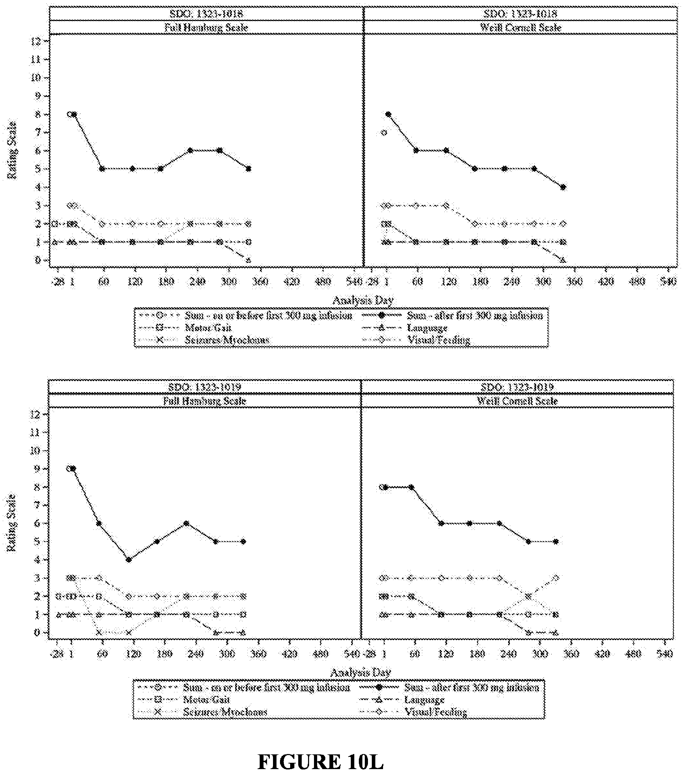

**Figures 10A** to **10L** depict the clinical assessments of 24 patients accrued over the treatment duration and show the 0 to 12 combined Hamburg (left panel) motor (squares), language (triangles), seizures (crosses), and visual composite score (diamonds) and the 0 to 12 combined WCMC (right panel) gait (squares), language (triangles), myoclonus (crosses), and feeding (diamonds) composite score. Open circles represent aggregate CLN2 scores obtained on or before the first 300 mg infusion of rhTPP 1, while closed circles represent aggregate CLN2 scores obtained after the first 300 mg infusion of rhTPP 1.
**Figure 11** shows a table of clinical laboratory assessments and events tested in the study described in Examples 4-10.
**Figure 12** shows a table listing the Hamburg LINCL Scale.
**Figure 13** shows median (range) PK parameters for cerliponase alfa following single doses of 30, 100, and 300 mg by ICV infusion.
**Figure 14** shows median (range) PK parameters for cerliponase alfa following 300 mg QOW by ICV infusion.
**Figure 15** shows mean concentration-time profile of cerliponase alfa in CSF and plasma following 300 mg QOW by ICV infusion. Time 0 represents the start of infusion. SD=stable dose phase.
**Figure 16** shows patient- and visit-matched CSF versus plasma exposure of cerliponase alfa at 300 mg ICV QOW.
**Figures 17A****-17D** show patient characteristics and cerliponase alfa PK in CSF and plasma. Individual patient data shown as circles. Figure 17A, gender; Figure 17B, baseline age; Figure 17C, baseline bodyweight; Figure 17D, baseline CLN2 score. Box represents the interquartile range (IQR) between first (Q1) and third (Q3) quartiles, bar within the box represents the median, and whiskers represent the minimum and maximum values excluding outliers (i.e., values outside the standard span of data defined as the range from Q1-1.5*IQR to Q3+1.5*IQR; open circles).
**Figures 18A-18B** show individual visit-matched PK of cerliponase alfa and ADA status. Figure 18a, CSF; Figure 18b, plasma/serum. Open circles shown for patients with negative ADA response at study visit and closed circles for patients with positive ADA response at study visit.
**Figure 19** shows change from baseline in the combined score for motor-language function of the CLN2 Clinical Rating Scale and cerliponase alfa PK in CSF. Individual patient data shown as circles. Box represents the interquartile range (IQR) between first (Q1) and third (Q3) quartiles, bar within the box represents the median, and whiskers represent the minimum and maximum values excluding outliers (i.e., values outside the standard span of data defined as the range from Q1-1.5*IQR to Q3+1.5*IQR; open circles).

### DETAILED DESCRIPTION OF THE INVENTION

The following definitions may be useful in aiding the skilled practitioner in understanding the disclosure. Unless otherwise defined herein, scientific and technical terms used in the present disclosure shall have the meanings that are commonly understood by those of ordinary skill in the art. Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges, subject to any specifically excluded limit in the stated range.

The term "family history" refers to a subject having a blood relative diagnosed with CLN2 disease, e.g., a sibling, parent, grandparent, great-grandparent, etc.

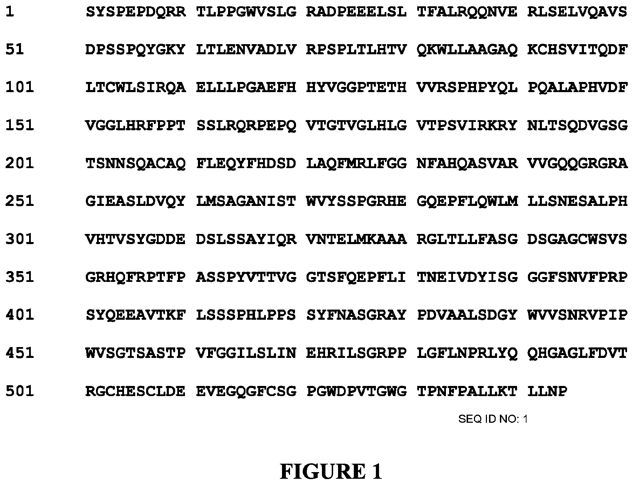

The term "fragment" refers, in one aspect, to a recombinant protein comprising a portion of the rhTPP1 proenzyme amino acid sequence set forth in SEQ ID NO:1 and Figure 1. For example, a fragment may contain at least about 60%, at least about 70%, at least about 80%, at least about 90%, or at least about 95% of the amino acid sequence set forth in SEQ ID NO:1. In another aspect, a fragment may comprise the full-length (368 amino acids long; amino acids 177 - 544 of SEQ ID NO:1) mature TPP1 enzyme amino acid sequence set forth in SEQ ID NO:2, a portion thereof, and/or at least the catalytic triad formed by the amino acid residues S456, E253, and D341. A fragment retains catalytic activity. For example, a fragment exhibits tripeptidyl exopeptidase activity and/or exhibits catalytic activity that results in the sequential release of tripeptides from the N-terminus of a protein substrate. In certain aspects, a "fragment" of the rhTPP1 proenzyme comprises at least 500 consecutive amino acids of SEQ ID NO:1, at least 450 consecutive amino acids of SEQ ID NO:1, at least 400 consecutive amino acids of SEQ ID NO:1, at least 368 amino acids of SEQ ID NO:1, at least 350 amino acids of SEQ ID NO:1 or at least 300 consecutive amino acids of SEQ ID NO: 1. In other aspects, a "fragment" of the rhTPP1 proenzyme comprises at least 350 consecutive amino acids of SEQ ID NO:2, at least 325 consecutive amino acids of SEQ ID NO:2, at least 300 consecutive amino acids of SEQ ID NO:2, at least 275 consecutive amino acids of SEQ ID NO:2, at least 250 consecutive amino acids of SEQ ID NO:2 or at least 200 consecutive amino acids of SEQ ID NO:2.

The term "intracerebroventricular" refers to administration of a composition into the ventricular system of the brain, e.g., via injection, infusion, or implantation (for example, into a ventricle of the brain).

The term "intraocular" refers to the administration of a composition to the eye region, e.g., via injection, infusion, or implantation (for example, into the eyeball) or topical/ophthalmic administration (for example, using a cream, ointment, gel or liquid drops).

The term "intrathecal" refers to administration of a composition into the lumbar region, e.g., via injection, infusion, or implantation (for example, into the subarachnoid space of the spinal cord).

The term "therapeutically effective" refers to any therapeutic benefit that arises as a result of the treatment methods of the present invention. For example, such an effect can be the beneficial effects that manifest in an appropriate target tissue or organ, where such beneficial physiological effect is compared to that physiological parameter being measured in the absence of the enzyme replacement therapy. Such a therapeutic effect may be any reduction or elimination of one or more clinical or subclinical manifestations of CLN2 disease. For example, a therapeutically effective treatment improves, reverses, delays, prevents, or reduces deterioration of one or more physiological function and/or neurological symptom of CLN2 as described herein.

The term "stable" or "stabilized" refers to a protein-containing formulation in which the protein component therein essentially retains its physical, functional and/or chemical stability upon storage over time. Stability can be measured at a selected temperature for a selected time period. Preferably, the formulation is stable at room temperature (about 30 °C) or at about 40 °C for at least 1 month and/or stable at about 2 °C to about 8 °C for at least 1 year and preferably for at least 2 years. For example, the extent of protein degradation or aggregation during storage can be used as an indicator of protein stability. Thus, a "stable" formulation may be one wherein less than about 20%, more preferably less than about 10%, and most preferably less than about 5% of the protein component is present in a degraded or aggregated form in the formulation following storage. "Stable" formulations retain essentially the same functional or therapeutic characteristics of the newly prepared formulation. Various analytical techniques for measuring protein stability are available in the art and are reviewed, for example, in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993).

The term "prevents" or "reduces" or grammatical equivalents thereof when used in reference to the prevention or reduction of one or more symptoms or physiological consequences of CLN2 disease in a subject means that the rate of decline of that/those symptom(s) in the treated CLN2 subject is slower than that observed in an untreated CLN2 subject. In this regard, the untreated CLN2 may be the same subject that is subsequently treated with a composition of the present invention or may be the average rate of decline of the symptom(s) of interest as observed from the natural history study results disclosed herein.

In jurisdictions that forbid the patenting of methods that are practiced on the human body, the meaning of "administering" rhTPP1 or a formulation thereof to a human subject refers to medical uses for rhTPP1 or a formulation thereof, for example, rhTPP1 or a formulation thereof for use in treating CLN2 disease as described herein or use of rhTPP 1 for the manufacture of a medicament for treating CLN2 disease as described herein. The broadest reasonable interpretation that is consistent with laws or regulations defining patentable subject matter is intended. In jurisdictions that do not forbid the patenting of methods that are practiced on the human body, "administering" rhTPP1 or a formulation thereof includes both methods practiced on the human body and also the foregoing activities.

The present disclosure provides formulations and kits comprising rhTPP 1, and methods of using the same to treat CLN2 disease. Administration of rhTPP1 allows for cellular uptake of the protein by the cation independent mannose 6 phosphate receptor (CI-MPR) and localization to the lysosomes in cells throughout the central nervous system. The enzyme uptake into the lysosomes and subsequent activation promotes increased catabolism of storage material in affected tissues, reduces the progressive accumulation of the lysosomal storage material, and arrests decline of the disease. The formulations and methods of the disclosure provide therapeutic benefits that surpass those of currently approved treatments.

### Formulations

With regard to the present disclosure, the formulations comprises an amount of rhTPP 1 and in exemplary aspects, the formulation is suitable for intracerebroventricular, intrathecal, and/or intraocular administration. In one aspect, the rhTPP1 comprises SEQ ID NO: 1 or a fragment thereof. RhTPP1 proteins suitable for use in the formulations and methods described herein, and methods of obtaining the rhTPP1 proteins, are described in U.S. Patent Nos. 6,302,685 and 8,277,800.

In one aspect, the rhTPP1 comprises the amino acid sequence of SEQ ID NO: 1 (amino acids 1-544 of the amino acid sequence shown in Figure 1) or a fragment thereof possessing catalytic activity. In another aspect, the rhTPP1 comprises the amino acid sequence of SEQ ID NO:2 (amino acids 177-544 of the amino acid sequence shown in Figure 1) or a fragment thereof possessing catalytic activity. In still another aspect, the rhTPP1 has detectable enzyme activity or is processed *in vivo* to a form of the enzyme that has detectable enzyme activity (i.e., is "functional") and has at least about 70% sequence identity with SEQ ID NO: 1 or SEQ ID NO:2. For example, the functional rhTPP1 is at least about 70% identical, at least about 75% identical, at least about 80% identical, at least about 85% identical, at least about 90% identical, at least about 95% identical, or at least about 97% identical, to SEQ ID NO: 1 or SEQ ID NO:2. In one aspect, the formulation is a liquid formulation that comprises rhTPP 1 at a concentration of about 1 mg/mL to about 100 mg/mL, for example, about 10 mg/mL to about 50 mg/mL, about 25 mg/mL to about 40 mg/mL, or about 30 mg/mL to about 60 mg/mL. In various aspects, the formulation comprises rhTPP1 at a concentration of from about 1 mg/mL to about 100 mg/mL, from about 5 mg/mL to about 80 mg/mL, from about 10 mg/mL to about 50 mg/mL, from about 20 mg/mL to about 40 mg/mL, from about 25 mg/mL to about 35 mg/mL, more specifically about 1 mg/mL, about 10 mg/ml, about 20 mg/mL, about 30 mg/mL, about 40 mg/mL, about 50 mg/mL, about 60 mg/mL, about 70 mg/mL, about 80 mg/mL, about 90 mg/mL, or about 100 mg/mL. In one aspect, the formulation has a pH of about 5.5 to about 7.5 or about 6.0 to about 7.0, for example, about 5.5, about 6.0, about 6.5, about 7.0, or about 7.5.

In one aspect, a formulation comprising rhTPP 1 of the disclosure further comprises one or more excipients that maintains the level of a key electrolyte in the cerebrospinal fluid (CSF) or ocular fluid. For example, in one aspect, in addition to the rhTPP 1 or fragment thereof, the formulation further comprises potassium chloride at a concentration of about 0.01 mg/mL to about 1 mg/mL, for example, about 0.1 mg/mL to about 0.5 mg/mL, about 0.2 mg/mL to about 0.8 mg/mL, about 0.2 mg/mL to about 0.4 mg/mL, about 0.15 mg/mL to about 0.25 mg/mL, or about 0.05 mg/mL to about 0.3 mg/mL. In another aspect, the formulation further comprises magnesium chloride hexahydrate at a concentration of about 0.01 mg/mL to about 1 mg/mL, for example, about 0.1 mg/mL to about 0.5 mg/mL, about 0.1 mg/mL to about 0.8 mg/mL, about 0.1 mg/mL to about 0.3 mg/mL, about 0.15 mg/mL to about 0.25 mg/mL, or about 0.05 mg/mL to about 0.3 mg/mL. In another aspect, the formulation further comprises calcium chloride dihydrate at a concentration of about 0.01 mg/mL to about 1 mg/mL, for example, about 0.1 mg/mL to about 0.5 mg/mL, about 0.2 mg/mL to about 0.8 mg/mL, about 0.15 mg/mL to about 0.25 mg/mL, about 0.1 mg/mL to about 0.3 mg/mL, or about 0.05 mg/mL to about 0.3 mg/mL. In still another aspect, the formulation comprises a combination of all or any of the foregoing.

In another aspect, the formulation comprising rhTPP1 further comprises one or more buffering agents. For example, in various aspects, the formulation further comprises sodium phosphate dibasic heptahydrate at a concentration of about 0.01 mg/mL to about 1 mg/mL, for example, about 0.1 mg/mL to about 0.5 mg/mL, about 0.05 mg/mL to about 0.4 mg/mL, or about 0.1 mg/mL to about 0.3 mg/mL; and/or sodium phosphate monobasic monohydrate at a concentration of about 0.01 mg/mL to about 1 mg/mL, for example, about 0.01 mg/mL to about 0.2 mg/mL, about 0.05 mg/mL to about 0.3 mg/mL, or about 0.08 mg/mL to about 0.4 mg/mL.

In another aspect, the formulation further comprises an isotonicity agent, such as sodium chloride at a concentration of about 1 mg/mL to about 20 mg/mL, for example, about 1 mg/mL to about 10 mg/mL, about 5 mg/mL to about 15 mg/mL, or about 8 mg/mL to about 20 mg/mL. Other buffering agents and isotonicity agents known in the art are suitable and may be routinely employed for use in the formulations of the present disclosure.

In one aspect, a formulation comprising about 30 mg/mL of rhTPP 1 further comprises sodium phosphate dibasic heptahydrate at a concentration of about 0.11 mg/mL, sodium phosphate monobasic monohydrate at a concentration of about 0.08 mg/mL, sodium chloride at a concentration of about 8.77 mg/mL, potassium chloride at a concentration of about 0.22 mg/mL, magnesium chloride hexahydrate at a concentration of about 0.16 mg/mL, calcium chloride dihydrate at a concentration of about 0.21 mg/mL, and a diluent, such as water for injection.

The rhTPP1 formulations of the present disclosure are stable and can be stored for extended periods of time without an unacceptable change in quality, potency, or purity. In one aspect, the formulation is stable at a temperature of about 5 °C (e.g., 2 °C to 8 °C) for at least 1 month, for example, at least 1 month, at least 3 months, at least 6 months, at least 12 months, at least 18 months, at least 24 months, or more. In another aspect, the formulation is stable at a temperature of less than or equal to about -20 °C for at least 6 months, for example, at least 6 months, at least 12 months, at least 18 months, at least 24 months, at least 36 months, or more. In another aspect, the formulation is stable at a temperature of less than or equal to about -40 °C for at least 6 months, for example, at least 6 months, at least 12 months, at least 18 months, at least 24 months, at least 36 months, or more. In another aspect, the formulation is stable at a temperature of less than or equal to about -60 °C for at least 6 months, for example, at least 6 months, at least 12 months, at least 18 months, at least 24 months, at least 36 months, or more.

In one aspect, a formulation of the disclosure is preservative-free and/or stabilizer-free and thus does not contain any of thimerosal, phenylmercurate salts, chlorhexidene, phenol, benzoic acid, sorbic acid, parabens, alcohols, or other preservatives commonly found in parenteral or ophthalmic formulations.

In another aspect, the formulation of the present invention may comprise one or more preservatives, stabilizers or excipients. In this regard, numerous well known and routinely employed preservatives, stabilizers and excipients useful for protein-containing formulations for intrathecal or ICV delivery are known in the art. More specifically, examples of such additives to enzyme-containing formulations for use in intrathecal or ICV delivery are described in WO2013/096899.

### Methods

The disclosure provides methods of treating CLN2 disease comprising administering a therapeutically effective amount of a formulation comprising rhTPP1 described herein to a subject in need thereof. The disclosure also provides a composition comprising rhTPP1 for use in treating CLN2 disease described herein and use of rhTPP 1 in the manufacture of a medicament for treating CLN2 disease described herein. In one aspect, severity and progression of CLN2 disease and the therapeutic benefit of administration of rhTPP 1 in a patient can be measured using a Hamburg or WCMC clinical disease rating scale. Both the Hamburg and WCMC scales consist of four disease-related domains, which are scored in ratings on subscales of 0 to 3 points, such that 3 points is normal, 2 points is abnormal but functional, 1 point is abnormal and markedly dysfunctional, and 0 points is no residual function. Two of the four domains, gait/motor and language, are shared in common between the two scales, and have high intrinsic content validity. Each scale in total captures changes that occur as a function of both disease progression and disease management. Gait, language and vision scales capture disease progression. Seizure frequency, movement disorders and feeding are dependent on care decisions, particularly anticonvulsant medications and feeding tube management. The clinical progression is often assessed using the aggregate language and gait subscales, such that a rating of 6 points represents age-based normal and 0 points is complete loss of function. Table 1 depicts the WCMC and Hamburg CLN2 disease scales.

**TABLE 1**

| **Weill Cornell Scale (WCMC)** | | | | **Hamburg Scale** | | |
|---|---|---|---|---|---|---|
| Gait | 3 | Normal | | Motor | 3 | Walks normally |
| | 2 | Abnormal but independent | | | 2 | Frequent falls, obvious clumsiness |
| | 1 | Abnormal requiring assistance | | | 1 | No unaided walking or crawling only |
| | 0 | Nonambulatory | | | 0 | Immobile, mostly bedridden |
| Language | 3 | Normal | | Language | 3 | Normal |
| | 2 | Abnormal | | | 2 | Recognizably abnormal |
| | 1 | Barely understandable | | | 1 | Hardly understandable |
| | 0 | Unintelligible or no speech | | | 0 | Unintelligible or no language |
| | | | | | | |
| Myoclonus (motor) | 3 | None of myoclonus, chorea/tremor/athetosis, and upgoing toes | | Visual | 3 | Recognizes desirable object, grabs at it |
| | 2 | One of myoclonus, chorea/tremor/athetosis, or upgoing toes | | | 2 | Grabbing for objects uncoordinated |
| | 1 | Two of myoclonus, chorea/tremor/athetosis, or upgoing toes | | | 1 | Reacts to light |
| | 0 | Myoclonus and chorea/tremor/ athetosis and upgoing toes | | | 0 | No reaction to visual stimuli |
| Feeding | 3 | No swallowing dysfunction | | Seizures | 3 | No seizure in 3 months |
| | 2 | Mild swallowing dysfunction | | | 2 | 1-2 seizures in 3 months |
| | 1 | Moderate swallowing dysfunction | | | 1 | 1 seizure per month |
| | 0 | Gastrostomy tube-dependent | | | 0 | >1 seizure per month |

In various aspects, the disclosure provide a method of treating CLN2 disease, or one or more clinical symptoms of CLN2 disease, comprising administering a composition comprising a therapeutically effective amount of rhTPP1 to a subject in need thereof, use of rhTPP1 in the manufacture of a medicament for the treatment of CLN2 disease in a subject, or rhTPP 1 for use in treating CLN2 disease in a subject.

The disclosure also provides methods of preventing one or more clinical symptoms of CLN2 disease comprising administering a formulation comprising rhTPP 1 described herein to a subject in need thereof, optionally wherein the subject has a family history of CLN2 disease. In various aspects, the disclosure provide a method of preventing one or more clinical symptoms of CLN2 disease comprising administering a composition comprising a therapeutically effective amount of rhTPP1 to a subject in need thereof, use of rhTPP 1 in the manufacture of a medicament for the prevention of one or more clinical symptoms of CLN2 disease in a subject, or rhTPP 1 for use in preventing one or more clinical symptoms of CLN2 disease in a subject, optionally wherein the subject has a family history of CLN2 disease.

The disclosure further provides methods of treating CLN2 disease comprising administering rhTPP1 to a subject in need thereof at a dose effective to maintain a physiological function or slow or reduce deterioration of a physiological function in the subject, wherein the physiological function is language function, motor function, vision, or feeding function. The disclosure also provides use of rhTPP 1 in the manufacture of a medicament for maintaining a physiological function or slowing or reducing deterioration of a physiological function in a subject having CLN2, and rhTPP 1 for use in maintaining a physiological function or slowing or reducing deterioration of a physiological function in a subject having CLN2 disease; wherein the physiological function is language function, motor function, vision, or feeding function.

In one aspect, a method of treating a subject having CLN2 disease or a family history of CLN2 disease comprises administering a dose of rhTPP 1 effective to maintain language function or slow or reduce deterioration of language function to the subject. In one aspect, the deterioration of language function is a reduction of at least one point compared to a previous rating determined before or during treatment as measured using a WCMC or Hamburg disease rating scale. In both the WCMC and Hamburg scales, a rating of 3 points indicates normal language; 2 points indicates (recognizably) abnormal language; 1 point indicates barely/hardly understandable language; and 0 points indicates unintelligible or no language. In one aspect, the dose of rhTPP 1 is effective to maintain the subject's language rating at the same level as a previous rating determined before or during treatment, e.g., 3 points, 2 points or 1 point. In another aspect, the dose of rhTPP 1 is effective to slow or reduce CLN2-associated deterioration of language function in the subject, which can be demonstrated by maintenance of the language rating at the same level for a longer period of time or a smaller decrease in the language function rating, compared to what would be expected considering the natural progression of the disease.

In another aspect, a method of treating a subject with CLN2 or a family history of CLN2 comprises administering a dose of rhTPP 1 effective to maintain motor function or slow or reduce deterioration of motor function to the subject. In one aspect, the deterioration of motor function is a reduction of at least one point compared to a previous rating determined before or during treatment as measured using a WCMC or Hamburg disease rating scale. Either the clinical rating scale for gait in the WCMC scale or for motor in the Hamburg scale can be used to evaluate motor function. In both the WCMC and Hamburg scales, a rating of 3 points indicates normal walking; 2 points indicates abnormal but independent walking, e.g., with frequent falls or obvious clumsiness; 1 point indicates abnormal walking requiring assistance, e.g., no unaided walking or crawling only; and 0 points indicates the subject in non-ambulatory/immobile, e.g., mostly bedridden. In one aspect, the dose of rhTPP 1 is effective to maintain the subject's motor function rating at the same level as a previous rating determined before or during treatment, e.g., 3 points, 2 points, or 1 point. In another aspect, the dose of rhTPP 1 is effective to slow or reduce CLN2-associated deterioration of motor function in the subject, which can be demonstrated by maintenance of the motor rating at the same level for a longer period of time or a smaller decrease in the motor function rating, compared to what would be expected considering the natural progression of the disease.

In still another aspect, a method of treating a subject with CLN2 or a family history of CLN2 comprises administering a dose of rhTPP 1 effective to maintain vision or slow or reduce deterioration of vision to the subject. In one aspect, the deterioration of vision is a reduction of at least one point compared to a previous rating determined before or during treatment as measured using a Hamburg disease rating scale. According to the Hamburg scale, a rating of 3 points indicates the subject recognizes a desirable object and grabs at it; 2 points indicates grabbing for objects uncoordinated; 1 point indicates the subject reacts to light, and 0 points indicates the subject has no reaction to visual stimuli. In one aspect, the dose of rhTPP1 is effective to maintain the subject's vision rating at the same level as a previous rating determined before or during treatment, e.g., 3 points, 2 points or 1 point. In another aspect, the dose of rhTPP1 is effective to slow or reduce CLN2-associated deterioration of vision in the subject, which can be demonstrated by maintenance of the vision rating at the same level for a longer period of time or a smaller decrease in the vision rating, compared to what would be expected considering the natural progression of the disease.

In another aspect, a method of treating a subject with CLN2 or a family history of CLN2 comprises administering a dose of rhTPP 1 effective to maintain feeding function or slow or reduce deterioration of feeding function to the subject. In one aspect, the deterioration of feeding function is a reduction of at least one point compared to a previous rating determined before or during treatment as measured using a WCMC rating scale. According to the WCMC scale, a rating of 3 points indicates no swallowing dysfunction; 2 points indicates mild swallowing dysfunction; 1 point indicates moderate swallowing dysfunction, and 0 points indicates the subject is gastronomy-tube dependent. In one aspect, the dose of rhTPP1 is effective to maintain the subject's feeding function rating at the same level as the previous rating determined before or during treatment, e.g., 3 points, 2 points, or 1 point. In another aspect, the dose of rhTPP1 is effective to slow or reduce CLN2-associated deterioration of feeding function in the subject, which can be demonstrated by maintenance of the feeding function at the same level for a longer period of time or a smaller decrease in the feeding rating, compared to what would be expected considering the natural progression of the disease.

The disclosure further provides methods of treating CLN2 disease comprising administering rhTPP1 to a subject in need thereof at a dose effective to improve a physiological function, wherein the physiological function is language function, motor function, vision, or feeding function. The disclosure also provides use of rhTPP 1 in the manufacture of a medicament for improving a physiological function in a subject having CLN2, or rhTPP1 for use in improving a physiological function in a subject having CLN2; wherein the physiological function is language function, motor function, vision, or feeding function. Considering the progressively degenerative nature of the disease, an improvement in language function, motor function, vision, and/or feeding function, indicating that the subject has regained lost function, is especially desirable, but difficult to achieve with current treatment options.

In one aspect, a method of treating a subject with CLN2 disease comprises administering a dose of rhTPP 1 effective to improve language function to the subject. In one aspect, the improvement in language function is an increase of at least one point compared to a previous rating determined before or during treatment as measured using a WCMC or Hamburg disease rating scale. For example, a subject can improve from a rating of 1 point or 2 points to a rating of 3 points, indicating a return to normal language, or improve from a rating of 1 point to a rating of 2 points.

In another aspect, a method of treating a subject with CLN2 disease comprises administering a dose of rhTPP 1 effective to improve motor function to the subject. In one aspect, the improvement in motor function is an increase of at least one point compared to a previous rating determined before or during treatment as measured using a WCMC or Hamburg disease rating scale. For example, a subject can improve from a rating of 1 point or 2 points to a rating of 3 points, indicating a return to normal walking, or improve from a rating of 1 point to a rating of 2 points.

In one aspect, a method of treating a subject with CLN2 disease comprises administering a dose of rhTPP 1 effective to improve vision to the subject. In one aspect, the improvement in vision is an increase of at least one point compared to a previous rating determined before or during treatment as measured using a Hamburg disease rating scale. For example, a subject can improve from a rating of 1 point or 2 points to a rating of 3 points, or improve from a rating of 1 point to a rating of 2 points.

In another aspect, a method of treating a subject with CLN2 disease comprises administering a dose of rhTPP 1 effective to improve feeding function to the subject. In one aspect, the improvement in feeding function is an increase of at least one point compared to a previous rating determined before or during treatment as measured using a WCMC disease rating scale. For example, a subject can improve from a rating of 1 point or 2 points to a rating of 3 points, indicating a return to normal swallowing, or improve from a rating of 1 point to a rating of 2 points or 3 points.

The disclosure further provides methods of treating CLN2 disease comprising administering rhTPP1 to a subject in need thereof at a dose effective to prevent or treat a neurological symptom of the disease, wherein the neurological symptom is a seizure, decrease in brain volume, decrease in gray matter in the brain, or increase of cranial cerebrospinal fluid (CSF). The disclosure also provides use of rhTPP 1 in the manufacture of a medicament for preventing or treating a neurological symptom in a subject having CLN2 or a family history of CLN2, and rhTPP1 for use in preventing or treating a neurological symptom in a subject having CLN2 or a family history of CLN2; wherein the neurological symptom is a seizure, decrease in brain volume, decrease in gray matter in the brain, or increase of cranial CSF.

In one aspect, a method of treating a subject having CLN2 or a family history of CLN2 comprises administering a dose of rhTPP 1 effective to maintain or reduce the number of seizures to a subject. In one aspect, the dose is effective to reduce the number of seizures per month that the subject experiences. In another aspect, the dose is effective to increase the seizure rating by at least one point compared to a previous rating determined before or during treatment as measured using a Hamburg disease rating scale. According to the Hamburg scale, a rating of 3 points indicates no seizure in 3 months; 2 points indicates 1 to 2 seizures in 3 months; 1 point indicates 1 seizure per month; and 0 points more than 1 seizure per month. In one aspect, the dose of rhTPP1 is effective to maintain the subject's seizure rating at the same level as the previous rating determined before or during treatment, e.g., 3 points, 2 points, or one point. In another aspect, the dose of rhTPP 1 is effective to maintain or reduce the number of seizures in the subject, which can be demonstrated by maintenance of the number of seizures per month for a longer period of time or a smaller decrease in the seizure rating, compared to what would be expected considering the natural progression of the disease.

In another aspect, a method of treating a subject having CLN2 or a family history of CLN2 comprises administering a dose of rhTPP 1 effective to maintain brain volume or slow or reduce the decrease in brain volume to a subject. Brain atrophy increases as the disease progresses, resulting in a loss of brain volume and an associated increase in the volume and relative proportion of intracranial CSF. Brain volume can be measured using methods known in the art, including imaging techniques such as magnetic resonance imaging (MRI), computed tomography (CT/CAT), positron emission tomography (PET), single photon emission computerized tomography (SPECT), electroencephalography (EEG), magnetoencephalography (MEG), and near infrared spectroscopy (NIRS). In one aspect, the dose of rhTPP1 is effective to slow or reduce the CLN2-associated decrease in brain volume in the subject, which can be demonstrated by maintenance of brain volume for a longer period of time or a smaller decrease in brain volume, compared to what would be expected considering the natural progression of the disease.

In another aspect, method of treating a subject having CLN2 or a family history of CLN2 comprises administering a dose of rhTPP1 effective to maintain gray matter in the brain or slow or reduce the decrease of gray matter in the brain to a subject. A loss of gray matter due to brain atrophy occurs as the disease progresses, resulting in a decrease in gray matter as a percentage of brain volume. The amount of gray matter in the brain can be assessed using methods known in the art, for example, imaging techniques such as MRI, CT/CAT, PET, SPECT, EEG, MEG, and NIRS. In one aspect, the dose of rhTPP 1 is effective to slow or reduce the decrease in gray matter in the subject, which can be demonstrated by maintenance of gray matter volume for a longer period of time or a smaller decrease in gray matter as a percentage of brain volume, compared to what would be expected considering the natural progression of the disease.

In another aspect, a method of treating a subject having CLN2 or a family history of CLN2 comprises administering a dose of rhTPP1 effective to maintain the volume of cranial CSF or slow the increase in the volume of cranial CSF to a subject. Cranial CSF increases in volume and proportion of total CSF as a result of cerebral atrophy. The amount and proportion of cranial CSF can be assessed using methods known in the art, for example, imaging techniques such as MRI and CT/CAT. In one aspect, the dose of rhTPP 1 is effective to slow or reduce the increase in cranial CSF in the subject, which can be demonstrated by maintenance of the volume of cranial CSF for a longer period of time or a smaller increase in cranial CSF as a percentage of total CSF, compared to what would be expected considering the natural progression of the disease.

The foregoing methods, compositions for use, and uses may further comprise any of the following features, alone and in combination.

In one aspect, a method, composition for use, or use of the disclosure comprises administering a formulation, composition or dose comprising rhTPP 1 to a subject continuously or continually over a period of at least about 1 hour, for example, at least about 1 hour, at least about 2 hours, at least about 3 hours, at least about 4 hours, at least about 5 hours, at least about 6 hours, or more. In another aspect, a method or use of the disclosure comprises administering a formulation, composition or dose comprising about 20 mg, about 30 mg, about 50 mg, about 100 mg, about 200 mg, or about 300 mg of rhTPP1 to a subject in need thereof. In one aspect, a method or use of the disclosure comprises administering a formulation, composition or dose having a volume of about 20 mL or less, about 15 mL or less, about 10 mL or less, about 7.5 mL or less, or about 5 mL or less, for example, about 20 mL, about 15 mL, about 10 mL, about 9 mL, about 8 mL, about 7 mL, about 6 mL, about 5 mL, about 4 mL, about 3 mL about 2 mL, about 1 mL, or about 0.5 mL per dose or administration event.

In various aspects, a method, composition for use, or use of the disclosure comprises administering a formulation, composition or dose comprising rhTPP1 to a subject at a rate of less than or equal to about 2.5 mL of the formulation, composition or dose per hour; less than or equal to about 75 mg of rhTPP 1 per hour; or less than or equal to about 75 mg of rhTPP 1 per 2.5 mL of formulation or composition per hour. The formulation, composition or dose is optionally administered continuously or continually over a period of at least about 4 hours.

In one aspect, a method, composition for use, or use of the disclosure comprises administering a formulation, composition or dose comprising rhTPP1 once every 2 weeks. In one aspect, the formulation, composition or dose is administered intracerebroventricularly. In another aspect, the formulation, composition or dose is administered intrathecally. In still another aspect, the formulation, composition or dose is administered intraocularly. In one aspect, the formulation, composition or dose is administered intracerebroventricularly or intrathecally, as well as intraocularly. Intracerebroventricular delivery allows penetration to the deep gray structures of the brain such as the thalami, striatum and midbrain, due to the physiology of CSF flow in which ventricular delivery allows flow into third and fourth ventricles, but also percolates through the neuropil of the cerebral hemispheres, along a slight pressure gradient from ventricle to subarachnoid space. Intrathecal and intracerebroventricular administration of recombinant enzyme to treat lysosomal storage disorders are described in U.S. Patent No. 7,442,372.

A formulation, composition, or dose of rhTPP 1 of the disclosure may be administered in a single bolus injection or series of injections (e.g., into the brain, lumbar region, or eye), or as a continuous or continual infusion, e.g., using an infusion pump or other implanted device. In one aspect, a formulation, composition, or dose of rhTPP 1 is administered using an infusion system comprising tubing, an in-line filter (e.g., about 0.2 µm), a reservoir (e.g., intrathecal or intracerebroventricular), and a catheter. Frequently, when a composition is administered intrathecally or intracerebroventricularly, in order to prevent adverse side effects resulting from artificially increasing intracerebral or intrathecal pressure, a volume of CSF comparable to the volume of composition to be administered is first removed from the subject before the composition is administered. As described in Example 3, however, it is herein demonstrated that a formulation, composition, or dose of rhTPP 1 of the disclosure may be administered without removal of any volume of CSF from the subject just prior to administration of the formulation, composition, or dose of rhTPP 1.

In one aspect, a method or use of the disclosure comprises administering about 10 mL of a formulation, composition or dose comprising about 300 mg of rhTPP 1 intracerebroventricularly over a period of about 4 hours every other week to a subject having CLN2.

The formulations and compositions of the present invention may be directly administered to a subject in need (i.e., non-isovolumetric) or may be administered subsequent to removal of a defined volume of CSF from the subject prior, wherein that defined volume is approximately the same as the volume of the composition subsequently administered (i.e., isovolumetric).

In one aspect, a method, composition for use, or use of the disclosure further comprises administering a flushing solution to the subject following administration of the rhTPP1. The flushing solution is administered via the same route as the rhTPP1 and using the same delivery system (e.g., an infusion system), to remove any rhTPP1 remaining in the delivery system and to ensure the subject received the full intended dose of rhTPP 1. In one aspect, the flushing solution is administered (e.g., using the same catheter previously used to administer a composition comprising rhTPP1) to the subject in an amount between about 0.5 mL and about 5 mL, for example, about 0.5 mL, about 1 mL, about 2 mL, about 3 mL, or about 5 mL. In one aspect, the flushing solution comprises the same components as the formulation or composition comprising rhTPP 1, but without the rhTPP 1. In one aspect, the flushing solution comprises sodium phosphate dibasic heptahydrate at a concentration of about 0.11 mg/mL, sodium phosphate monobasic monohydrate at a concentration of about 0.08 mg/mL, sodium chloride at a concentration of about 8.77 mg/mL, potassium chloride at a concentration of about 0.22 mg/mL, magnesium chloride hexahydrate at a concentration of about 0.16 mg/mL, calcium chloride dihydrate at a concentration of about 0.21 mg/mL, and a diluent, such as water for injection.

In various embodiments, the time of maximum concentration (Tₘₐₓ) of rhTPP1 in cerebrospinal fluids is between 4 and 10 hours after end of infusion. In various embodiments, elimination half-life (t_{1/2}) of rhTPP 1 in cerebrospinal fluid is between 5 and 20 hours.

### Pediatric Subjects

The present disclosure is also directed to methods of treating CLN2 disease, or one or more symptoms associated with CLN2 disease, in a subject, and methods of delaying the onset of CLN2 disease, or a symptom thereof, in a subject, wherein the subject in exemplary is a pediatric subject, e.g., less than about 18 years old. In aspects of the invention, the subject is less than 3 years old. In various aspects, the subject is less than 2 years old. In various aspects, the subject is less than 1 year old. In various aspects, the subject is greater than or about 1 month to about 3 months old or greater than or about 1 month to about 6 months old or greater than or about 1 month old to about 9 months old or greater than or about 1 month old to about 12 months old. In various aspects, the subject is less than or about 12 months old but at least or about 2 weeks old, 4 weeks old, 6 weeks old, 12 weeks old, or 16 weeks old. In various instances, the subject is less than 12 months old but at least or about 5 months old, 6 months old, 7 months old, 8 months old, 9 months old, 10 months old, or 11 months old. In various instances, the subject is at least 12 months old but less than or about 24 months old or less than or about 23 months old or less than or about 22 months old or less than or about 21 months old or less than or about 20 months old or less than or about 19 months old or less than or about 18 months old or less than or about 17 months old or less than or about 16 months old or less than or about 15 months old or less than or about 14 months old or less than or about 13 months old. In various instances, the subject is less than 24 months old and at least or about 13 months old or at least or about 14 months old or at least or about 15 months old or at least or about 16 months old or at least or about 17 months old or at least or about 18 months old or at least or about 19 months old or at least or about 20 months old or at least or about 21 months old or at least or about 22 months old or at least or about 23 months old. In various instances, the subject is greater than or about 24 months old and less than or about 36 months old, or less than or about 35 months old, or less than or about 34 months old, or less than or about 33 months old, or less than or about 32 months old, or less than or about 31 months old, or less than or about 30 months old, or less than or about 29 months old, or less than or about 28 months old, or less than or about 27 months old, or less than or about 26 months old, or less than or about 25 months old.

In exemplary embodiments, the method comprises administering to the subject a formulation comprising recombinant human tripeptidyl peptidase-1 (rhTPP1) in an effective amount for the desired outcome, e.g., treatment of CLN2 disease or symptom thereof or delayed onset of the CLN2 disease or symptom thereof. In instances of the presently disclosed methods of the invention, a dosage of about 300 mg or less rhTPP1 is administered to the subject. In exemplary aspects, the subject is greater than or about 2 years old, and, optionally, a dosage of about 300 mg rhTPP1 is administered to the subject. In various aspects, the subject is greater than or about 1 year old and less than 2 years old, and optionally, a dosage of about 200 mg rhTPP1 is administered to the subject. In exemplary aspects, the subject is administered multiple dosages of the rhTPP1. In exemplary aspects, the method comprises administering an initial dosage amount followed by administering subsequent dosages. In various aspects, the initial dosages are lower than the subsequent dosages. In various aspects, the initial dosages are higher than the subsequent dosages. In various aspects, the initial dosages are about 200 mg rhTPP1 and the subsequent dosages are higher. In exemplary aspects, the initial dosages are about 200 mg rhTPP 1 and the subsequent dosages are at least 50% higher, e.g., at least or about 300 mg rhTPP1. In exemplary aspects, each of the 1^{st}, 2^{nd}, 3^{rd} and 4^{th} dosages administered to the subject (e.g., who is greater than or about 1 year old and less than 2 years) is about 200 mg rhTPP1 and each of the 5^{th} and subsequent dosages administered to the subject is greater than about 200 mg rhTPP1. In exemplary instances, each of the 5^{th} and subsequent dosages administered to the subject is about 300 mg rhTPP1. In various aspects, the subject is greater than or about 6 months old and less than 1 year old, and, optionally, a dosage of about 150 mg rhTPP1 is administered to the subject. In various aspects, the subject is less than 6 months old, and, optionally, a dosage of about 100 mg rhTPP1 is administered to the subject. In aspects of the invention, the formulation is administered once every 2 weeks. In various aspects, the formulation is administered by infusion at a rate of about 2.5 mL per hour or at a lower rate.

In exemplary aspects, the subject, e.g., pediatric subject, exhibits a decrease in TPP1 enzyme activity based on a blood test. In exemplary instances, the subject, e.g., pediatric subject, is a sibling of an individual diagnosed with CLN2. In exemplary instances, the subject, e.g., pediatric subject, is not a sibling of an individual diagnosed with CLN2. In exemplary aspects, the subject, e.g., pediatric subject, has a total score on the motor and language subscales of about 3 to about 6 points (e.g., about 3 points, about 4 points, about 5 points, about 6 points). In exemplary aspects, the subject, after having administered the rhTPP1, has a score that is closer to a score associated with "healthy" or "normal" according to the scores delineated in Table 1.

In exemplary aspects, the subject, e.g., pediatric subject, has no prior treatment with a stem cell therapy, gene therapy, or enzyme supplementation therapy. In exemplary instances, the method comprises administering to the subject an antihistamine with or without an antipyretic before administration of the rhTPP1, optionally, about 30 to about 60 minutes before administration of the rhTPP1. In various aspects, the formulation comprises the rhTPP1 and at least one pharmaceutically acceptably carrier, diluent or excipient. In various instance, the formulation is any one of the aforementioned formulations, including but not limited to being one which comprises disodium hydrogen phosphate pentahydrate, monosodium phosphate monohydrate, sodium chloride, potassium chloride, magnesium chloride, calcium chloride hydrate, water for injection, or a combination thereof. In various instances, the method comprises administering to the subject a flush solution after administering the formulation. In various aspects, the flush solution is any one of those described herein. In various aspects, the flush solution comprises disodium hydrogen phosphate pentahydrate, monosodium phosphate monohydrate, sodium chloride, potassium chloride, magnesium chloride, calcium chloride hydrate, water for injection, or a combination thereof. In various aspects, the treatment period is at least 10 weeks, at least 20 weeks, at least 40 week, at least 80 weeks, or at least 96 weeks. In various aspects, the treatment period is longer than 96 weeks. In various aspects, the methods treat the CLN2 disease or delay the onset of the CLN2 disease, or the symptom thereof, without causing serious adverse events (SAEs). In various aspects, the subject has an ICV device. In various aspects, the method comprises implanting an ICV device in the subject.

### Kits

The disclosure further provides kits comprising a formulation of rhTPP 1 described herein, in a dose and form suitable for administration to a patient. In one aspect, the kit comprises a formulation comprising about 30 mg/mL of rhTPP1, sodium phosphate dibasic heptahydrate at a concentration of about 0.11 mg/mL, sodium phosphate monobasic monohydrate at a concentration of about 0.08 mg/mL, sodium chloride at a concentration of about 8.77 mg/mL, potassium chloride at a concentration of about 0.22 mg/mL, magnesium chloride hexahydrate at a concentration of about 0.16 mg/mL, calcium chloride dihydrate at a concentration of about 0.21 mg/mL, and a diluent, such as water for injection. In one aspect, the kit further comprise instructions for the intracerebroventricular, intrathecal, and/or intraocular administration of the therapeutic compositions of the present invention, in addition to the therapeutic formulation. In another aspect, the kit further comprises a flushing solution as described herein. In still another aspect, the kit further comprises a system for administering the formulation, comprising any or all of the following components: tubing, an in-line filter, a reservoir for implantation, and a catheter. In one aspect, the kit may comprise catheters, reservoirs, or other devices preloaded with the therapeutic formulations of the present disclosure. For example, catheters preloaded with about 100 mg of rhTPP1, about 200 mg of rhTPP1, or about 300 mg of rhTPP1, in a pharmaceutically acceptable formulation, are specifically contemplated. Alternatively, the kit may comprise catheters, reservoirs, or other devices that are refillable and appropriate amounts of the enzyme for refilling such devices.

In certain embodiments, kits of the present invention may comprise one or more of the following components: an extension line (e.g., product number 536040, Smiths Medical, Dublin OH), an in-line filter (e.g., product number FS116, Smiths Medical), a port needle (e.g., product number 21-2737-24, Smiths Medical), a syringe or two or more syringes (e.g., product number 309604, Becton Dickinson, Franklin Lakes, NJ) or a syringe needle or two or more syringe needles (e.g., product number 305196, Becton Dickinson).

The present disclosure will be more readily understood by reference to the following Examples, which are provided by way of illustration and are not intended to be limiting.

### Examples

The following Examples describe a formulation comprising rhTPP 1 for intracerebroventricular (ICV) administration and the results of administering the formulation to human patients compared to matched, untreated natural history patients.

### Example 1

### Formulation of rhTPP1 for Intracerebroventricular Administration

RhTPP 1 was produced in a genetically engineered CHO host cell line and purified by standard chromatography methods, as described in U.S. Patent No. 6,302,685 and Sleat et al. 1997, Science 277:1802-1805. The rhTPP1 was produced as an inactive proenzyme to be auto-activated at acidic pH upon uptake to the lysosome. The proenzyme form of rhTPP 1 has a calculated isotope average molecular weight of approximately 59 kDa. The mature enzyme has an apparent molecular weight of approximately 46 kDa. The amino acid sequence of the rhTPP1 proenzyme is set forth in SEQ ID NO:1 and shown in Figure 1. The pro-segment of the enzyme is the first 176 amino acid residues, and the mature enzyme is 368 amino acids in length starting at position 177 and is set forth in SEQ ID NO:2.

The rhTPP 1 formulation used in the Examples was a sterile solution for ICV infusion. It was a clear and colorless to pale yellow liquid containing rhTPP1 protein formulated at a concentration of 30 mg/mL. The formulation was packaged in a container closure system consisting of a Type 1 clear borosilicate glass vial closed with a fluoropolymer coated butyl rubber stopper and capped with aluminum seal. The formulation was stored at a temperature of -40 °C ± 10 °C and supplied frozen. The target pH value of the formulation was pH 6.5.

The composition of the rhTPP1 formulation used in the Examples is provided in Table 2.

**TABLE 2**

| **Component** | **Concentration (mg/mL)** | **Composition per vial** | **Function** | **Compendial Grade** |
|---|---|---|---|---|
| rhTPP1 | 30 | 30 mg | Active | NA |
| Sodium Phosphate Dibasic, Heptahydrate | 0.11 | 0.11 mg | Buffering Agent | USP/Ph.Eur |
| Sodium Phosphate Monobasic Monohydrate | 0.08 | 0.08 mg | Buffering Agent | USP/Ph.Eur |
| Sodium Chloride | 8.77 | 8.77 mg | Isotonicity | USP/Ph.Eur/JP |
| Potassium Chloride | 0.22 | 0.22 mg | Maintain the level of key CSF electrolyte | USP/Ph.Eur/JP |
| Magnesium Chloride Hexahydrate | 0.16 | 0.16 mg | Maintain the level of key CSF electrolyte | USP/ Ph.Eur |
| Calcium Chloride Dihydrate | 0.21 | 0.21 mg | Maintain the level of key CSF electrolyte | USP/Ph.Eur/JP |
| Water for Injection (qs) | NA | 1.00 mL | Diluent | USP/Ph.Eur/JP |

The rhTPP1 formulation was carefully designed to mimic characteristics of human CSF, such as the concentrations of key electrolytes are similar to those found in human CSF *in vivo* and the formulation did not contain any conventional preservatives or stabilizers as excipients. No significant safety issues, i.e., serious adverse reactions, were reported or observed following administration of the rhTPP1 formulation, which could not have been previously predicted.

Stability studies were conducted at long-term (≤ -60°C) and accelerated conditions (5 ± 3 °C) in accordance with ICH guidelines and per protocol to monitor the time-temperature stability. Stability samples were stored in small-scale bottles composed of the same materials as the full-scale packaging. Stability data collected on supportive and clinical batches demonstrated that the rhTPP 1 formulation was stable at ≤ -60°C for at least 36 months and at 5 ± 3 °C for at least 6 months, which was surprising considering the formulation lacked preservatives and stabilizers commonly found in pharmaceutical products. Table 3 shows the results of the stability testing.

**TABLE 3**

| Test | Specification | Storage | Time Point (months) | | | | | | | |
|---|---|---|---|---|---|---|---|---|---|---|
| | | | 0 | 3 | 6 | 9 | 12 | 18 | 24 | 36 |
| **QUALITY** | | | | | | | | | | |
| Appearance | Colorless to pale yellow liquid | Long-term Accelerated | X | X | X | X | X | X | X | X |
| | | | X | X | X | | | | | |
| Sialic Acid | 2 - 7 mol/mol | Long-term Accelerated | X | | X | | X | X | X | X |
| | | | X | | X | | | | | |

| **POTENCY** | | | | | | | | | | |
|---|---|---|---|---|---|---|---|---|---|---|
| Specific Activity, Acid Activated | 5-15 U/mg | Long-term Accelerated | X | X | X | X | X | X | X | X |
| | | | X | X | X | | | | | |
| Specific Activity Without Acid Activation | ≤ 0.075 U/mg | Long-term Accelerated | X | X | X | X | X | X | X | X |
| | | | X | X | X | | | | | |
| Oligosaccharide Profile | Comparable to reference | Long-term Accelerated | X | X | X | X | X | X | X | X |
| | | | X | X | X | | | | | |
| | 10 - 26 % bis-mannose-6-phosphate oligomannose₇ | | | | | | | | | |
| Cellular Uptake | ≤ 10 nM | Long-term Accelerated | X | | X | | X | X | X | X |
| | | | X | | X | | | | | |

| **STRENGTH** | | | | | | | | | | |
|---|---|---|---|---|---|---|---|---|---|---|
| Protein Concentration | 27-33 mg/mL | Long-term Accelerated | X | X | X | X | X | X | X | X |
| | | | X | X | X | | | | | |

| **PURITY** | | | | | | | | | | |
|---|---|---|---|---|---|---|---|---|---|---|
| Related Substances by RP-HPLC | Report Result (% Main Peak) | Long-term Accelerated | X | X | X | X | X | X | X | X |
| | | | X | X | X | | | | | |
| Molecular Size Variants | ≥ 95% Monomer | Long-term Accelerated | X | X | X | X | X | X | X | X |
| | | | X | X | X | | | | | |
| Charge Heterogeneity | Comparable to reference | Long-term Accelerated | X | X | X | X | X | X | X | X |
| | | | X | X | X | | | | | |
| | ≥ 95 % Main Peak | | | | | | | | | |
| Related Substances | Comparable to reference | Long-term Accelerated | X | X | X | X | X | X | X | X |
| | | | X | X | X | | | | | |

| **COMPOSITION** | | | | | | | | | | |
|---|---|---|---|---|---|---|---|---|---|---|
| pH | 6.0-7.0 | Long-term Accelerated | X | X | X | X | X | X | X | X |
| | | | X | X | X | | | | | |
| Osmolality | 270-330 mOSm/Kg | Long-term Accelerated | X | X | X | X | X | X | X | X |
| | | | X | X | X | | | | | |

### Example 2

### Natural History Study

The quantitative assessment of CLN2 natural history disease progression was analyzed in a cohort of 41 untreated CLN2 patients. The Hamburg clinical scale was used for the assessment of age-appropriate neurological and functional domains affected by disease.

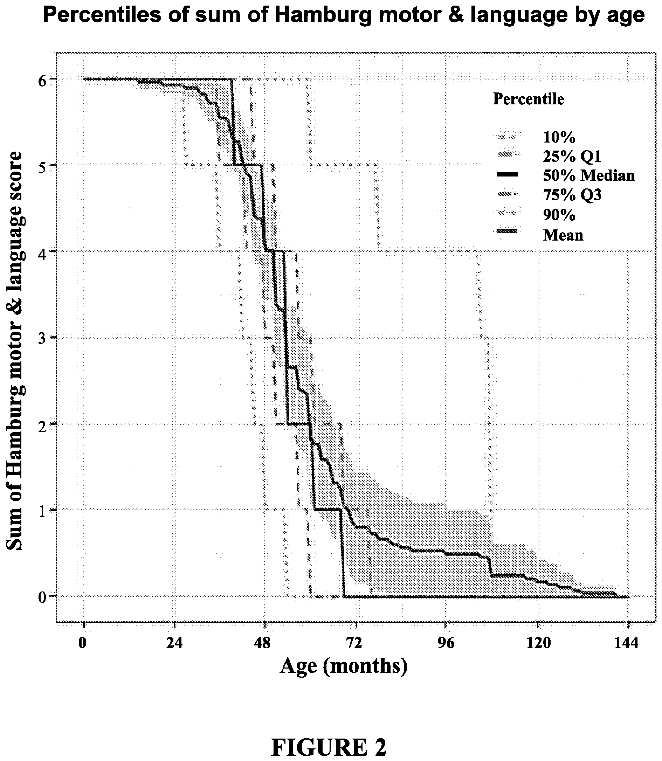

The quantitative description of the clinical decline in the untreated natural history CLN2 subjects is shown in Figure 2. The natural history analysis demonstrated a clear and predictable relationship of age to disease severity. After the onset of motor and language symptoms, there was essentially a rapid linear decline in which children, on average, lost about 2 milestone events each year (linear rate of decline 2.1 points per year). There was a largely predictable course, however, there were some 'late onset' cases which made up less than 20% of the population in the cohort. These patients tended to have later onset of symptoms and a longer period of mild disease, but then succumbed to rapid and active decline, typically 2 to 3 years later than the classic form.

Quantitative clinical progression from the Hamburg cohort was corroborated by superimposing the clinical ratings assessments from an independent (patients and raters) cohort from WCMC (n = 49). Although clinical descriptions of independent CLN2 cohorts are similar, this was the first confirmation of strong quantitative relationship in disease progression in separate patient groups. Both cohorts of CLN2 patients had a large majority of classic late infantile onset and progression, and a smaller proportion of children that had a 'late onset' phenotype, usually having early manifestations of disease at age 5 years rather than at 3 years. The scale using motor (gait) and language function reproducibly captured the neurologic decline of CLN2 patients. Based on the foregoing analyses, the cohort of natural study subjects was determined to be an appropriate non-treatment control population, and the average rate of decline of a symptom of CLN2 disease in this untreated, natural history population can be used as an effective and informative comparator for any prevention or reduction in the rate of decline of a symptom in a subject suffering from CLN2 disease caused by administration of a composition of the present invention.

### Example 3

### Phase 1/Phase 2 Open Label Dose-Escalation Study in CLN2 patients

The study was an open label treatment clinical trial to evaluate the safety, tolerability and efficacy of a rhTPP 1 formulation of the disclosure delivered to children with CLN2 disease through an ICV catheter at a dose of 300 mg (10 mL total volume) every other week. The study was designed to assess safety and tolerability starting at low doses (30 mg and 100 mg), but all patients escalated to the high expected therapeutic dose (300 mg) when the lower doses were noted by an independent data monitoring committee to be safe. The study duration for all enrolled patients was 48 weeks of treatment at the stable expected therapeutic dose of 300 mg ICV every other week. The primary study objectives were to evaluate safety and tolerability of a rhTPP 1 formulation of the disclosure administered to subjects with CLN2 disease by an implanted ICV reservoir and cannula and to evaluate effectiveness using a CLN2 disease-specific rating scale score in comparison with natural history data after 12 months of treatment. The secondary study objectives were to evaluate the impact of treatment on measurement of brain atrophy in comparison with CLN2 disease natural history data after 12 months of treatment.

The major inclusion criteria were a CLN2 diagnosis and an enrollment age of at least 3 years old. Patients having a baseline disease rating score less than 3 at the time of screening (using the Hamburg 0 to 6 aggregate motor/language scale) were excluded from the study. Patients less than 3 year old were likely to not progress due to age rather than treatment, as depicted by the horizontal line on the progression curve. Patients with a score at screening of 2 or less were also less linear, more variable and considered potentially more refractory to treatment due to the stage of disease. Thus, the treatment group was simply defined by age and score to include early and highly predictable decline.

Mean age at enrollment was 4.0 years old, slightly more girls than boys, predominantly Caucasian. The clinical CLN2 score at screening and baseline is shown in Table 4 below, which shows the Hamburg motor/language score for each study cohort and totals at both screening and baseline.

**TABLE 4**

| **CLN2 Ratings - 6 point Hamburg Scale - Last Assessment within Each Dosing Period** | | | | | |
|---|---|---|---|---|---|
| **Analysis Population: Intent-to-treat** | | | | | |
| **Analysis Dataset: Entire Dosing Period** | | | | | |
| | **Cohort 1 (n = 3)** | **Cohort 2 (n = 3)** | **Cohort 3 (n = 3)** | **Stable Dosing Only (n = 14)** | **Overall (n = 23)** |
| **Screening** | | | | | |
| **6** | **1 (33%)** | **0** | **1 (33%)** | **0** | **2 (9%)** |
| **5** | **0** | **0** | **0** | **2 (14%)** | **2 (9%)** |
| **4** | **0** | **0** | **0** | **6 (43%)** | **6 (26%)** |
| **3** | **2 (67%)** | **3 (100%)** | **2 (67%)** | **6 (43%)** | **13 (57%)** |

| **Study Baseline** | | | | | |
|---|---|---|---|---|---|
| **6** | **1 (33%)** | **0** | **1 (33%)** | **0** | **2 (9%)** |
| **5** | **0** | **0** | **0** | **2 (14%)** | **2 (9%)** |
| **4** | **0** | **0** | **0** | **5 (36%)** | **5 (22%)** |
| **3** | **2 (67%)** | **3 (100%)** | **1 (33%)** | **6 (43%)** | **12 (52%)** |
| **2** | **0** | **0** | **1 (33%)** | **1 (7%)** | **2 (9%)** |
| **1** | **0** | **0** | **0** | **0** | **0** |
| **0** | **0** | **0** | **0** | **0** | **0** |

Overall, there was a skew in the pre-treatment CLN2 scores towards more advanced disease. Given the rapid progression of the disease and the ascertainment difficulties, a skew towards the lower scores was expected. Further, there was some decline in the score at screening and in the period (up to two weeks) to the baseline assessment (just before placement of the ICV reservoir). Four patients from the screening group that scored 3 slipped a point at baseline, and two patients in the screening group that scored a 4 lost a point to 3 at baseline. The two patients that entered as a 6 (i.e., grossly normal) were siblings of affected children. The disposition, demographics, and characteristics of the subject population are summarized in Table 5 below.

**TABLE 5**

| | | Overall |
|---|---|---|
| Disposition | Subjects enrolled | 24 |
| | Subjects treated | 24 |
| | Subjects completed | 23 |
| | Subjects discontinued | 1^{a} |
| Age (years) | Mean (SD) | 4.3 (1.24) |
| | 6 | 2 |
| | 5 | 2 |
| Baseline CLN2 score | 4 | 6 |
| Sum of Motor/Language | 3 | 12 |
| | 2 | 2 |
| | Mean (SD), Median | 3.6 (1.06), 3.0 |
| Genotype^{b} | Common. | 9 (37.5%) |
| | Common x Other | 8 (33.3%) |
| | Other | 7 (29.2%) |

| | | |
|---|---|---|
| ^{a} Enrolled patient 1287-1007 had a single dose and withdrew consent due to inability to comply with study procedures ^{b} Similar distribution to natural history population *Common Genotypes: c.622C>T and c.509-1G>C | | |

All patients enrolled received stable dosing of 300 mg ICV every other week. Cohort 1 was exposed to ≥ 1 month at 30 mg ICV every other week, then ascended to 100 mg ICV every other week for ≥ 4 weeks, while Cohort 2 was started on 100 mg ICV every other week for ≥ 4 weeks. Both Cohort 1 and Cohort 2 ascended to 300 mg ICV every other week, and all subsequent patients including Cohort 3 initiated dosing at the stable dose regimen of 300 mg ICV every other week and continued for ≥ 48 weeks. The 300 mg dose was administered in 10 mL infused over a period of about 4 hours via an ICV catheter. A volume of CSF, e.g., equivalent to the amount of the rhTPP1 formulation to be administered, was not removed just prior to the start of the infusion, which was atypical, but surprisingly did not cause any adverse effects. Immediately following administration of the 300 mg dose, a flushing solution in an amount of about 2 mL was administered to the subject via the same ICV catheter. The flushing solution was identical to the formulation in Table 2, but did not contain rhTPP1. The bolus dose of 300 mg of enzyme per administration event was significantly higher than previous intrathecal or ICV administered enzyme replacement therapies and, as such, the safety and efficacy profiles observed after administration of such a high dose of drug could not have been previously predicted. More specifically, administration of a 300 mg bolus dose of rhTPP1 without associated serious, unmanageable adverse events could not have been previously predicted.

### Results

*Effect of Treatment on Clinical Assessments of Gait and Language:* The primary assessment tool for the quantitative evaluation of clinical severity was the 0 to 6 point aggregate of the gait and language subscales common to both the Hamburg and WCMC disease rating scales. This scale captured the predictable, rapid, and progressive clinical decline in matched, untreated natural history patients that was used as a comparator for the primary efficacy analysis.

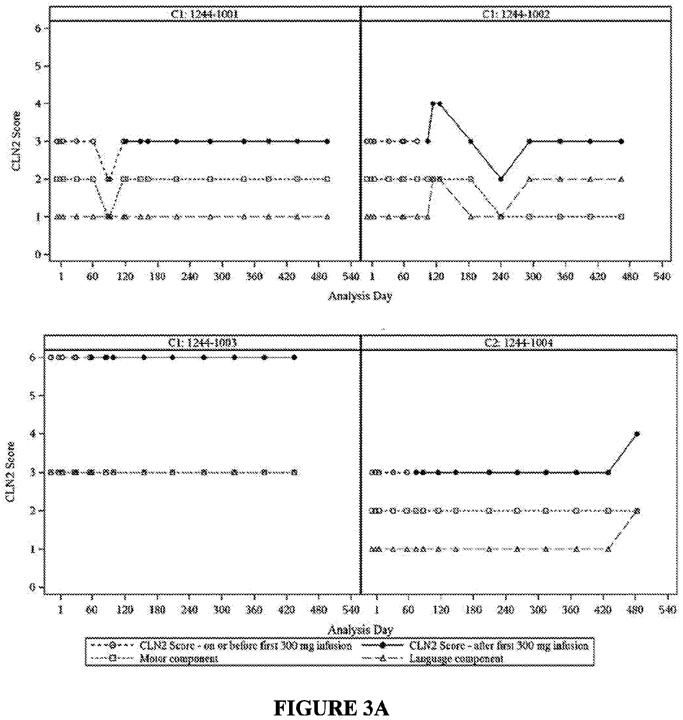

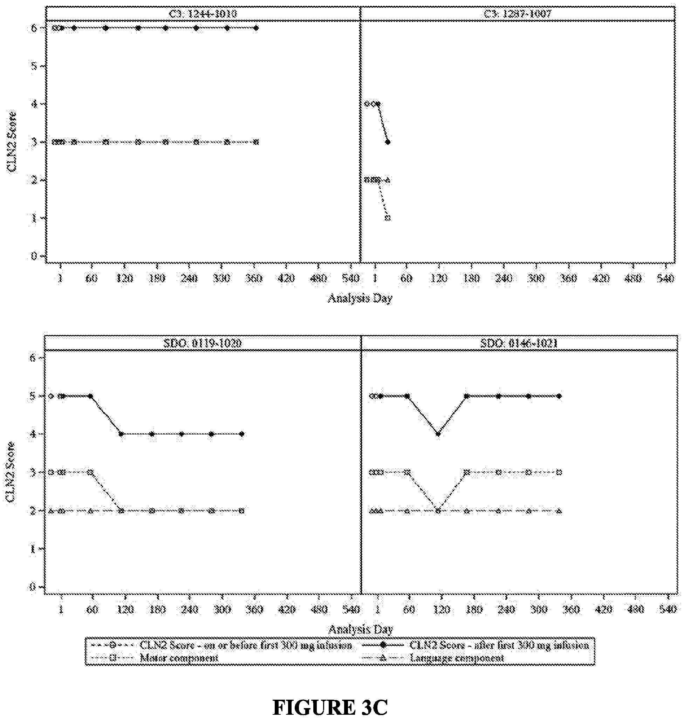

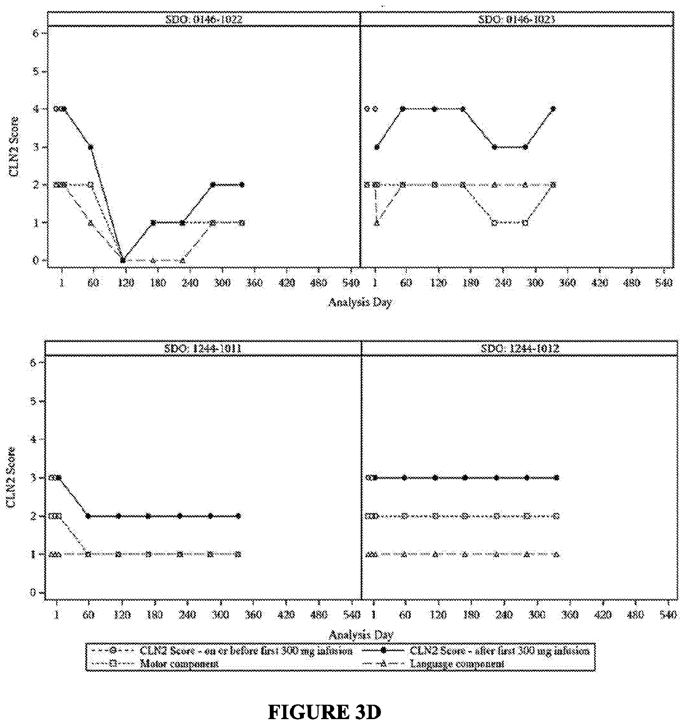

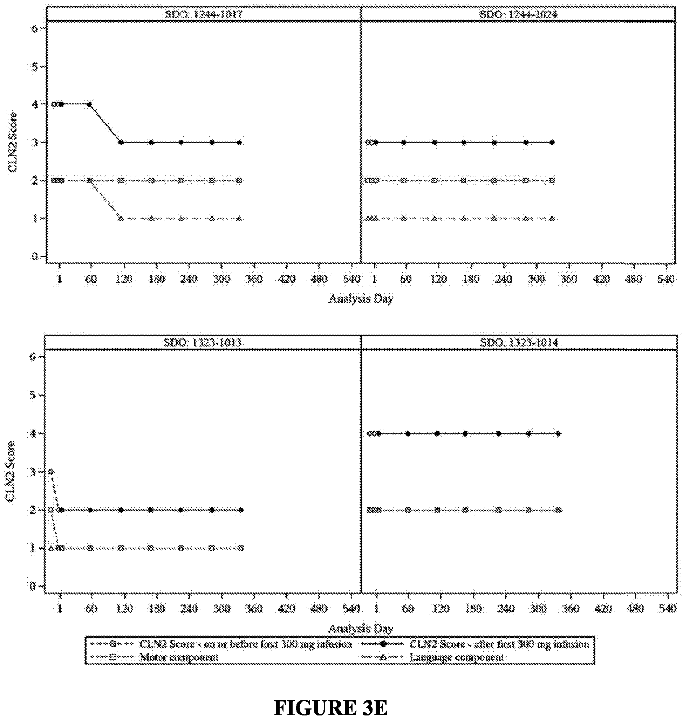

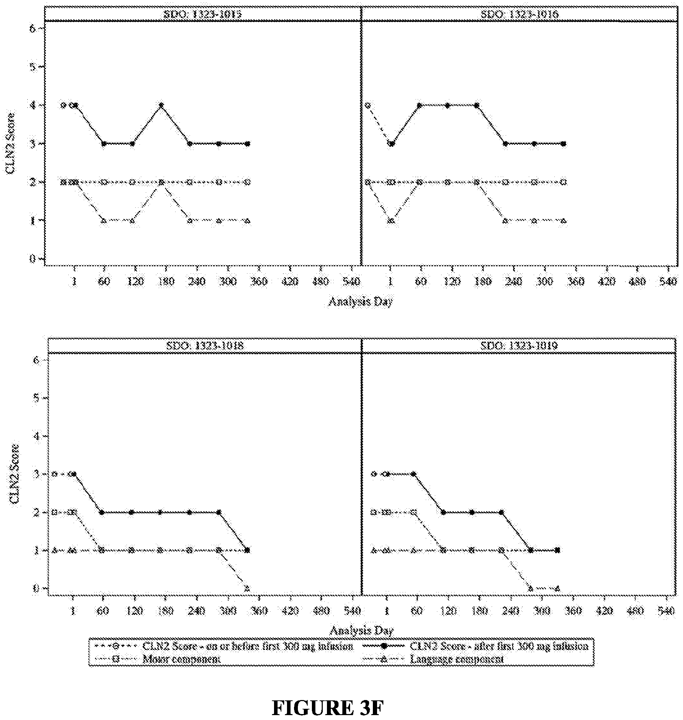

The gait/language disease rating score for 23 patients with treatment duration of more than 42 weeks is shown in Figures 3A to 3F. Of the 23 patients, 3 patients (1244-1001, 1244-1002, and 1244-1003) were from Cohort 1 (C1), 3 patients (1244-1004, 1244-1006, and 1287-1005) were from Cohort 2 (C2), 3 patients (1244-1008, 1244-1009, 1244-1010) were from Cohort 3 (C3), and 14 patients (0119-1020, 0146-1021, 0146-1022, 0146-1023, 1244-1011, 1244-1012, 1244-1017, 1244-1024, 1323-1013, 1323-1014, 1323-1015, 1323-1016, 1323-1018, and 1323-1019) were from the 300 mg Stable Dosing Only (SBO) group. As expected, language deficit was typically more advanced than gait deficit. Entry scores were not randomly distributed; 12 patients had significant disease progression with a combined entry score of 3 points, and 2 patients had a combined entry score of 6 points. Given rapid progression and disease ascertainment, children frequently present with evident decline or as siblings of those with evident decline.

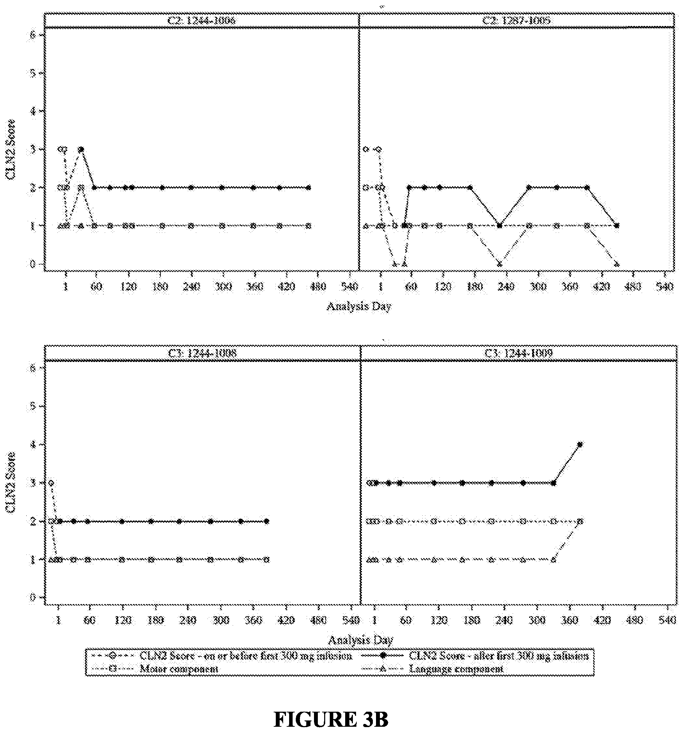

Following treatment with a rhTPP1 formulation of the disclosure (shown in Table 2 above), the CLN2 gait/language disease rating score was stabilized, as shown in Figures 3A to 3F. Eleven of 23 patients had no unreversed decline over the treatment period. Four patients had a single unit decline early in the treatment period, but no un-reversed decline thereafter. Two patients (1244-1008 and 1323-1013) decreased by one unit from 3 to 2 points between screening and baseline, but did not experience any additional loss in ratings while on treatment. Based on the results, there was evident treatment benefit in all patients regardless of cohort (starting dose) or entry score. In a number of patients, there were reversed ratings drops. For example, Patient 1287-1005 (Figure 3B) had a rating decrease of 2 units in the first month of treatment, which represented a loss of function for both gait and language. However, this patient regained a unit at treatment day 60, and had no net change afterwards until analysis day 440. The regained score was the acquisition of language, underscoring the clinical importance of single unit changes.

Neither of the 2 patients with a score of 6 at entry lost a rating unit. Seven of the 12 patients with an entry score of 3 had no unreversed decline, and 2 were stable after an initial single unit decline. Thus, treatment benefit was evident in patients with significant deficits and disease progression.

As demonstrated in the CLN2 natural history study, the median rate of decline in the untreated natural history population was estimated at 2.1 units each year. Thus, all patients in the treatment group had improved ratings compared to the expected outcomes of the untreated natural history population.

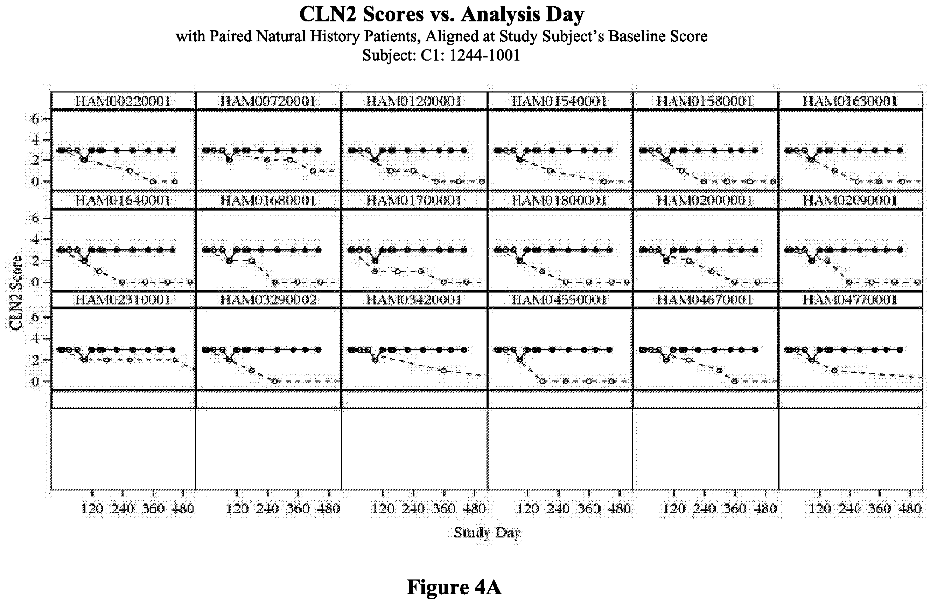

To establish a clearer relationship in the disease course between treated and matched, untreated natural history patients, each study patient was matched with untreated natural history patients by the parameters of baseline CLN2 score, age and genotype. Although there are no clear subgroups or factors predictive of progression in CLN2 disease, these parameters are most commonly used to define disease severity. Individual treated patients were compared to each member of the natural history cohort that had similar gait/language rating score at baseline, as shown in Figures 4A to 4I. Patients in the study were matched by baseline CLN2 score, as follows: for a given study patient with a given baseline score, all natural history patients who reported one or more CLN2 evaluations with that same CLN2 score were identified. If the study patient's baseline CLN2 score was 2, 3, 4, or 5, then each natural history patient's CLN2-vs-time profile was time-shifted left or right so that it overlaid the study patient's baseline score. If the natural history patient had multiple assessments equaling the study patient's baseline CLN2 score, then the mid-time point of the multiple assessments was used for time-shifting. If the study patient's baseline CLN2 score was 6 points, then the natural history patient's last score of 6 points was used for time-shifting. Sensitivity analyses were conducted using other matching criteria, and results from these analyses were consistent with the score-matched analyses.

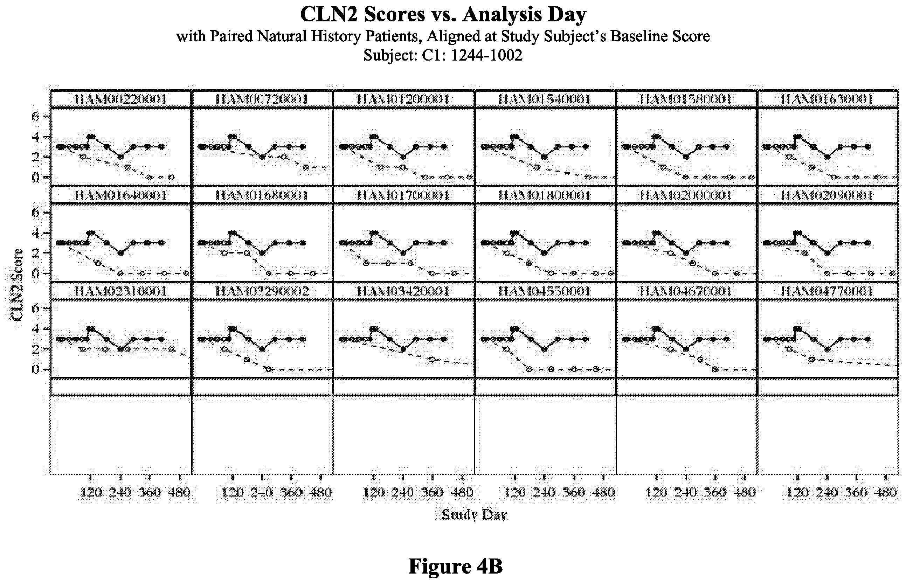

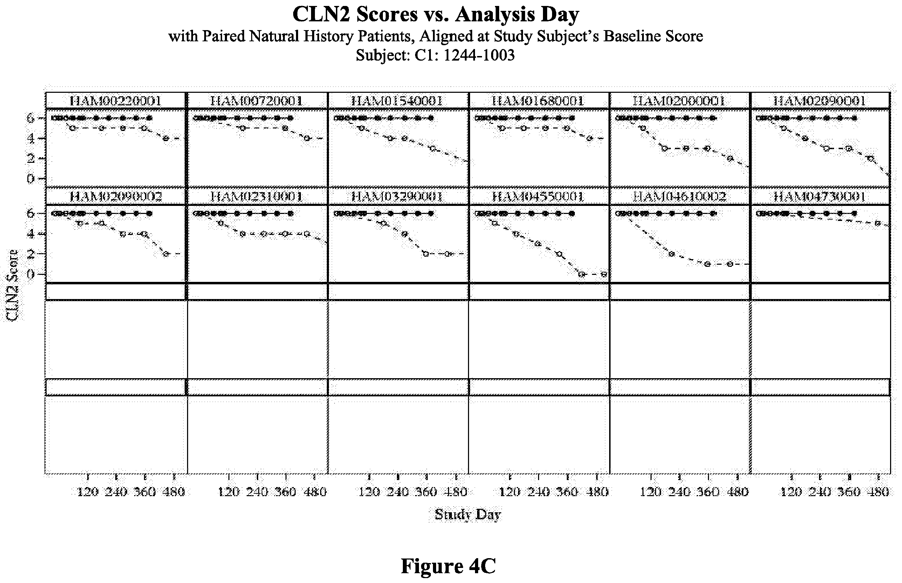

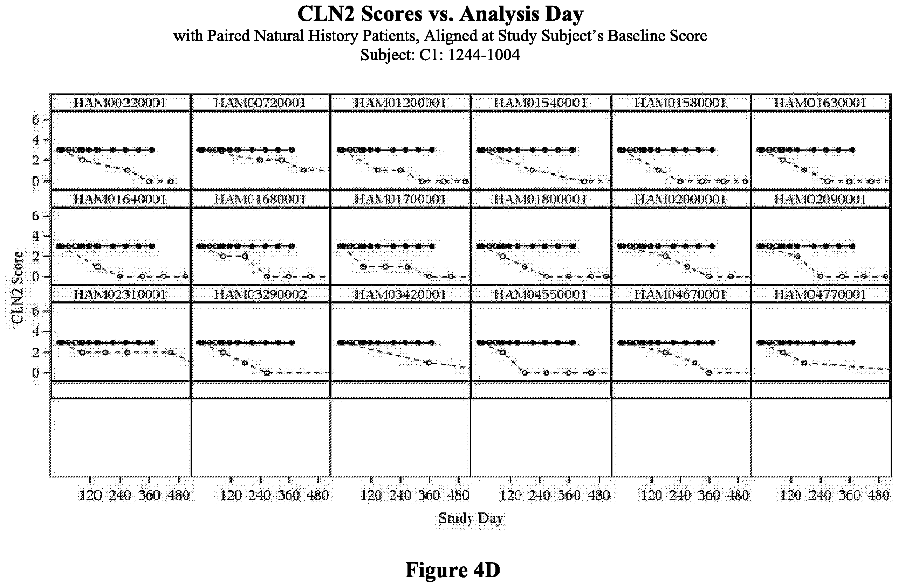

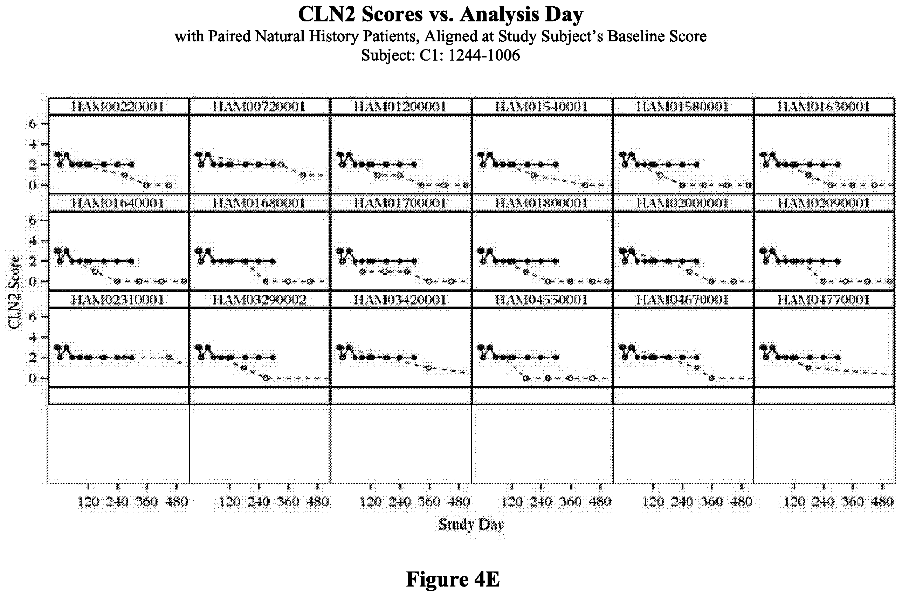

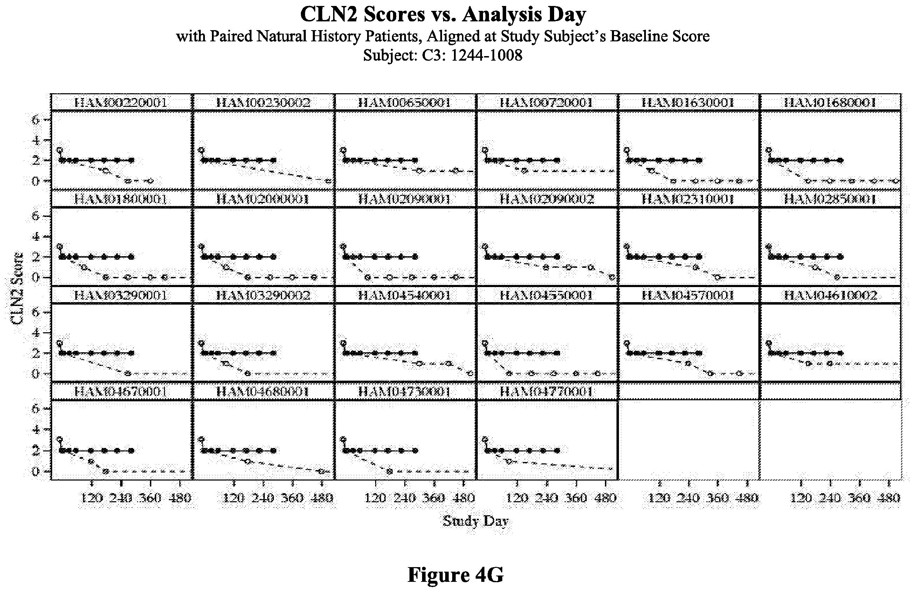

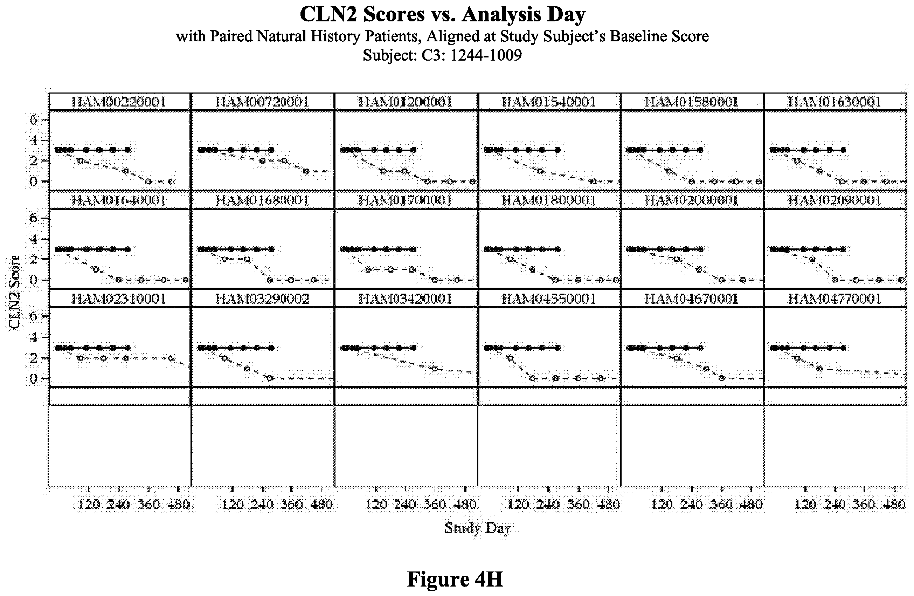

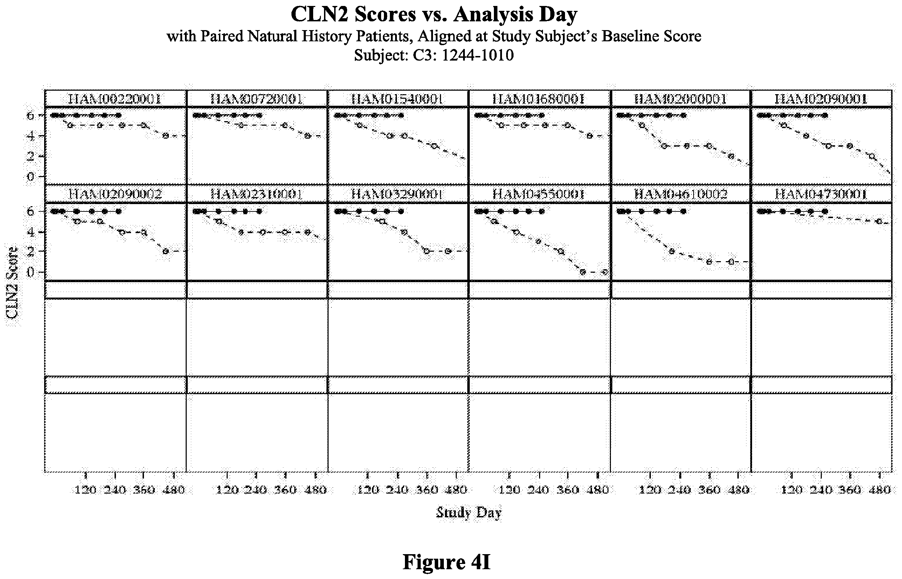

Figures 4A to 4I show results from subjects treated with a rhTPP 1 formulation of the disclosure plotted against matched, untreated natural history patients. The treated subjects and untreated natural history patients were matched by disease rating score using the 0 to 6 unit gait and language subscales as an aggregate. The ratings of each were compared over the period of one year of treatment. There was a treatment benefit for the subjects that received rhTPP 1 compared to all members of the matched, untreated natural history patient group. Subject 1244-1001 (Figure 4A) had a rating decrease from 3 units to 2 units after 120 days of treatment, but regained a unit and had no net change afterwards. Subject 1244-1002 (Figure 4B) had a rating increase from 3 units to 4 units, a decrease from 4 units to 2 units, and an increase from 2 units to 3 units, resulting in overall maintenance of the disease rating at the end of the study compared to Day 1. Subjects 1244-1003 (Figure 4C) and 1244-1010 (Figure 4I) maintained a rating of 6 units, i.e., normal motor and language function, throughout the study. Subjects 1244-1004 (Figure 4D) and 1244-1009 (Figure 4H) maintained a rating of 3 units throughout the study. Subject 1244-1006 (Figure 4E) had a rating decrease from 3 units to 2 units initially, but regained a unit before decreasing again from 3 units to 2 units with no net changes afterwards. Subject 1244-1008 (Figure 4G) had a rating decrease from 3 units to 2 units initially, with no net changes afterwards.

In contrast to all of the treated subjects, the majority of their matched, untreated natural history patients had an unreversed rating decrease from 3 units to 0 units by the end of the comparative period, indicating a progression to complete lack of function for gait and language combined. The matching analysis demonstrates the treatment benefit for those patients that maintain their disease rating score and also that have an initial ratings drop, but subsequently stabilize.

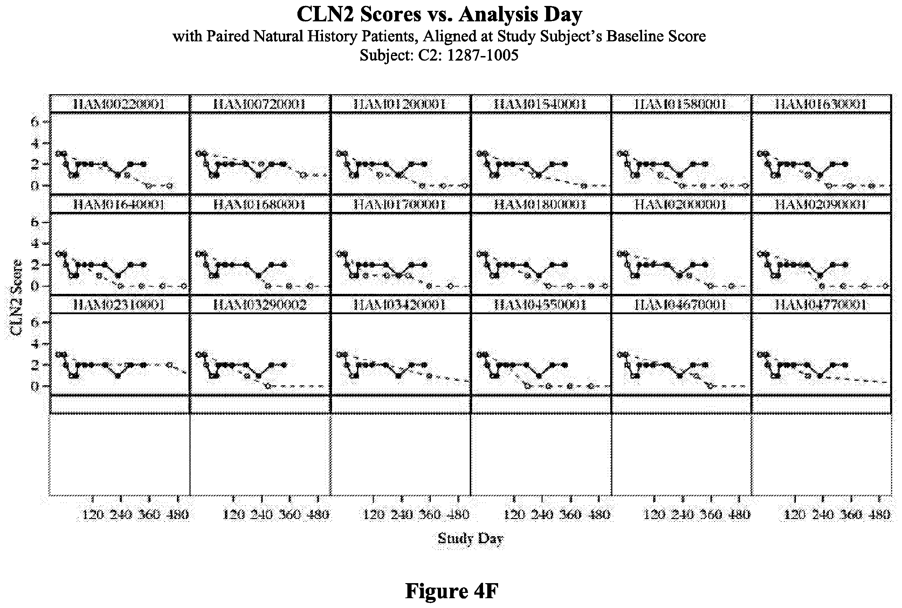

The most complicated response (Subject 1287-1005) is shown in Figure 4F. Although this patient dropped rapidly by 2 units from a baseline score of 3 points to a study score of 1 in the first month of the study, the patient was able to regain a unit and stabilize at a score of 2 points. The interpretation of this course was clarified by comparison to score-matched untreated natural history patients. The clinical progress was worse in 15 of the 18 score-matched untreated natural history patients, and the same in just 2 score-matched untreated natural history patients (a single untreated natural history match was non-evaluable). The clinical course in untreated patients always was worsening, frequently with little time between lost milestones. There was never re-establishment of lost function and subsequent stabilization. Matching to the most complex treated profile was also indicative, therefore, of a clear treatment benefit.

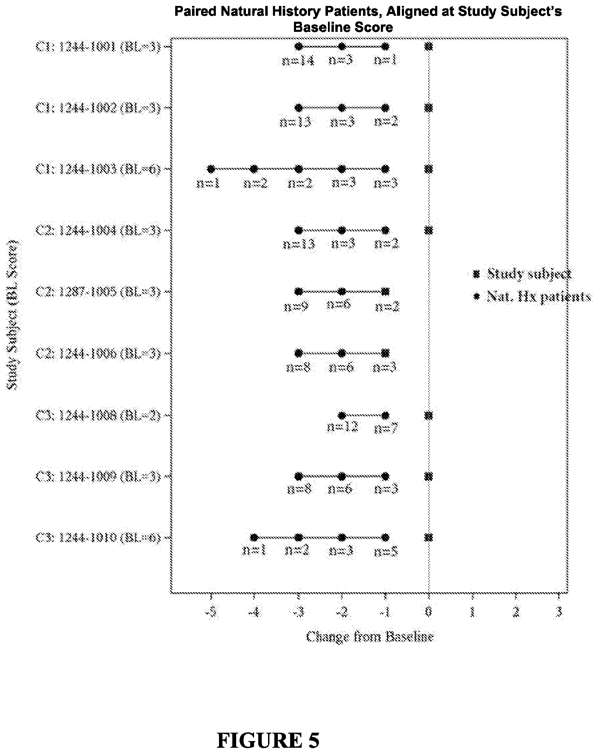

Figure 5 displays the distribution of clinical change from baseline in matched, untreated natural history patients for the treatment duration of the patient match compared to the study subjects. As previously stated, 7 of 9 (> 75%) patients had no change in baseline disease rating scale. For those 7 patients in the treatment period, all matched untreated natural history patients had at least a single unit decline, but more common was a multiple unit decline or 2 units to 4 units. As an example, Patient 1244-1001 had 1 match that lost a single point, 3 matches that lost 2 points and 14 matches that lost all 3 available language/gait disease rating points. Therefore, in the same period of time, there was no change in the treated patient, but 14 of 18 (> 75%) matched untreated natural history patients lost all gait and language function. There was a floor effect with a baseline entry score of 3 points in which many matched untreated natural history patients lost all available rating units, however, the 2 patients with entry scores of 6 points (Patients 1244-1003 and 1244-1010) importantly showed these matches also were actively deteriorating, some with 4 and 5 point declines in the treatment period. This observation was a clear clinical demonstration of substantial treatment effect; most treated children retained entry clinical ratings in the context of untreated natural history matches that actively lost language and independent gait in the same time period, many to complete loss of function. The remaining 2 treated patients that lost a single point (Subjects 1287-1005 and 1287-1006) still had better clinical ratings than the vast majority of matches in the treatment period. In total, of the score-matched untreated natural history patients, 97% had worse ratings than the treated subjects.

Using multiple matching criteria (e.g., baseline, age, and genotype), nearly 100% of comparisons showed a favorable treatment effect compared to matched untreated natural history patients. The average treatment difference across matching criteria in all treated patients as compared to natural history patients ranged from 1.9 to 2.1 points depending on the matching criteria utilized.

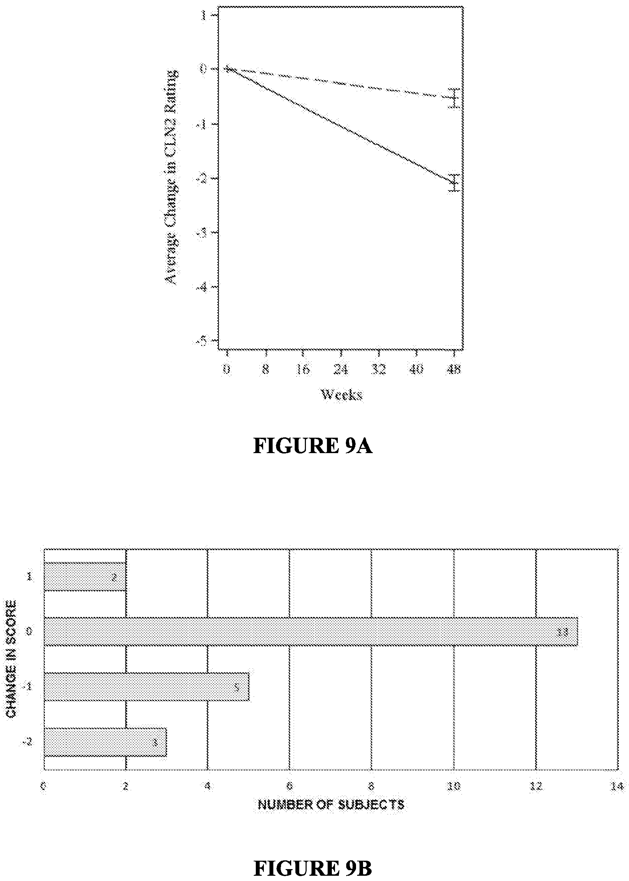

Figure 9A shows the average change in motor-language rating for patients treated for ≥ 48 weeks (n = 21; dashed line) and the natural history cohort (n = 41; solid line). The mean decline in the disease rating for the treated patients was 0.43 (standard deviation 0.839), with a median decline of 0.00 units over 48 weeks. In contrast, the mean decline in the disease rating for the natural history cohort was 2.09 (standard deviation 0.966), with a median decline of 1.87 units over 48 weeks. Overall, there was significant sparing (p<0.0001) of 79% of the expected clinical decline for the treated patients. Figure 9B shows the change in motor-language scores for patients (n = 23) from the last measurement before the first 300 mg dose (baseline) to the last 300 mg dose at ≥ 48 weeks. Overall, 65% (15 out of 23) of patients either improved or had no clinical disease progression during treatment, and 87% (20 out of 23) of the patients performed better during treatment (i.e., had a change of score of -1 or greater) compared to untreated subjects from the natural history study. These analyses uniformly supported the conclusion that there was a dramatic and clinically meaningful stabilization of CLN2 score in treated patients in comparison to matched members of the untreated natural history group, which rapidly and predictably decline.

*Effect of Treatment on Clinical Assessments of Vision:* In untreated CLN2 patients, vision loss occurs later than the decline in language and gait, however once symptomatic, the course is predictably rapid and progresses to blindness. Thus, preservation of vision is an important treatment outcome. Loss of vision can be captured on a 0 to 3 point subscale in a similar fashion to other subscales, in which 3 is normal and 0 is functionally blind. There was no unreversed loss in the vision subscale domain for the majority of the treated patients during the treatment period. When the treated patients were matched to untreated natural history subjects by score, age and genotype using the scale composites of the gait, language and vision subscales (0 to 9 units), it was clear that untreated matched natural history patients lost additional points in comparison to the treated group.

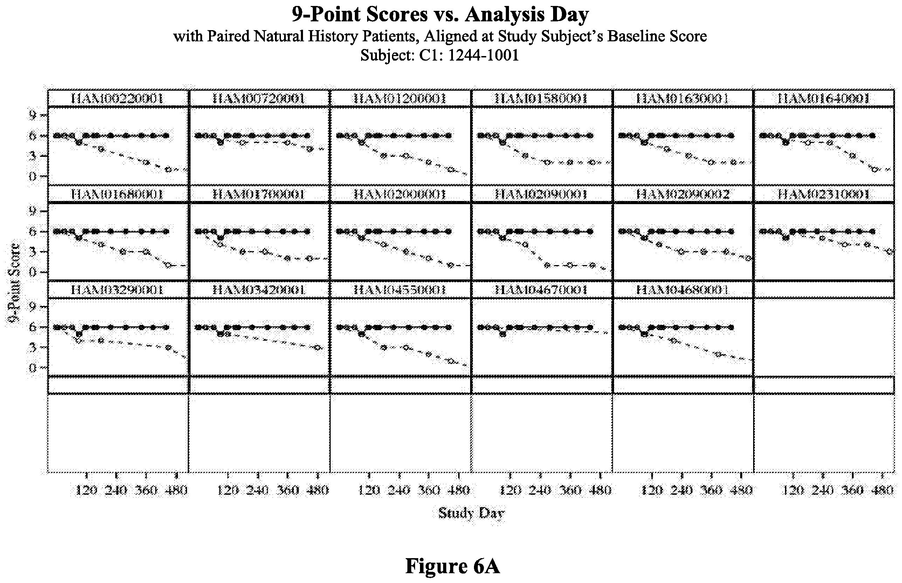

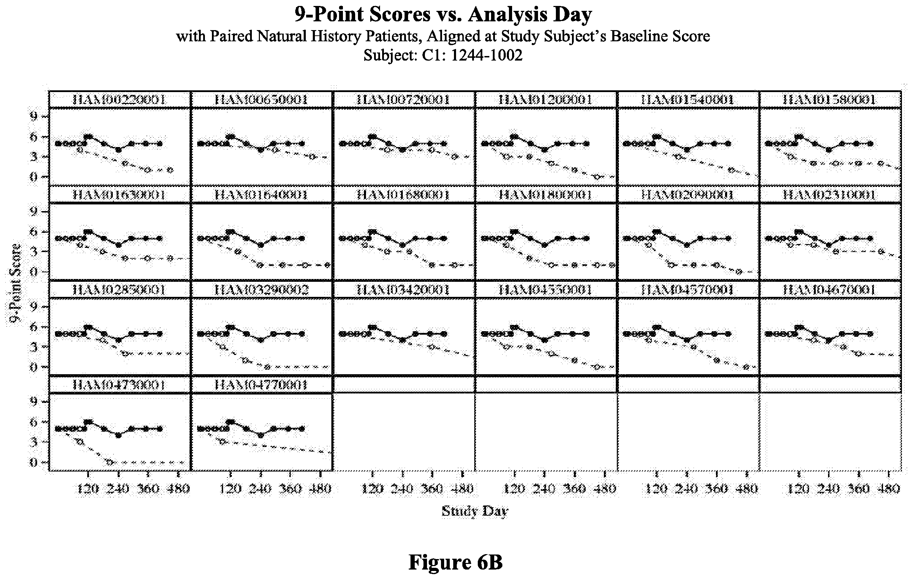

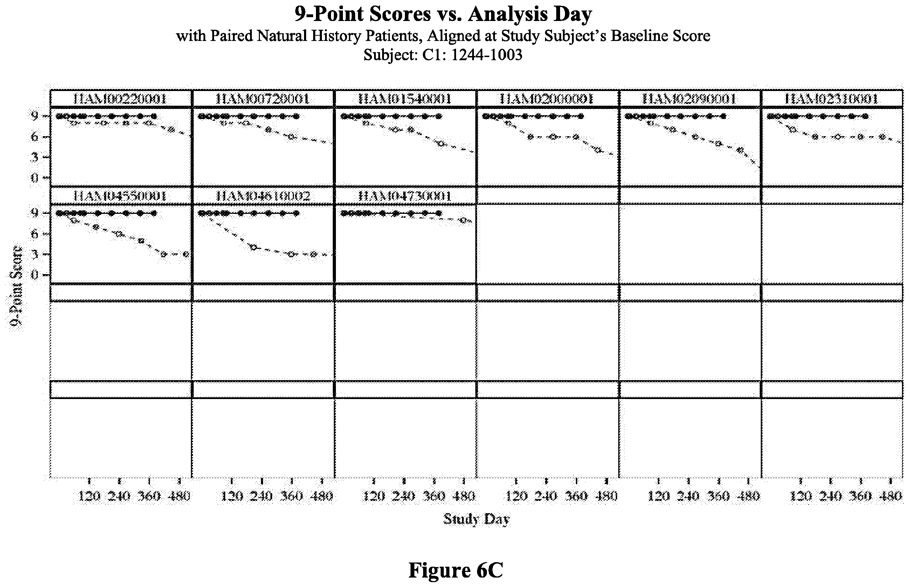

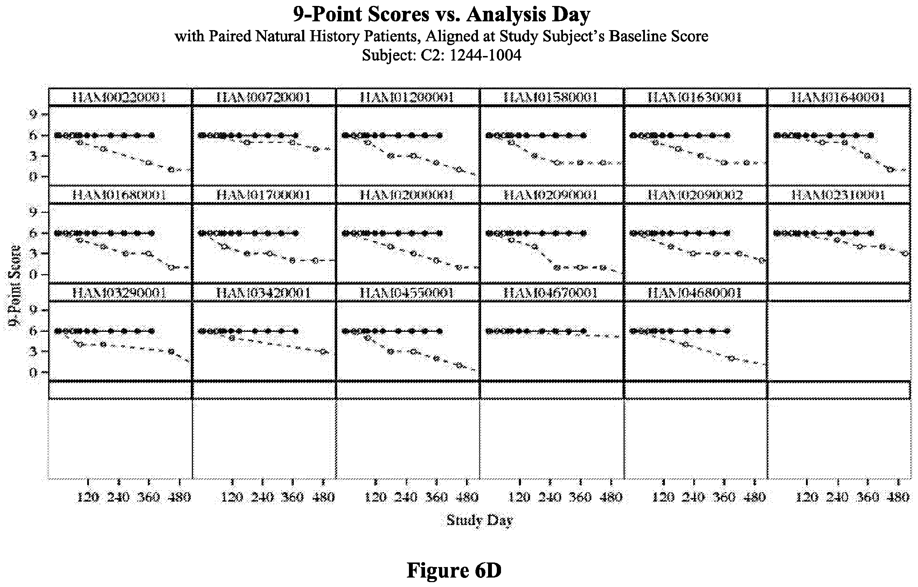

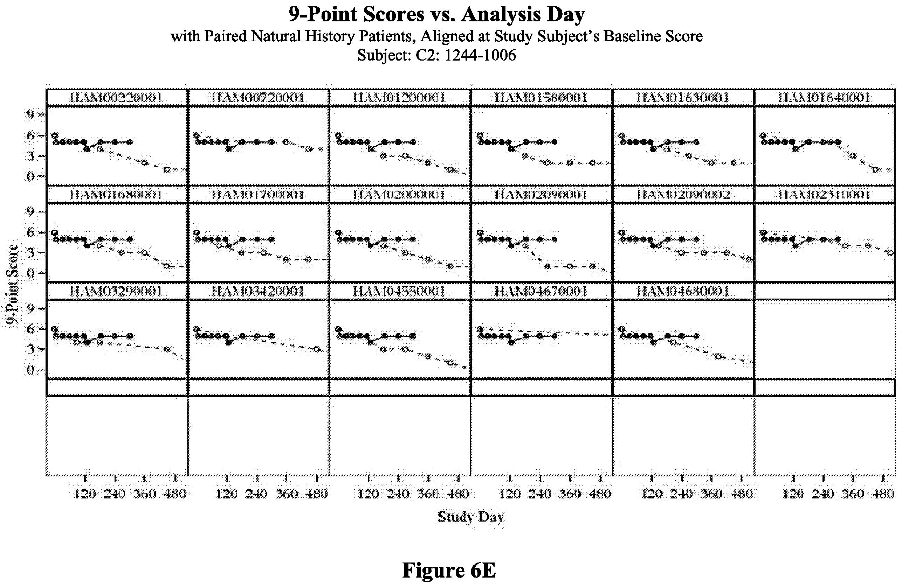

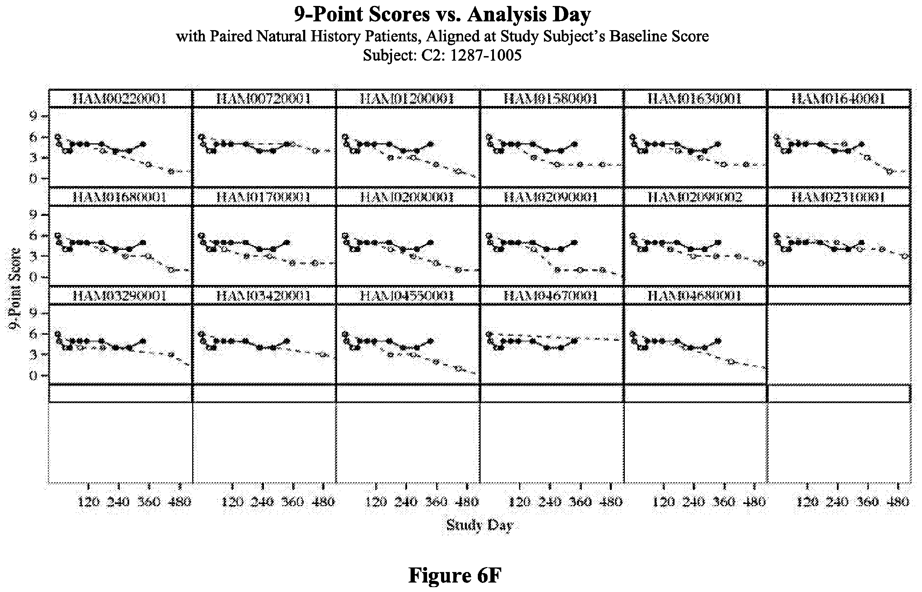

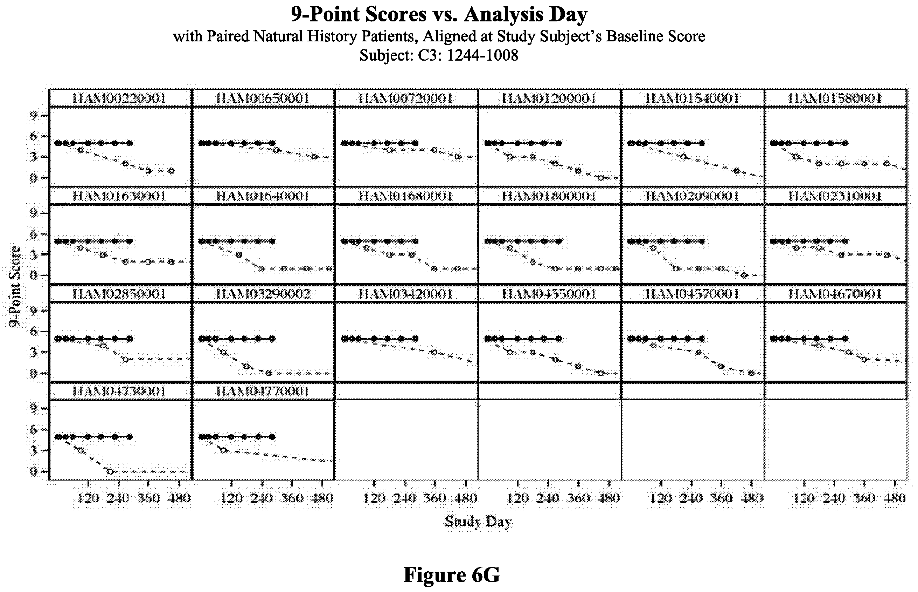

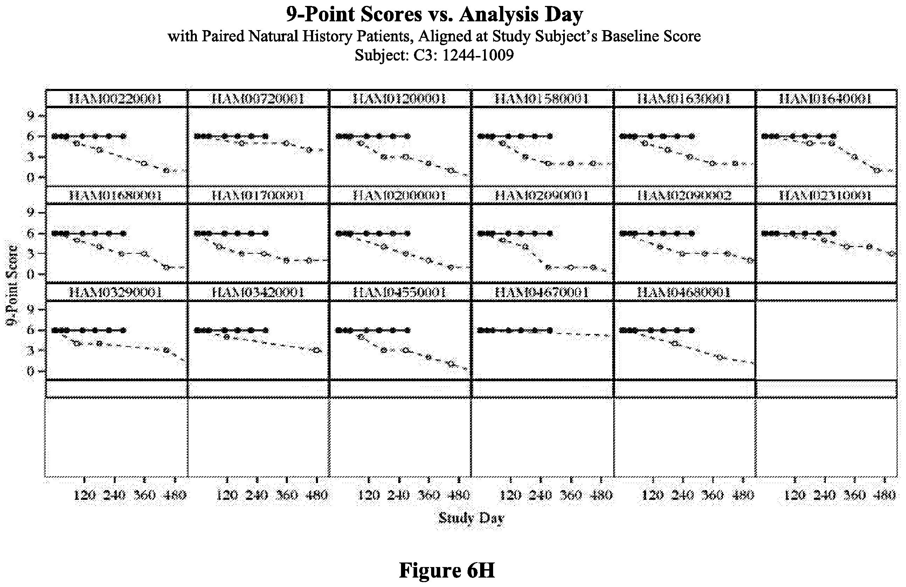

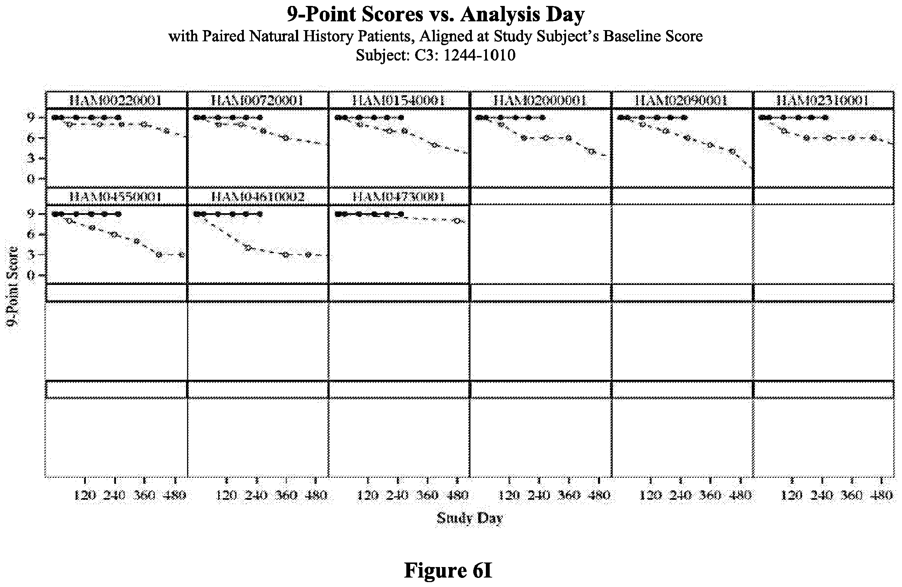

Figures 6A to 6I show results from nine subjects treated with a rhTPP1 formulation of the disclosure plotted against matched, untreated natural history patients matched by disease rating score using the 0 to 9 unit gait, language and vision subscales as an aggregate. Subject 1244-1001 (Figure 6A) had a rating decrease of one unit from 6 to 5 points, but then shortly regained a point to a score of 6, with no net changes afterwards. Subject 1244-1002 (Figure 6B) had a rating increase from 5 points to 6 points, followed by a decrease from 6 points to 4 points, then an increase from 4 points to 5 points, resulting in overall maintenance of the disease rating at the end of the study compared to Day 1. Subjects 1244-1003 (Figure 6C) and 1244-1010 (Figure 6I) maintained a rating of 9 points throughout the study, indicating normal gait, language function and vision; subjects 1244-1004 (Figure 6D) and 1244-1009 (Figure 6H) maintained a rating of 6 points throughout the study, and subject 1244-1008 (Figure 6G) maintained a rating of 5 points throughout the study. Subject 1244-1006 (Figure 6E) had an initial rating decrease of one unit from 6 points to 5 points, followed by a further decrease to 4 points, but regained a unit to a rating of 5 points, with no net changes thereafter. Subject 1287-1005 (Figure 6F) had a rating decrease from 6 points to 4 points, followed by an increase from 4 points to 5 points and a decrease to 4 points, but regained a unit again, to a final rating of 5 points.

Addition of the vision subscale resulted in no change in the nine treated patients over the treatment period. However, score-matched, untreated natural history patients had significant contribution of vision loss to the aggregate score. There were multiple untreated natural history matches with a greater than 3 point difference compared to the treated patients, showing the contribution of vision decline to the aggregate score over the study period. Addition of the vision subscale thus increased the difference between treated patients and matched untreated natural history patients. As there was no unreversed loss of a disease rating unit in the matched, untreated natural history patients, the observation of treatment effect from rhTPP1 relating to arresting disease progression and stabilizing function can be extended from motor/gait and language to include the clinical domain of vision.

*Effect of Treatment on Total Disease Assessment*: Patients were also assessed over the course of the study using a combined 12-point scale containing the complete Hamburg or WCMC scores. The scores on the 12-point scale were the sum of the patient's individual scores for (1) motor/gait, (2) language, (3) seizures/myoclonus and (4) visual/feeding. Figures 10A to 10L show results from subjects treated with a rhTPP1 formulation of the disclosure using the 0 to 12 unit combined Hamburg (left panel) and WCMC scale (right panel). Sixteen of 23 patients had no unreversed decline on at least one scale, and 8 had an increased score on at least one scale at the end of the treatment period compared to baseline, confirming an overall treatment benefit in patients receiving rhTPP 1.

*Effect of Treatment on Brain Volume:* MRI was used to evaluate a secondary endpoint in treated patients. The disease process is characterized by atrophy, cell loss and signal abnormalities. These parameters correlate individually or as a composite score to patient age and disease rating score. Thus, there is a general consensus that disease progression correlates to MRI indices of atrophy, and multiple MRI parameters have been shown to correlate with age and disease severity in CLN2 disease (Dyke et al., AJNR Am J Neuroradiol. 2013;34(4):884-9); (Paniagua et al., Clin Neuroradiol. 2013; 23(3): 189-96). The imaging database that supports these conclusions is based on cross-sectional correlation of significant numbers of patients, however, there is no within-patient longitudinal acquisition of MRI images. Therefore, there is not the same ability to match longitudinal study-derived MRI analysis to a similarly derived natural history database.

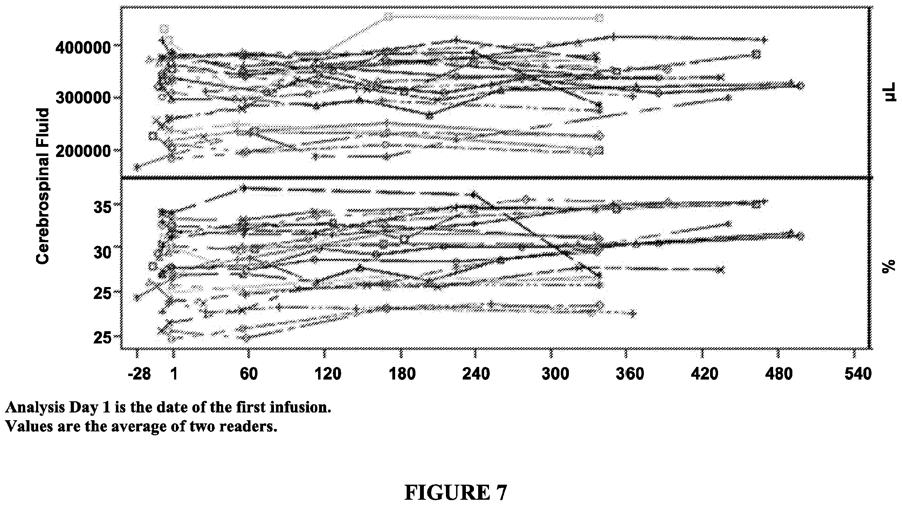

For analysis of treated patients, MRI acquisition parameters were standardized across the hardware platforms at the study sites. Data was acquired locally, redacted of identifying information, and transmitted to a central imaging core lab. The images were randomized such that the independent radiologist did not know the patient or temporal relationship to baseline. The changes in brain volume were reconstructed from the randomized independent central read. The data was analyzed to compare the studies longitudinally compared to baseline across the treated population. Figure 7 shows the summary of MRI-measured brain volumes in the treated patients. Cerebral atrophy results in greater volume and proportion of intracranial CSF. An increase in these measurements of atrophy is correlated to age and severity in CLN2 patients. Longitudinal plots of CSF volume and proportion for the treated patients indicated there also appeared to be little, if any change, in measurements of CSF parameters. All patients had MRI volumetry that appeared constant, and consistent with, the stabilization of ratings assessments.

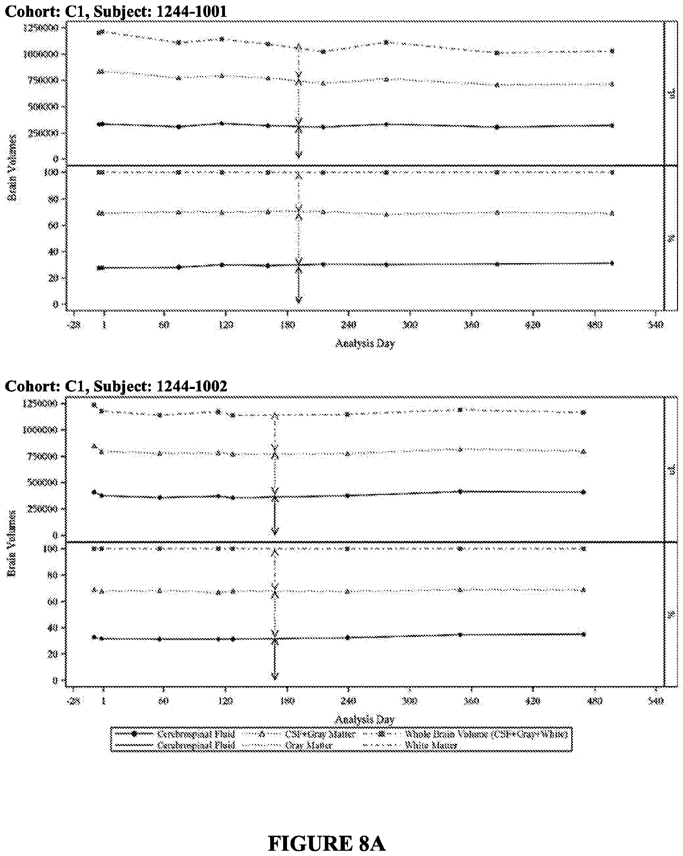

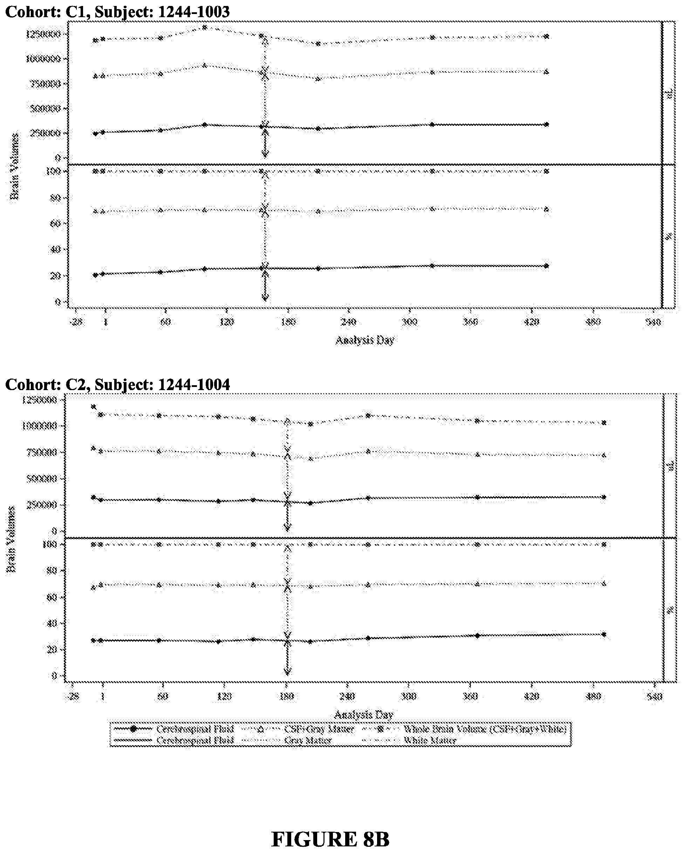

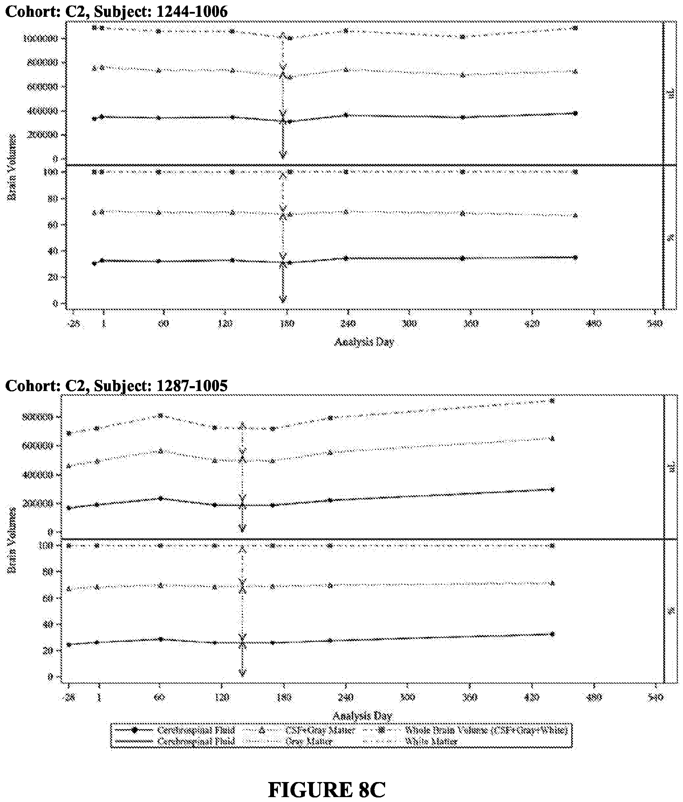

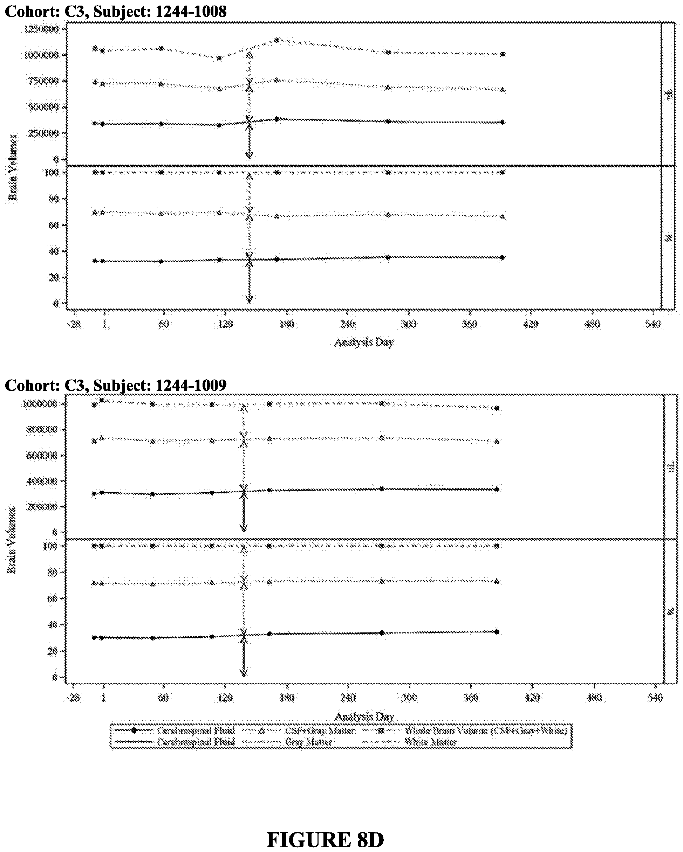

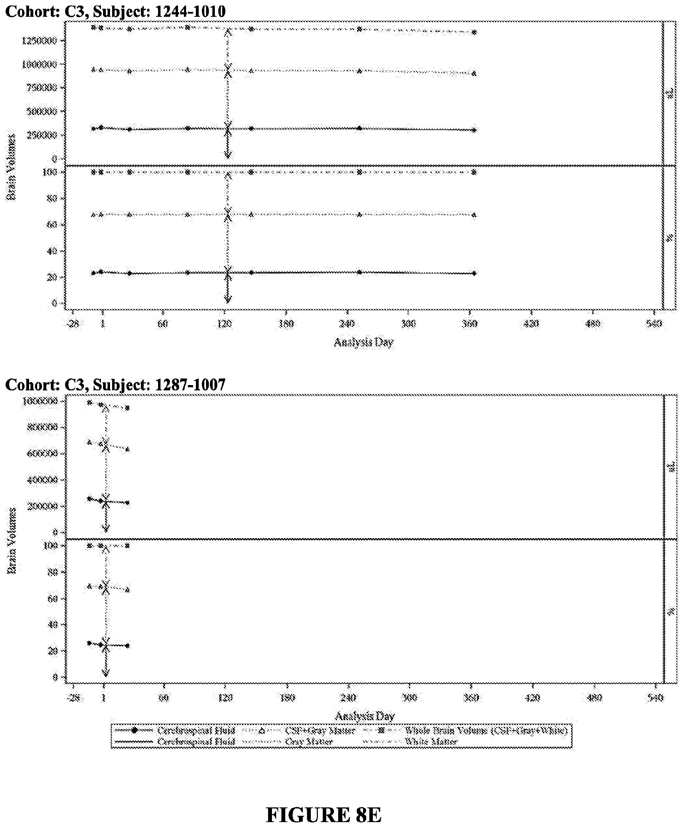

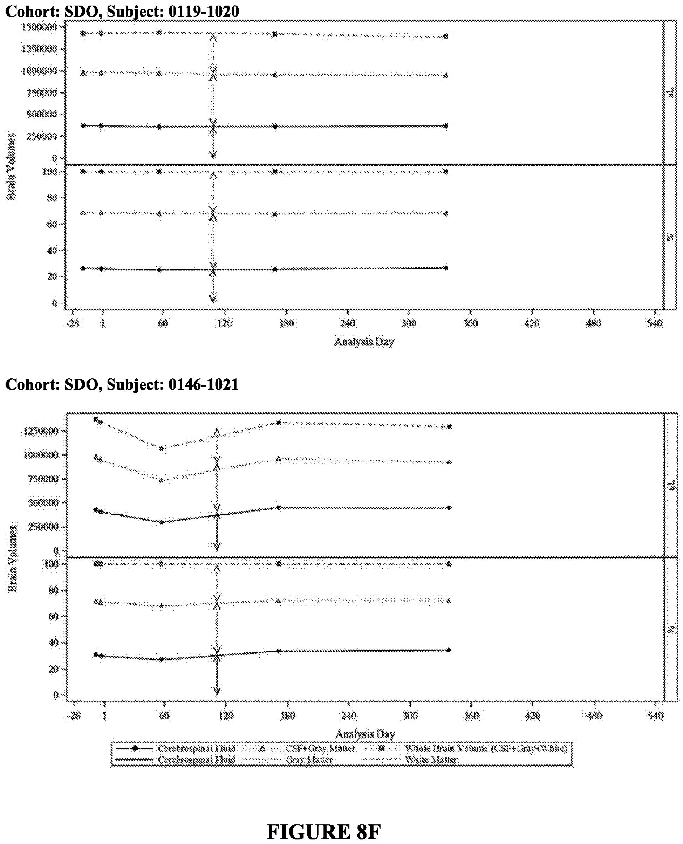

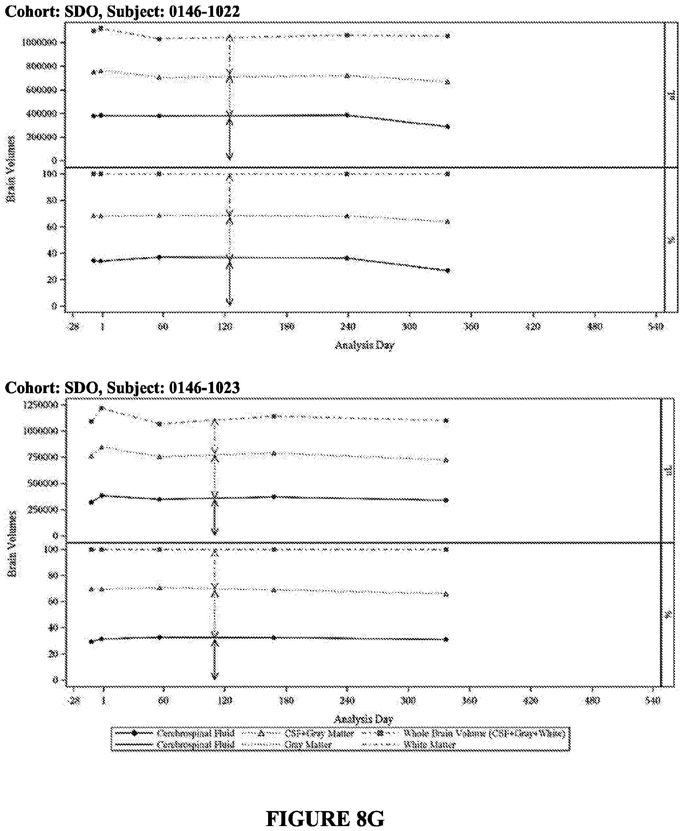

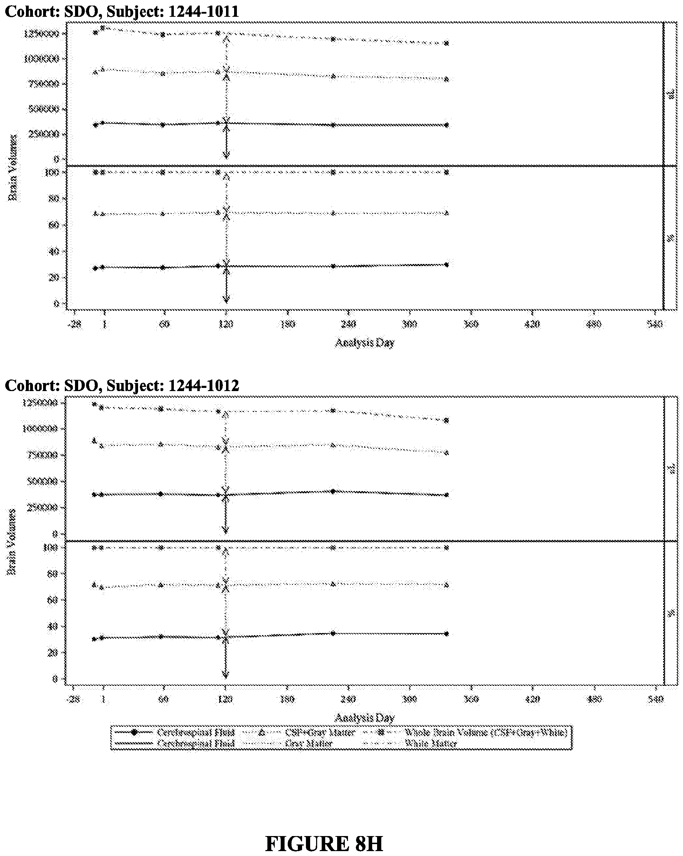

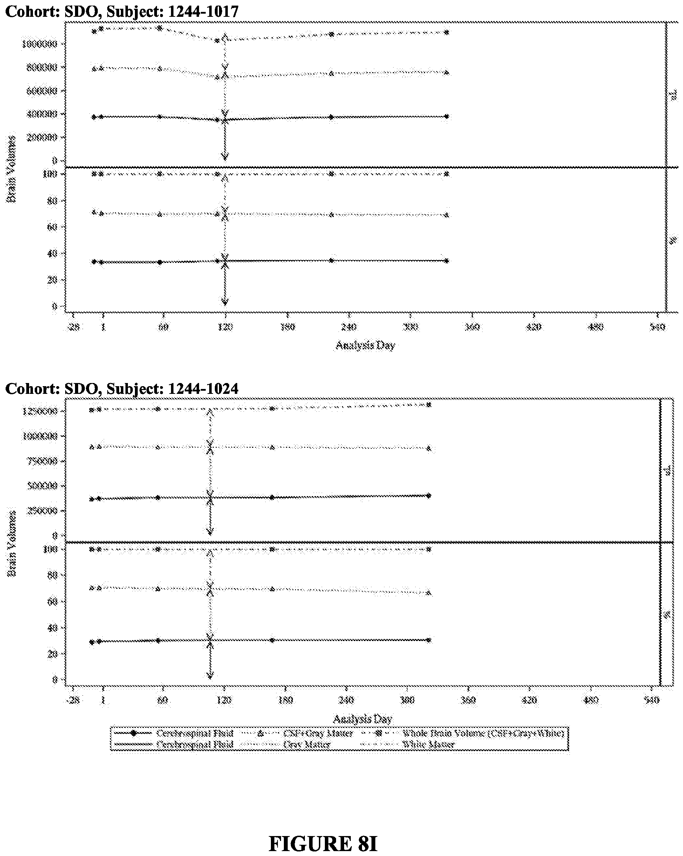

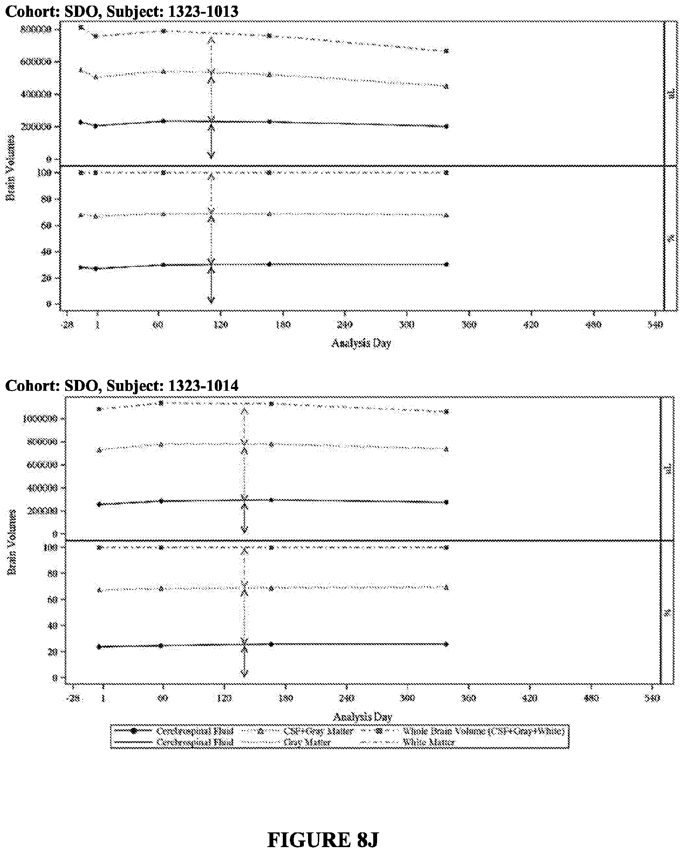

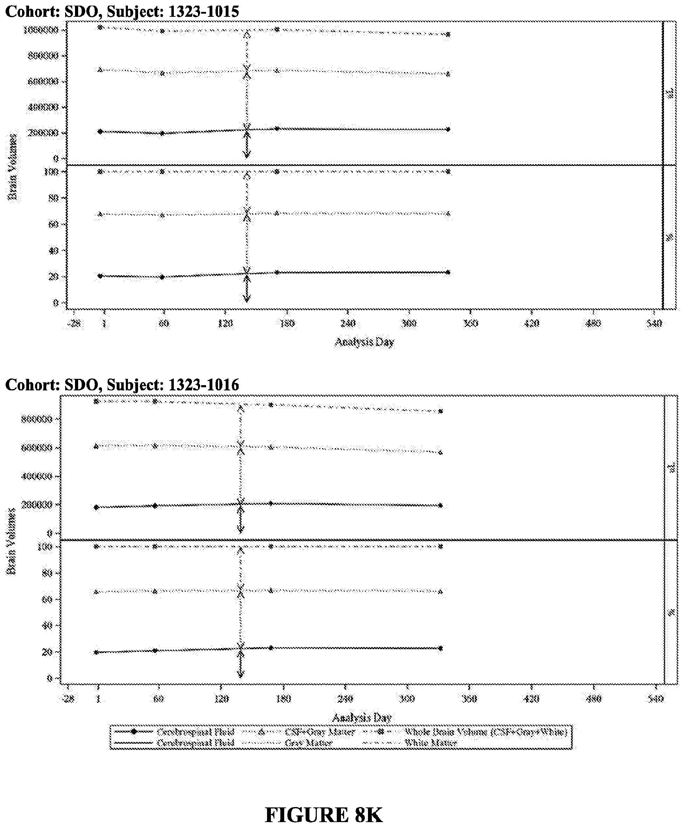

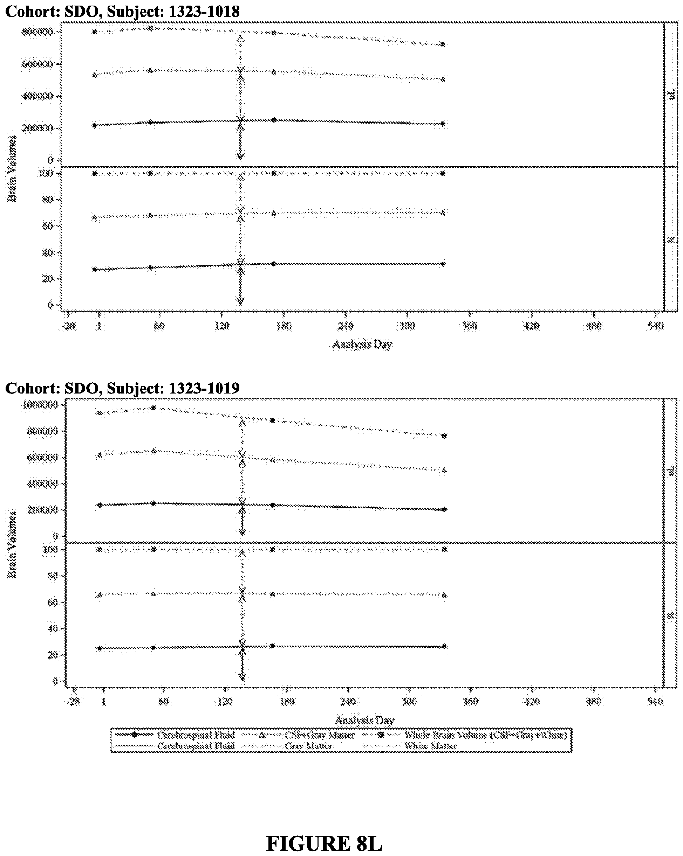

Figures 8A to 8L show the longitudinal MRI assessment of brain volumes in the treated patients. Active neurodegeneration in CLN2 patients is characterized by predominant loss of gray matter and compensatory gain of CSF. However, in the assessed period, there were very stable brain volumes and no evidence of a neurodegenerative process in treated patients. The volume of gray matter, shown in Figures 8A to 8L as the difference between the CSF and gray matter plot (dashed line) and CSF plot (solid line) in each of the top and bottom panels, was stable throughout the study for each of the treated patients. The change in the volume of cortical grey matter as a percentage of total brain volume from the last measurement prior to the first 300 mg infusion (baseline) compared to the last observation at ≥ 48 weeks of treatment is shown in Table 6 below.

**TABLE 6**

| | Overall (n = 23) |
|---|---|
| Change from Baseline to Last Observation | |
| N | 23 |
| Mean (SD) | -2.3 (2.01) |
| Median | -2.6 |
| Min , Max | -5.8 , 3.1 |

The longitudinal change of the cortical volume is -1% each year in normal children age 4-12, but -12.5% each year for untreated CLN2 patients. During treatment with rhTPP1, the volume of CSF, gray matter, and white matter stayed relatively constant, attenuating 89% of disease-related loss of cortical volume.

*Adverse Events*: One patient withdrew from the study due to inability to comply with the protocol. The remaining 23 patients remained on the study and tolerated treatment with the rhTPP 1 drug product via the ICV route. There were no deaths, treatment-related withdrawals, or study discontinuations due to a safety-related reason. Consistent with minimal impact of device implantation, all patients were dosed within a week of surgery. Out of a total of 325 infusions, only 5 (1.5%) were interrupted for any reason, with only 2 (0.6%) of these interrupted for adverse event-related reasons. The most frequent non-CLN2 disease associated adverse events observed in the study were pyrexia, hypersensitivity, and upper respiratory tract infection (each in 25% of subjects overall). In general, these events were mild, self-limited and managed medically. Investigator-defined hypersensitivity events were associated with few peripheral manifestations and were medically managed with a combination of antipyretics, antihistamine and/or steroids. Laboratory data demonstrated a lack of clinically relevant changes in peripheral labs. In the CSF, some patients had mild, transient pleocytosis with no change in CSF glucose or protein. In summary, the evaluation of safety parameters demonstrated that treatment with a rhTPP 1 formulation of the disclosure via ICV infusion was tolerated in all patients.

### Conclusion

The clinical study demonstrated that every patient with a treatment exposure of more than 36 weeks had significant clinical benefit, characterized by complete arrest in the progression of CLN2 disease, which constituted maximal treatment benefit, as gain of function was not expected in the time frame for patients with moderate progression and active degeneration.

This finding was even more compelling when the patients were matched to members of the natural history database based on multiple parameters including baseline disease rating, age and genotype. This matching revealed that during the same period of time that treated subjects experienced halted disease progression on treatment with the rhTPP 1 drug product, the matched untreated natural history patients experienced substantial loss of function. All treated patients showed, therefore, arrest of disease progression compared to active disease progression in matched untreated natural history patients. The median rate of decline in the untreated natural history population was estimated at 2.1 points each year based on the available natural history data, with each unit of decline representing a significant milestone loss of physiological function. For the majority of patients entering the study, the preservation of 2 units translated to continued independent ambulation and meaningful communication.

Overall, the results demonstrated that a rhTPP 1 formulation and method of treatment of the disclosure has an acceptable safety/tolerability profile. No subjects discontinued the study or treatment due to an adverse event. One subject withdrew from the study after one treatment dose due to inability to comply with the protocol. Analysis of PK and immunogenicity revealed high CNS delivery, and no formation of antibodies in the CSF.

The foregoing Examples demonstrate that the formulations and methods comprising rhTPP 1 described herein are effective in preventing or treating CLN2 disease and/or one or more clinical symptoms of CLN2. In a disease whose clinical course is characterized by rapid, inexorable and irreversible neurodegenerative disease progression, halting disease progression, especially in each treated patient, is a substantial and unexpected clinical benefit.

### EXAMPLE 4

This example describes a Phase 2 open-label study to evaluate the safety, tolerability, and efficacy of a formulation comprising rhTPP 1 for intracerebroventricular administration in pediatric patients less than 18 years of age with CLN2 disease.

### Study Objectives

The primary objectives of this study include the following: (1) to evaluate safety and tolerability of TPP1 administered via intracerebroventricular (ICV) device and (2) to evaluate treatment effectiveness as a delay in progression of CLN2 motor-language clinical scale.

The secondary objectives of this study include the following: (1) to assess immunogenicity in CSF and serum, (2) to measure MRI parameters of disease progression, and (3) to assess impact of treatment on the total Hamburg clinical rating scale.

The exploratory objectives of this study include the following: (1) to assess development achievement, (2) to assess abnormal involuntary movements, (3) to evaluate retinal anatomy using optical coherence tomography (OCT), (4) to determine seizure onset, type and frequency, (5) to assess quality of life metrics, and (6) to analyze putative molecular biomarkers from CSF and plasma.

### Investigational Plan: Overall Study Design and Plan

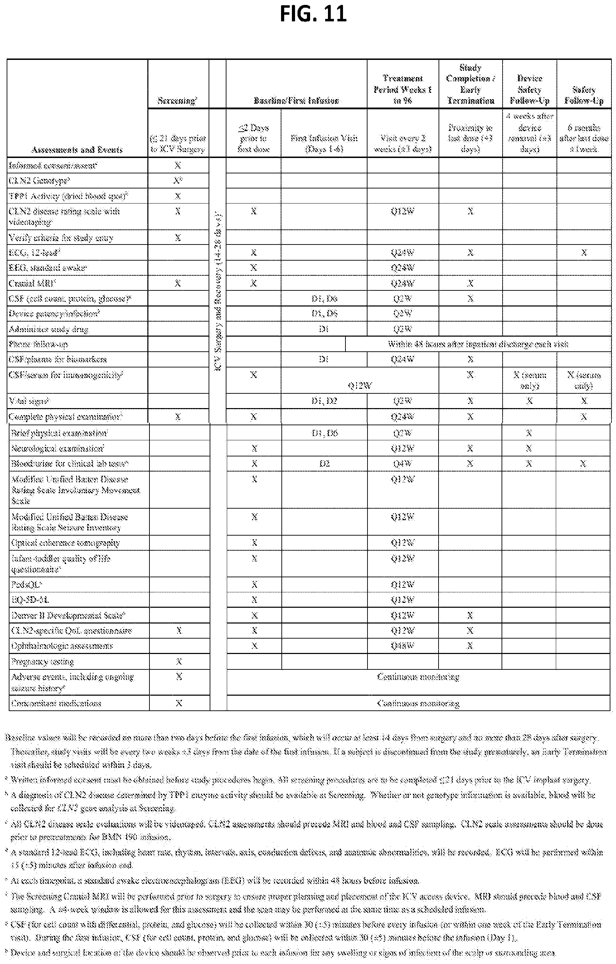

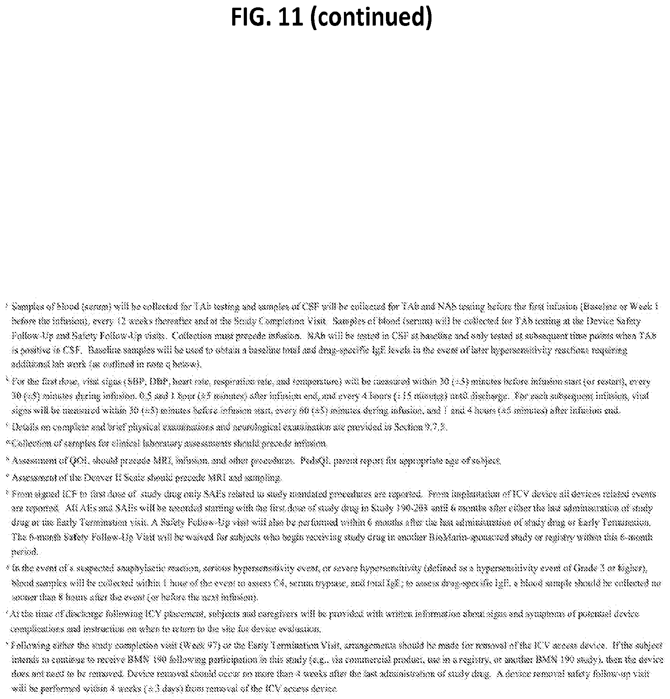

This will be a Phase 2 open-label study in CLN2 disease patients with confirmed mutation of the *CLN2* gene and impaired *TPP1* activity. The formulation comprising rhTPP 1 (referred to herein as "TPP1") will be administered every two weeks by ICV infusion. Because practical and ethical concerns preclude contemporaneous or untreated control subjects, findings may be compared with historical data from existing CLN2 disease registries and/or data from siblings, as appropriate. A summary of events is provided by visit in Figure 11.

Study eligibility will be determined before (≤ 21 days) subjects are admitted to the hospital for surgical implantation of an ICV access device. The Baseline visit to collect clinical scores and clinical laboratory parameters will be completed no more than two days before the first infusion.

The planned enrollment for this study is up to 5 subjects. Patients eligible to enroll in this study must have a sibling with a confirmed CLN2 diagnosis who was enrolled in a prior study described in Example 3. All subjects will be administered a dose of TPP1 (300 mg) every 14 days from the date of the first infusion (+3 days) for at least 96 weeks. Dosing can be adapted in the judgement of the investigator to adverse events by reduction to 150 mg and/or by reducing the infusion rate during the course of the trial.

Patients in this study will be required to have an ICV reservoir surgically implanted for administration of TPP 1. An MRI will be performed in advance of the procedure to ensure proper planning and placement of the ICV access device. Patients will be monitored closely in a nursing-intensive environment for 48 hours post-procedure. Following ICV access device placement, subjects and their caregivers will be given written instructions providing details on signs and symptoms of concern for device complications and instruction on when to return to the site for device evaluation. An additional follow-up phone call will be conducted within 48 hours of inpatient discharge.

The first infusion will occur at least 14 days from surgery to implant the ICV reservoir and no more than 28 days after surgery. Thereafter, study visits will be every two weeks ±3 days from the date of the first infusion (+3 days). Generally, functional and QOL assessment should precede MRI and sampling, which should precede infusion; sample collection may occur when subjects are sedated for MRI. If a subject is discontinued from the study prematurely, an Early Termination visit should be scheduled within 3 days.

For all infusions, subjects will be monitored in an inpatient setting for a minimum of 24 hours from the start of the infusion. For the first infusion only, subjects will also return for a follow-up visit to clinic 72 hours after the start of infusion. After all visits, the parent or legal guardian will be telephoned within 48 hours to determine health status.

Vital signs will be measured within 30 (±5) minutes before, every 60 (±5) minutes during, and 1 and 4 hours (±5 minutes) after every infusion. For the first dose, vital signs will be measured within 30 (±5) minutes before infusion start (or restart), every 30 (±5) minutes during infusion, 0.5 and 1 hour (±5 minutes) after infusion end, and every 4 hours (±15 minutes) until discharge.

Efficacy will be measured using CLN2 motor-language clinical rating scale. Total Hamburg 0 to 12 point CLN2 scale will be collected. Other secondary efficacy measures will include developmental status, seizure frequency, involuntary movements, and retinal anatomy and quality of life metrics. The safety and tolerability of treatment will be assessed by collection of adverse events (AEs), physical findings, vital signs, ECGs, EEGs and clinical laboratory tests. AEs will be assessed by the investigator for severity, seriousness, and relationship to study drug and/or the ICV access device.

Because hypersensitivity reactions may be associated with ERT administration, subjects should be pretreated with an age-appropriate dose of antihistamine (and antipyretic, if appropriate) medication ~30 minutes before infusion. Subjects may be pretreated, at the discretion of the Investigator, with age-appropriate sedative medication approximately 30 minutes before TPP1 infusion according to institution's standard practices.

A temporal relationship to study drug infusion will be utilized to define TREs. This should be distinguished from the clinical assessment of an infusion reaction. Thus, an adverse event occurring within 24 hours of the start or restart of TPP 1 infusion will be defined as a TRE.

Some of the events that occur within the 24 hour period post-infusion may be hypersensitivity mediated. Hypersensitivity reactions are characterized by an adverse local or general response from exposure to an allergen. Symptoms of hypersensitivity reactions may include fever, chills/rigors, skin symptoms (urticaria, angioedema, rash), respiratory symptoms (dyspnea, wheezing, stridor), gastrointestinal symptoms (nausea, vomiting, abdominal pain) and/or cardiovascular changes (hypotension/hypertension).

In severe cases, anaphylaxis - a systemic hypersensitivity reaction - may also occur. Anaphylaxis is the most severe form of hypersensitivity reaction in which symptoms may occur during or within hours of an infusion; if anaphylaxis goes untreated, death may result.

Symptoms of anaphylaxis may include involvement of skin and/or mucosal tissue (e.g., generalized hives, pruritus or flushing, swollen lips-tongue-uvula) respiratory compromise (e.g., dyspnea, wheeze-bronchospasm, stridor, reduced peak expiratory flow, hypoxemia), and reduced blood pressure or end-organ dysfunction (e.g., hypotonia, syncope, incontinence).

To date there has been no anaphylaxis or anaphylactoid reactions in studies with TPP1. However, in the event of a suspected anaphylactic reaction, serious hypersensitivity event, or severe hypersensitivity (defined as a hypersensitivity event of Grade 3 or higher), blood samples will be collected within 1 hour of the event to assess C4, serum tryptase, and total IgE; to assess drug-specific IgE, a blood sample will be collected no sooner than 8 hours after the event (or before the next infusion).

Another possible AE is infection from the indwelling ICV catheter. If an infection is suspected, blood and CSF samples will be drawn for assessments. The subsequent course of therapy may include antibiotic therapy and catheter reposition or withdrawal. If TPP1 treatment is suspended, TPP 1 may resume if no more than 4 consecutive missed doses elapse after the last given dose.

A schedule of planned assessments during the study is provided in Figure 11.

### Screening

Before any study-related procedure is performed, parents or legal guardians will provide informed consent for study-eligible patients. Patients will be assessed to determine whether they meet study entry criteria (Example 5) and are suitable candidates for implantation of an ICV access device. A diagnosis of CLN2 disease determined by TPP1 enzyme activity (dried blood spot) should be available at Screening. Whether or not this genotype information is available, blood will be collected for CLN2 gene analysis at the Screening visit. In addition, blood for TPP1 enzyme activity (dried blood spot) will be collected at the Screening visit and analyzed centrally. Screening procedures are to be completed ≤ 21 days prior to the ICV reservoir implant surgery.

CLN2 rating scales will be performed in full, as appropriate to the subject. The Ratings Assessment Guidelines provide detailed instructions on rating scale assessment, and the Imaging Charter (See Denver Development Scale II) provides instructions on MRI evaluation by a centralized facility. In addition, a complete physical examination will be conducted

### Surgery and First Dose

Study subjects will be admitted to the hospital for surgical implantation of an MRI-compatible ICV access device in the lateral ventricle of the right hemisphere; surgery and anesthesia will be under the direction of a neurosurgeon (refer to Study Pharmacy Manual for compatible ICV reservoirs and cannula). An MRI will be performed prior to the surgery to ensure proper planning and placement of the ICV access device. Generally, surgery and postoperative care will be determined by standards of care at the study centers and specific clinical needs of the subject. Subjects will be monitored in a nursing-intensive setting for a minimum of 48 hours post-procedure. Following ICV access device placement, subjects and their caregivers will be given written instructions providing details on signs and symptoms of concern for device complications and instruction on when to return to the site for device evaluation. An additional follow-up telephone call will be conducted within 48 hours after inpatient discharge.

The first infusion will occur at least 14 days from surgery to implant the ICV reservoir and no more than 28 days after surgery. At the first (and each subsequent) study drug administration, the investigator will evaluate the patency, location, and skin integrity of the reservoir. Access to the device is performed using strict sterile technique. Skin covering the reservoir is inspected for an appropriate needle insertion site. The needle insertion site must be intact, without evidence of breakdown, wound, infection or rash. The needle used is a small gauge non-boring tip. Once the reservoir has been accessed, the needle is immobilized to ensure minimal movement or risk of removal. Guidance for assessing the reservoir devices for leakage and replacement is provided in the Study Pharmacy Manual. At the discretion of the investigator and/or neurosurgeon, the reservoir may be replaced during the clinical study.

### Discussion of Study Design, including choice of Control Group

This study must be performed in patients with CLN2 disease because neither ICV access device implantation nor potential development of TPP 1 autoimmunity, and their unknown long-term health consequences, are acceptable risks for healthy volunteers. However, these risks in a patient population are balanced by the near certainty of severe disability and death within a few years.

The diagnosis of CLN2 disease is often based on assay of enzyme activity, which will be required for participation in this study. A diagnosis of CLN2 disease determined by TPP 1 enzyme activity (dried blood spot) should be available at Screening. Whether or not genotype information is available, blood will be collected for CLN2 gene analysis at the Screening visit.

Because CLN2 disease is fatal in childhood, the study population must be children. Because therapeutic benefit is unlikely in advanced CLN2 disease, given the extensive depletion of cortical neurons, lack of extensive CLN2 disease progression is required for study participation, resulting in a study population comparable to that of a TPP1 gene therapy study (Worgall, 2008, Hum.Gene Ther.). Safety evaluation would also be limited in advanced patients, who are essentially in a vegetative state. The study is limited to affected children age 1 year or older.

A clinical rating scale, the Hamburg Scale, was developed to document the natural history of CLN2 disease (Steinfeld, 2002, Am.J.Med.Genet.).

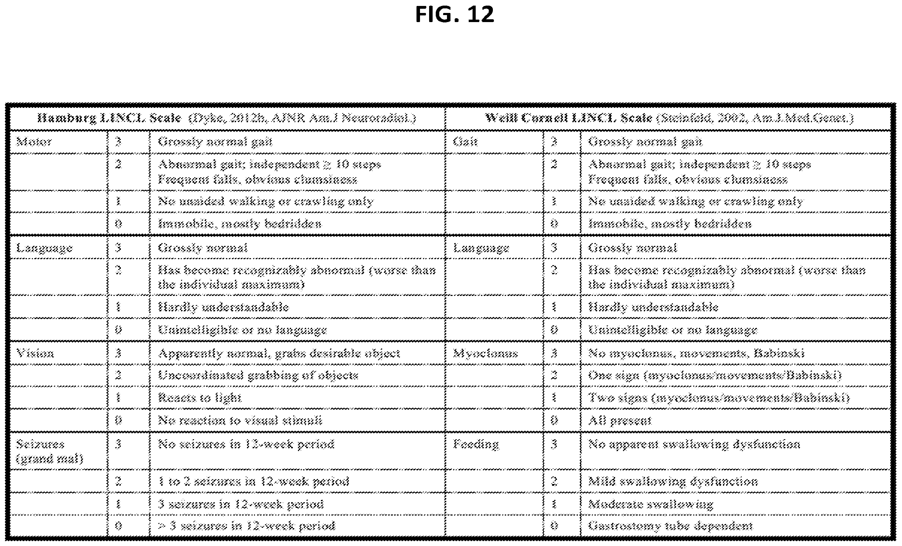

Although all four domains of the Hamburg rating scale will be completed (Figure 12), the motor and language domains are most useful for this study. Language and motor are the earliest domains to lose function, therefore the aggregate CLN2 motor-language scale are appropriate metrics for efficacy. The effectiveness endpoint will compare motor and language subscale scores of treated study subjects with those of untreated patients acquired from the natural history data of CLN2 disease registries and/or data from siblings as appropriate.

The remaining two domains in the rating scale (Figure 12) are less likely to be informative for this study. In order to capture the seizure and movement disorder manifestations of CLN2 disease in more detail, adapted domains from the Unified Batten Disease Rating Scale (UBDRS) are incorporated into the protocol. The UBDRS domains record involuntary movements and seizures according to type, frequency and severity (Kwon, 2011, Neurology).

Because of practical (limited number of available patients) and ethical (neurosurgery in children with fatal neurologic disease) concerns, this study design cannot involve contemporaneous, matched, randomized, blinded, or untreated control subjects (Arkin, 2005, Hum.Gene Ther.); (Crystal, 2004, Hum.Gene Ther.). Motor and language subscales and total scores for the Hamburg CLN2 disease scale will be compared with historical natural history data from the registry at the University Medical Center in Hamburg, Germany and/or data from siblings, as appropriate.

### EXAMPLE 5

This example describes the selection of the study population of the Phase 2 open-label study described in Example 4.

### Investigational Plan: Selection of Study Population

Patients with confirmed CLN2 disease progression may be eligible to participate in this study. Additional criteria for participation in this study are detailed below. Subjects must satisfy the following criteria in order to enroll in the study (Table 7):

**TABLE 7**

| Inclusion Criteria | Exclusion Criteria | | |
|---|---|---|---|
| • Diagnosis of CLN2 disease determined by TPP 1 enzyme activity available at Screening | Individuals who meet any of the following exclusion criteria will not be eligible to participate in the study: | | |
| • At least 1 sibling with confirmed CLN2 disease who was enrolled in the study of Example 3 | | | • Another inherited neurologic disease, e.g., other forms of CLN or seizures unrelated to CLN2 disease (patients with febrile seizures may be eligible) |
| • Age ≥1 year at the time of informed consent | | | |
| • Written informed consent from parent or legal guardian and assent from subject, if appropriate | | | • Percutaneous feeding tube placement |
| | | | • Has received stem cell, gene therapy, or ERT for CLN2 disease |
| • Ability to comply with protocol required assessments (laboratory sample collection, EEG, ECG, MRI, etc.) | | • Contraindications for neurosurgery (e.g., congenital heart disease, severe respiratory impairment, or clotting abnormalities) | |
| | | | • Contraindications for MRI scans (e.g., cardiac pacemaker, metal fragment or chip in the eye, aneurysm clip in the brain) |
| | | | • Episode of generalized motor status epilepticus within 4 weeks before the First Dose visit |
| | | | • Severe infection (e.g., pneumonia, pyelonephritis, or meningitis) within 4 weeks before the First Dose visit (enrollment may be postponed) |
| | | | • Presence of ventricular abnormality (hydrocephalus, malformation) |
| | | | • Presence of ventricular shunt |
| | | | • Has known hypersensitivity to any of the components of TPP 1 |
| | | | • Has received any investigational medication within 30 days before the first infusion of study drug or is scheduled to receive any investigational drug other than TPP 1 during the course of the study |
| | | | • Has a medical condition or extenuating circumstance that, in the opinion of the investigator, might compromise the subject's ability to comply with the protocol required testing or procedures or compromise the subject's wellbeing, safety, or clinical interpretability |
| | | | • Pregnancy any time during the study; a female subject judged by the investigator to be of childbearing potential will be tested for pregnancy |

### EXAMPLE 6

This example describes the duration of subject participation for the study described in Examples 4 and 5.

Subject participation will involve surgical implantation of the ICV access device followed by 14 to 28 days of post-operative recovery and will continue for at least 96 weeks of treatment for all subjects. Treatment will continue until all procedures are completed, the subject withdraws consent and discontinues from the study, is discontinued from the study by the investigator, or the study is terminated. A Safety Follow-Up visit will be conducted 6 months after the final TPP1 infusion, but will not be required if the subject enrolls in an extension study, registry, or otherwise has continued access to TPP1 within 6 months after the last infusion (refer to Section 10.2.1 for AE/SAE reporting instructions).

### EXAMPLE 7

This example describes the treatments administered in the study described in Examples 4 to 6.

All study subjects will be administered TPP1 by ICV infusion every two weeks preferably in the morning after a minimum fast of 2 hours. When a feeding tube is used, the tube should be turned off 2 hours before infusion. All subjects will be administered TPP 1 infusions of 300 mg, but dose reductions to 150 mg will be allowed as needed for safety reasons.

### Directions for Administration

Surgical implantation of an ICV reservoir will take place prior to study drug administration. The investigator will evaluate the patency, location, and skin integrity of the reservoir at each study drug administration (refer to the Study Pharmacy Manual). At each administration, the investigator will draw 1-2 ml of CSF into the device cannula to check for patency prior to administering the study drug. Access to the device is performed using strict sterile technique. Skin covering the reservoir is inspected for an appropriate needle insertion site. The needle insertion site must be intact, without evidence of breakdown, wound, infection or rash. The needle used is a small gauge non-boring tip. Once the reservoir has been accessed, the needle is immobilized to ensure minimal movement or risk of removal. At the discretion of the investigator and/or neurosurgeon, the reservoir may be replaced during the clinical study.

All study subjects will be administered TPP1 by ICV infusion every two weeks preferably in the morning after a minimum fast of 2 hours. When a feeding tube is used, the tube should be turned off 2 hours before infusion Study procedures for each study visit should precede study drug infusion unless otherwise specified. The date, time, volume, and concentration of each dose of study drug administered to each subject will be recorded in the dispensing log provided for the study as well as on the appropriate CRF. The Study Pharmacy Manual provides further instructions on preparation and administration of study drug.

Subjects will be admitted to the hospital for every TPP1 infusion. For all infusions, subjects will be monitored in an inpatient setting for a minimum of 24 hours from the start of the infusion. For the first infusion only, subjects will also return for a follow-up visit to clinic 72 hours after the start of infusion. After all visits, the parent or legal guardian will be telephoned within 48 hours to determine health status.

Because hypersensitivity reactions may be associated with ERT administration, subjects should be pretreated with age-appropriate doses of antihistamine (and antipyretic, if appropriate) medication approximately 30 minutes before TPP1 infusions. Subjects may be pretreated, at the discretion of the Investigator, with age-appropriate sedative medication approximately 30 minutes before TPP1 infusion according to institution's standard practices. Clinical, developmental, and QOL assessments are to be performed before premedication for infusions.

For administration at 300 mg, TPP 1 will be infused ICV at 2.5 mL/hour to deliver the entire volume over approximately 4 ( ± 1) hours. Uniform infusion rate should be ensured by use of a syringe pump with appropriate delivery range, delivery rate accuracy, and alarms for incorrect delivery or occlusion. If the dose needs to be stopped for safety or other reasons, it may be restarted at the same rate and completed so long as the total dose is administered within 10 hours of preparation of the dose syringes.

The normal infusion will be 300 mg administered over 4 ( ± 1) hours. If the subject experiences an AE, the investigator may, upon consultation with the medical monitor, reduce the dose or infusion rate for future infusions. Changes to the infusion should be performed in the following order:
(I) Reduce the infusion rate and administer the drug over a total of 6 hours
(II) Reduce the dose to 150mg administered over the standard time of 4 ( ± 1) hours
(III) Reduce the infusion rate and administer 150mg over a total of 6 hours

### Safety Monitoring

Following ICV access device placement, subjects and their caregivers will be given written instructions providing details on signs and symptoms of concern for device complications and instruction on when to return to the site for device evaluation. Subjects will be admitted to the hospital for every TPP 1 infusion. For all infusions, subjects will be monitored in an inpatient setting for a minimum of 24 hours from the start of the infusion. For the first infusion only, subjects will also return for a follow-up visit to clinic 72 hours after the start of infusion. After all visits, the parent or legal guardian will be telephoned within 48 hours to determine health status.

For the first infusion, vital signs (See Vital Signs) will be measured within 30 (±5) minutes before infusion start or restart, every 30 (±5) minutes during infusion, 0.5 and 1 hour (±5 minutes) after infusion end, and every 4 hours (±15 minutes) until discharge.

For each subsequent infusion, vital signs will be measured within 30 (±5) minutes before infusion start, every 60 (±5) minutes during infusion, and 1 and 4 hour (±5 minutes) after infusion end.

Subjects require regular monitoring for adverse events and epileptic seizures by appropriately trained personnel throughout the duration of the infusion. If epileptic seizures develop, the infusion may be interrupted at the discretion of the investigator. Because hypersensitivity reactions (anaphylaxis or general allergic) may occur, it is required that appropriately trained personnel and equipment for emergency resuscitation (including epinephrine) be available near the bedside during study drug infusion. In case emergency treatment is needed, all subjects should have an intravenous line during infusion.

Symptoms of hypersensitivity reactions may include fever, chills/rigors, skin symptoms (urticaria, angioedema, rash), respiratory symptoms (dyspnea, wheezing, stridor), gastrointestinal symptoms (nausea, vomiting, abdominal pain), and/or cardiovascular changes (hypotension/hypertension). If more severe symptoms, such as angioedema (tongue or throat swelling) or stridor, develop, the infusion should be stopped.

To date there has been no anaphylaxis or anaphylactoid reactions in studies with TPP1. However, in the event of suspected anaphylaxis local guidelines should be followed. In the event of a suspected anaphylactic reaction, serious TRE, or severe TRE (defined as a TRE of Grade 3 or higher), blood samples will be collected within 1 hour of the event to assess C4, serum tryptase, and total IgE; to assess drug-specific IgE, a blood sample should be collected no sooner than 8 hours after the event (or before the next infusion).

Safety assessments will be conducted during and after each infusion. Subjects may be required to stay for a longer observation period at the investigator's discretion. If an AE consistent with a TRE (Section 7.4.1) is observed, appropriate intervention may include infusion interruption, infusion rate decrease, or administration of antihistamine, oxygen, fluids, or steroids. If infusion is restarted after interruption, the initial rate should be approximately one-half the rate at which the reaction occurred. Further detail for infusion modification is provided in the Study Pharmacy Manual.

The parent or legal guardian will be instructed to contact the investigator to discuss any AE subsequent to discharge.

The use of ICV access devices can result in infections, intracerebral hemorrhage from chronic reservoir use, reservoir leakage, and seizures (Karavelis, 1996, Neurosurgery) (Kronenberg, 1998, Pain). Additional surgery may be required to fix or replace the devices. Patients will be monitored throughout the study for potential infections (high temperature, cough, rash, headache, swelling or drainage in the incision area) and signs of ICV reservoir leakage or failure (refer to the Study Pharmacy Manual).

Because of the inherent safety considerations surrounding the implanted device,
arrangements should be made for the subject to have the ICV access device removed no more than 4 weeks after either the Study Completion visit or the Early Termination visit. If the subject intends to continue to receive TPP1 following participation in this study (e.g., via commercial product, use in a registry, or another TPP1 study), then the device does not need to be removed. Following device removal, subjects should return to the site 4 weeks (±3 days) later for a safety follow-up visit.

### Method of Assigning Subjects to Treatment Groups

Subjects will be enrolled as they become available and without attention to any entry criterion or other patient characteristic.

### Selection of Dose

The planned dose is 300 mg.

The proposed dose level was derived from dose levels used in the TPP 1-null dachshund study (Vuillemenot, 2011, Mol.Genet.Metab). Pharmacological effects, including functional improvement and life extension, have been robustly demonstrated in TPP 1-null dachshunds at 4 mg and 16 mg dose levels. Since TPP1 activity in brain tissue is proximally related to CNS lysosomal storage materials, scaling by brain mass is judged to be predictive of the human therapeutic dose. Human-equivalent doses were calculated by brain mass scaling. The human brain on average achieves about 75% of adult mass by age 2 and 100% by age 5 (Giedd, 1996, Cereb. Cortex). If adult human brain mass is 1400 g, the range for healthy children aged 2 to 7 years would be 1050 to 1400 g. Given progressive brain atrophy in CLN2 disease patients, an average mass of 1000 g was assumed, producing a scaling factor of 20-fold based on average dachshund brain mass of 50 g.

The no-observed-adverse-effect level (NOAEL) which also yielded clinical disease benefit from nonclinical studies in Dachshund was 48 mg, which would correspond to 960 mg in human.

A preliminary analysis performed of the study in Example 3 demonstrated a positive benefit-risk profile in all children enrolled into the study at 300 mg dosed every 2 weeks through an intracerebroventricular (ICV) device. Further, in those children who had received more than 36 weeks of TPP1 dosing, clinical scores stabilized, in contrast to matched historical untreated controls in which decline was a rapid and profound in the majority of matches.

### Selection of Timing of Dose for Each Subject

A mean CNS half-life of approximately 2 weeks suggests biweekly dosing may sustain therapeutic TPP1 levels in the CNS. TPP1 concentrations in CSF remained above the lysosomal Kuptake for approximately 48 hours after single ICV or IT-L infusions in species with CSF dynamics similar to those in human (Vuillemenot, 2014, Toxicol. Appl. Pharmacol.), (Vuillemenot, 2011, Mol.Genet.Metab); 0190-09-071). In these same species (dog and monkey), CNS distribution of TPP1 was extensive in many brain regions.

### Selection of Infusion Volume and Rate

The nonclinical studies in the dachshund and cynomolgus monkey utilized an infusion rate of approximately 5% of the total CSF volume per hour. This was expected to represent a safe infusion rate that would minimize changes in total CSF volume and intracranial pressure. The dachshunds received ICV infusions at a rate of 0.6 mL/hour for 2-4 hours, while monkeys received 0.88 mL/hour for 3.6 hours. No effects were observed in these studies indicative of safety concerns due to the infusion rate. In the CLN2 patient population, the estimated CSF volume is approximately 100 mL. For the proposed clinical trial, a volume of 10 mL infused over a 4 hour period represents an infusion rate of 2.5% of the total CSF volume per hour, which is approximately half the rate that had no safety effects in the nonclinical studies. Therefore, we expect that 10 mL infused over 4 hours would be safe in CLN2 patients.

### Blinding

This is a single-arm, open-label study. Study site assessments of safety and clinical severity will be performed without blinding to treatment and subjects will be enrolled as they become eligible.

As defined in the Imaging Charter, oversight of MRI evaluation will be performed by independent radiologists at a central imaging facility. The interpreting radiologists and software analysis will be blinded to subject and time on study. All subject-identifying information will be redacted before endpoints are assessed.

### Prior and Concomitant Medications

Medications (prescription, over-the-counter, and herbal) and nutritional supplements taken during the 30 days before informed consent will be recorded in the CRF at Screening. At each subsequent visit (or within one week of the Early Termination visit), change in any medication (dosage, frequency, new medication, or cessation) since the previous visit will be recorded in the CRF.

Any concomitant medication added or discontinued during the study should be recorded in the CRF (or within one week of the Early Termination visit).

Subjects may be taking anticonvulsants and medications for myoclonus, tremor, agitation, and pain. Investigators will be asked to keep these regimens constant throughout the study, unless changes are required due to lack of efficacy or toxicity.

### Treatment Compliance

Study drug will be administered to subjects at the study site by a qualified professional. Date, time, volume, and concentration of each dose must be recorded in the dispensing log as well as on the appropriate CRF. In the event that a dose of study treatment is missed or incomplete, the investigator should record the reason and any other pertinent information on the CRF as appropriate.

### Dietary or Other Protocol Restrictions

There are no dietary or other protocol restrictions for this study. Subjects who require a PEG (percutaneous endoscopic gastrostomy) tube as the standard of care during the course of the study will be allowed to continue in the study. When a feeding tube is used, the tube should be turned off 2 hours before infusion.

### EXAMPLE 8

This example describes the efficacy and safety variables of the study of Examples 4-7.

### Efficacy Variables

Although this study is designed to assess safety and tolerability primarily, efficacy will be measured using CLN2 motor-language clinical rating scale. Total Hamburg 0 to 12 point CLN2 scale and MRI measures of disease progression will be collected as secondary endpoints. Other exploratory efficacy measures will include developmental status, seizure frequency, involuntary movements, retinal anatomy and quality of life metrics.

### CLN2 Clinical Rating Scale

Disease severity will be evaluated by the CLN2 clinical rating scale (Steinfeld, 2002, Am.J. Med. Genet.); (Worgall, 2008, Hum.Gene Ther.). This scale consists of four domains with intrinsic content validity. Within each domain, a score from 0 to 3 is assigned and overall scores are calculated by summing the four domain scores for a final rating of 0 (severely impaired) to 12 (normal).

Since the motor and language domains are most relevant to CLN2 disease progression (Section 9.2), they will be used to assess efficacy (Appendix 1).

Raters will be identified as qualified practitioners, who have been trained on the definitions and implementation of the CLN2 disease rating scale. All raters at all sites will be required to pass a training session designed to standardize the definitions and scale anchor points across the study, before study ratings take place. Whenever possible, a single rater should evaluate each enrolled patient for the duration of treatment. Further, patient ratings should take place at the same time in the study visit, preferably in the morning before procedures and/or infusion takes place. CLN2 scale assessments should be done prior to pretreatments for TPP 1 infusion.

### Denver Development Scale II

The Denver II is a revision and update of the Denver Developmental Screening Test.

Both tests were designed to monitor the development of infants and preschool-aged children. The tests cover four general functions: personal social (such as smiling), fine motor adaptive (such as grasping and drawing), language (such as combining words), and gross motor (such as walking). Ages covered by the tests range from birth to 6 years.

### Modified Unified Batten Disease Rating Scale Involuntary Movement Inventory

The modified Unified Batten Disease Rating Scale (mUBDRS) Involuntary Movement inventory is a rating scale that measures the type, frequency and severity of common involuntary movements associated with CLN2 disease such as myoclonus and dystonia.

### Magnetic Resonance Imaging

All image data will be acquired on a 1.5 Tesla MRI platform. Study MRIs will include localizer, 3D T1-weighted sagittal, T2-weighted gradient-echo, diffusion-weighted axial and FLAIR axial acquisitions, as specified in the Imaging Charter. Total scanner time is less than 60 minutes and is expected to be accomplished in the majority of subjects with sedation.

Volumetric analysis of images will be done by estimating both volume of total cortical grey matter and proportion of the cranial CSF.

All patients will have an MRI without contrast prior to implantation of the ICV access device, to ensure proper planning and placement. No study assessments associated with an MRI will be performed at the time of this MRI, and any readings taken during this MRI will not be included in the analysis of study data.

An MRI scan also should be performed whenever infection or shunt dysfunction is suspected by the investigator. In this case, according to the Imaging Charter, the MRI should be with or without intravenous contrast with at least T1-weighted axial and sagittal aspects. For suspected meningitis, an MRI of the brain should be performed with and without contrast, according to the Imaging Manual.

### Optical Coherence Tomography

Optical coherence tomography (OCT) is a non-invasive imaging test that uses light waves to take cross-sectional pictures of the retina's layers in order to measure their thickness. These measurements can aid in early detection and treatment for retinal diseases. OCT will be performed locally and should precede infusions.

### Modified Unified Batten Disease Rating Scale Seizure Inventory

The mUBDRS Seizure Inventory measures the type and frequency of seizures in CLN2 patients in the prior 3 month interval. The inventory is completed with the aid of caregiver/family member recall in the period between study visits.

### PedsQL

The PedsQLTM Generic Core Scales are designed to measure Quality of Life in children and adolescents. The assessments are brief, practical and developmentally appropriate. The instrument is responsive to clinical change over time (Msall, 2005, Ment.Retard.Dev Disabil.Res Rev). The four parent reports cover the ages from 1-12 months, 13-24 months, 2-4 years, and 5-7 years, and include questions regarding physical, emotional, and social functioning, with school functioning where applicable. Patients who become older than age 7 during the study will no longer be assessed using this tool.

### EQ-5D-5L

The EQ-5D-5L instrument is a self-reported questionnaire designed to measure general health status (The EuroQol Group, 1990, Health Policy), (Brooks, 1996, Health Policy). The EQ-5D-5L is composed of 2 parts: a descriptive system that assesses 5 levels of perceived problems (mobility, self-care, usual activities, pain/discomfort, and anxiety/depression) in 5 dimensions and the EQ visual analogue scale (EQ VAS) assessment for overall health.

### CLN2-specific QoL Questionnaire

The CLN2-specific QoL Questionnaire is a disease specific supplement to the PedsQL using the same format and quantitation. The questionnaire is a novel instrument that was designed in collaboration with patient family and advocacy groups to capture elemental care and quality of life issues in late infantile CLN2 disease.

### Infant Toddler Quality of Life Questionnaire

The Infant Toddler Quality of Life Questionnaire (ITQOL) is to assess levels of health and wellbeing in children aged between 2 months to 5 years. This tool asks parents of pre-school children to reflect on physical and psychosocial domains such as development, pain, moods, and impact of child health on parents.

### Immunogenicity

Immunogenicity tests will be performed at a central laboratory using validated immunogenicity assays on serum and CSF samples. Samples of blood (serum) will be collected for TAb testing and samples of CSF will be collected for TAb and NAb testing before the first infusion (Baseline or Week 1 before the infusion), every 12 weeks thereafter and at Safety Follow-Up (or within one week of the Early Termination visit). Collection must precede infusion. NAb will be tested in CSF at baseline and only tested at subsequent time points when TAb is positive in CSF.

Baseline samples will be used to obtain a baseline total and drug-specific IgE levels in the event of later hypersensitivity reactions requiring additional lab work.

To date there has been no anaphylaxis or anaphylactoid reactions in studies with TPP1. However, in the event of a suspected anaphylactic reaction, serious hypersensitivity event, or severe hypersensitivity (defined as a hypersensitivity event of Grade 3 or higher), blood samples will be collected within 1 hour of the event to assess C4, serum tryptase, and total IgE; to assess drug-specific IgE, a blood sample will be collected no sooner than 8 hours after the event (or before the next infusion).

### Clinical Laboratory Assessment

Blood and urine samples will be collected for routine clinical laboratory assessments (blood chemistries, hematology, urinalysis) and analyzed centrally. Collection should precede infusion.

Any abnormal test result determined clinically significant by the investigator should be repeated until its cause is determined, the value returns to baseline or within normal limits, or the investigator determines the abnormal value was no longer clinically significant.

All abnormal clinical laboratory pages should be initialed and dated by an investigator, along with a comment regarding clinical significance. Each clinically significant laboratory result is to be recorded as medical history at Screening and as an AE subsequently.

If known, the diagnosis associated with an abnormality in clinical laboratory results considered clinically significant by the investigator should be recorded on the AE CRF.

A table of clinical laboratory tests to be carried out as described herein follows (Table 8):

**TABLE 8**

| **Blood Chemistry** | **Hematology** | **Urinalysis** |
|---|---|---|
| albumin | hemoglobin | appearance |
| alkaline phosphatase | hematocrit | color |
| ALT (SGPT) | WBC count | pH |
| AST (SGOT) | RBC count | specific gravity |
| direct bilirubin | platelet count | ketones |
| total bilirubin | differential cell count | protein |
| blood urea nitrogen | | glucose |
| calcium | | bilirubin |
| carbon dioxide | | nitrite |
| chloride | | urobilinogen |
| total cholesterol | | hemoglobin |
| C-reactive protein | | |
| creatinine | | |
| creatine kinase | | |
| glucose | | |
| GGT | | |
| LDH | | |
| phosphorus | | |
| potassium | | |
| total protein | | |
| sodium | | |
| uric acid | | |

### Cerebrospinal Fluid Surveillance

Samples of standard clinical laboratory CSF for routine surveillance (cell count with differential, protein, and glucose) will be collected within 30 (±5) minutes before every infusion. A small volume of CSF will be collected from the ICV reservoir and analyzed locally. Collection should precede study drug infusion.

### Biomarkers

Samples of plasma and CSF will be collected to assay putative molecular/biochemical biomarkers. Collection should precede study drug infusion. Samples will be tested centrally.

### Other Laboratory Assessments

Subjects who experience an SAE possibly related to TPP1 or other AE of concern may have additional blood samples drawn to assess immunogenicity or safety parameters.

### Vital Signs, Physical Examinations, and Other Observations

### Vital Signs

For the first infusion, vital signs (SBP, DBP, heart rate, respiration rate, and temperature) will be measured within 30 (±5) minutes before infusion start or restart, every 30 (±5) minutes during infusion, 0.5 and 1 hour (±5 minutes) after infusion end, and every 4 hours (±15 minutes) until discharge.

For each subsequent infusion, vital signs will be measured within 30 (±5) minutes before infusion start, every 60 (±5) minutes during infusion, and 1 and 4 hour (±5 minutes) after infusion end.

### Physical Examination

A complete physical examination will include general appearance (head, eyes, ears, nose, and throat), cardiovascular, dermatologic, lymphatic, respiratory, gastrointestinal, genitourinary, musculoskeletal, and body weight and height.

A brief physical examination will include general appearance, cardiovascular, respiratory, neurologic, and gastrointestinal assessments.

Use of an ICV reservoir device for intracerebroventricular drug administration requires that patients be monitored throughout the study for potential infections (high temperature, cough, rash, headache, mental status changes, swelling or drainage in the incision area) and signs of ICV reservoir leakage or failure (swelling of skin around reservoir site, difficulty with CSF extraction, erythema of the scalp, bulging of reservoir device, or extravasation of fluid on infusion).

The investigator will evaluate the patency, location, and skin integrity of the reservoir at each study drug administration. The investigator will check for scalp edema, erythema or skin breakdown at the site of the reservoir prior to infusion. Patency will be assessed during pre-infusion sampling and again at the time of infusion. Difficulty in obtaining the required volume of CSF necessary for pre-infusion samples or signs of ICV reservoir leakage (swelling of skin around reservoir site, erythema of the scalp, bulging of reservoir device, or extravasation of fluid) will prompt further evaluation of the reservoir for failure prior to continuing with infusion. Additional surgical consultation including surgery may be required to fix or replace the device.

Clinically significant abnormalities will be recorded under Medical History at Screening or as AEs thereafter.

### Neurological Examination

A complete neurological examination will include level of consciousness, speech, language, cranial nerves, motor strength, motor tone, abnormal movements, reflexes, upper extremity sensation, lower extremity sensations, gait, Romberg, nystagmus, and coordination.

### Electrocardiogram

A standard 12-lead ECG, including heart rate, rhythm, intervals, axis, conduction defects, and anatomic abnormalities. ECG will be performed within 15 (±5) minutes after infusion end.

If a clinically significant abnormality is noted, the investigator or designee will evaluate whether study enrollment or continuation is appropriate; a clinically significant abnormality will be recorded under Medical History during Screening and as an AE thereafter.

### Electroencephalogram

A standard awake EEG will be recorded. If a clinically significant abnormality is noted, the investigator or designee will evaluate whether study enrollment or continuation is appropriate; a clinically significant abnormality will be recorded under Medical History during Screening.

### Pregnancy Testing

At Screening and at any time during the study, a female subject judged by the investigator to be of childbearing potential (as defined by onset of menses) will be tested for pregnancy with a urine pregnancy test; additional urine tests will be performed whenever pregnancy is in question. A serum pregnancy test will be performed if a urine test result is positive or equivocal.

### EXAMPLE 9

This example describes the study procedures of the study of Examples 4-8.

After the nature of the study has been explained, written informed consent by parent or authorized legal guardian must be obtained prior to any research-related procedures. Within 21 days of hospital admission to implant the ICV access device, the following procedures will be performed:
- Informed consent
- Confirm diagnosis of CLN2 disease determined by TPP 1 enzyme activity (dried blood spot)
- Blood for *CLN2* gene analysis
- Hamburg CLN2 disease rating scale (Figure 12) with videotaping
- Verify criteria for study entry
- Cranial MRI
- Complete physical examination, including medical history
- CLN2-specific QoL Questionnaire
- Pregnancy testing (in females of childbearing potential)
- After informed consent, assessment of SAEs related to protocol-imposed interventions and seizure history
- Concomitant medications

### Surgery Visit

Eligible study candidates will be admitted to the hospital for surgical implantation of an ICV access device to the right lateral ventricle. An MRI will be performed prior to surgery to ensure proper planning and placement of the ICV access device. At minimum, subjects will be observed in a nursing-intensive environment for 48 hours postoperatively. The following procedures will also be performed:
- AE assessment, including ongoing seizure history
- Concomitant medication

Following ICV access device placement, subjects and their caregivers will be given written instructions providing details on signs and symptoms of concern for device complications and instruction on when to return to the site for device evaluation. An additional follow-up phone call will be conducted within 48 hours of inpatient discharge.

### Baseline Visit and First Infusion

### Baseline Visit

The following *baseline* values will be recorded within 2 days before the first infusion (which will occur at least 14 days from surgery and no more than 28 days after surgery):
- Hamburg CLN2 disease rating scale (Appendix 1) with videotaping
- ECG (12-lead) (heart rate, rhythm, intervals, axis, conduction defects, and anatomic abnormalities)
- EEG (standard awake)
- Cranial MRI
- CSF (TAb and NAb in all subjects) and serum (TAb and drug-specific IgE in all subjects) for immunogenicity
- Complete physical examination
- Neurological examination
- Clinical laboratory testing (hematology, blood chemistry, and urinalysis)
- Modified Unified Batten Disease Rating Scale Involuntary Movement Scale (mUBDRS-Movement)
- Modified Unified Batten Disease Rating Scale Seizure Inventory (mUBDRS-Seizure)
- Optical coherence tomography
- Infant-toddler QOL questionnaire
- PedsQL
- EQ-5D-5L
- Denver II Developmental Scale
- CLN2-specific QoL Questionnaire
- Ophthalmologic assessments
- AE assessment, including ongoing seizure history
- Concomitant medications

### First Infusion

In general for all infusions, assessments of function and QOL should be completed before MRI and blood sampling; blood samples may be collected when subjects are sedated for MRI.

### First Infusion - Day 1

The following procedures will be performed on Day 1 of the first infusion:
- CSF surveillance (cell count, protein, glucose)
- Device patency/infection
- Study drug infusion
- CSF and plasma for biomarkers
- Vital signs within 30 (±5) minutes before infusion start, every 30 (±5) minutes during infusion, 0.5 and 1 hour (±5) after infusion end, and every 4 hours (±15 minutes) until discharge
- Brief physical examination
- AE assessment (investigator may collect additional blood samples for safety or immunogenicity testing for any AE of concern) and ongoing seizure history
- Concomitant medication assessment
- Post-infusion monitoring in an inpatient setting for a minimum of 24 hours

### First Infusion - Days 2-6

The following procedures will be performed on Days 2-6 following the first infusion:
- Vital signs (Day 2) (every 4 hours up to 20 hours after infusion end)
- Clinical laboratory testing (hematology, blood chemistry, and urinalysis) (Day 2)
- CSF surveillance (cell count, protein, glucose) (Day 6)
- Brief physical examination (Day 6)
- Device patency/infection (Day 6)
- AE assessment (investigator may collect additional blood samples for safety or immunogenicity testing for any AE of concern) (each day) and ongoing seizure history
- Concomitant medication assessment (each day)

Following inpatient discharge from the first infusion, a follow-up telephone call will be placed to parent/guardian within 48 hours.

### Every 2 Weeks

The following assessments and procedures should be performed every 2 weeks during the study. All study visits should occur every 2 weeks from the date of the first infusion (±3 days). All assessments and procedures should be completed before study drug infusion unless specified otherwise. In general, assessments of function and QOL should be completed before MRI and blood sampling; blood samples may be collected when subjects are sedated for MRI.

For all visits, if no safety issues are observed, a subject may be discharged after 24 hours if medically stable. A follow-up phone call to the parent or legal guardian will be conducted ~48 hours after discharge to determine health status.
- Brief physical examination
- CSF surveillance (cell count with differential, protein, glucose)
- Device patency/infection assessment
- Study drug administration
- Vital signs within 30 (±5) minutes before infusion start, every 60 (±5) minutes during infusion, 1 and 4 hours (±5 minutes) after infusion end
- AE assessment (investigator may collect additional blood samples for safety or immunogenicity testing for any AE of concern) and ongoing seizure history
- Concomitant medication assessment
- Telephone call to parent/guardian within 48 hours after visit

### Every 4 Weeks

The following assessments and procedures should be performed every 4 weeks during the study. Where applicable, blood sampling may occur during sedation for MRI.

Routine clinical laboratory tests (hematology, blood chemistry, and urinalysis)

### Every 12 Weeks

The following assessments and procedures should be performed every 12 weeks during the study. All assessments and procedures should be completed before study drug infusion unless specified otherwise. In general, assessments of function and QOL should be completed before MRI and blood sampling; blood samples may be collected when subjects are sedated for MRI.
- Hamburg CLN2 disease rating scale (Figure 12) videotaped
- Neurological examination
- CSF and serum sampling for immunogenicity
- mUBDRS-Movement
- mUBDRS-Seizure
- Optical coherence tomography
- Infant-toddler quality of life questionnaire
- PedsQL
- Denver II Developmental scale
- CLN2-specific QoL Questionnaire
- EQ-5D-5L

### Every 24 Weeks

The following assessments and procedures should be performed every 24 weeks during the study:
- ECG (12-lead) (heart rate, rhythm, intervals, axis, conduction defects, and anatomic abnormalities) within 15 (±5) minutes after infusion end
- EEG (standard awake)
- Cranial MRI
- CSF and plasma for biomarker assays
- Complete physical examination

### Every 48 Weeks

The following assessments and procedures should be performed every 48 weeks during the study:
- Ophthalmologic assessments

### Study Completion or Early Termination Visit

At Study Completion or Early Termination, subjects will return to the study site within 3 days. The following procedures will be completed:
- Hamburg CLN2 disease rating scale (Appendix 1) videotaped
- ECG (12-lead) (heart rate, rhythm, intervals, axis, conduction defects, and anatomic abnormalities) [if infusion, within 15 (±5) minutes after infusion end]
- Cranial MRI
- CSF surveillance (cell count, protein, glucose)
- CSF and plasma for biomarker assays
- CSF and serum sampling for immunogenicity
- Vital signs (SBP, DBP, heart rate, oral body temperature, and respiration rate)
- Complete physical examination
- Neurological examination
- Routine clinical laboratory tests (hematology, blood chemistry, and urinalysis)
- Denver II Developmental scale
- CLN2-specific QoL Questionnaire
- Ophthalmologic assessments
- AE assessment (investigator may collect additional blood samples for safety or immunogenicity testing for any AE of concern) and ongoing seizure history
- Concomitant medication assessment

Following the Study Completion Visit or Early Termination Visit, subjects who will not be continuing to receive TPP1 in another setting (e.g., commercial use, participation in a registry, participation in another TPP1 clinical study, etc.) should have their ICV access device removed. Removal of the device should occur no more than 4 weeks after the Study Completion Visit or ETV.

### Device and Safety Follow-Up

Subjects will return to the study site 4 weeks (±3 days) after removal of the ICV access device, when the following procedures will be completed:
- Vital signs (SBP, DBP, heart rate, oral body temperature, and respiration rate)
- Brief physical examination (including close examination of the former device site to check for signs of infection, etc.)
- Serum sampling for immunogenicity
- Neurological examination
- Routine clinical laboratory tests (hematology, blood chemistry, and urinalysis)
- AE assessment (investigator may collect additional blood samples for safety or immunogenicity testing for any AE of concern)
- Concomitant medication assessment

The 4-week Device Safety Follow-Up Visit will be waived for subjects who do not undergo device removal because they will be continuing to receive TPP 1 in another setting (e.g., commercial use, participation in a registry, participation in another TPP 1 clinical study, etc.).

### Safety Follow-Up

The Safety Follow-up visit is not required if a subject enrolls in an extension study or registry or otherwise continues to have access to TPP 1 within 6 months of the final infusion. If required, subjects will return to the study site 6 months after the last study treatment, when the following procedures will be completed:
- ECG (12-lead) (heart rate, rhythm, intervals, axis, conduction defects, and anatomic abnormalities) [if infused, within 15 (±5) minutes after infusion end]
- Serum sampling for immunogenicity
- Vital signs (SBP, DBP, heart rate, oral body temperature, and respiration rate)
- Complete physical examination
- Routine clinical laboratory tests (hematology, blood chemistry, and urinalysis)
- AE assessment (investigator may collect additional blood samples for
   safety or
   immunogenicity testing for any AE of concern) and ongoing seizure history
- Concomitant medication assessment

### Study Termination

The study will end after the last subject completes the last Safety Follow-Up visit. BioMarin reserves the right to discontinue the study any time for clinical or administrative reasons and to discontinue participation of an individual investigator or site for clinical or administrative reasons, including, but not limited to, poor enrollment or noncompliance with procedures of the protocol or GCP. In addition, the study may be terminated if, in the opinion of BioMarin, the safety of the study subjects may be compromised.

### EXAMPLE 10

This example describes results, modifications, and discussion of the study described in Examples 4-9.

### Drug Product and Flush Solution, Components and Formulation Development

The drug product of the study described in Examples 4-9 is a lyophilized injectable formulation that contained cerliponase alfa 150 mg per 5 mL of solution in a single 10 mL glass vial. The drug product contains the following excipients: disodium hydrogen phosphate pentahydrate, monosodium phosphate monohydrate, sodium chloride, potassium chloride, magnesium chloride, calcium chloride hydrate, and water for injection. A flush solution is supplied for the purpose of fully administering drug product that remains in the administration line to maintain line patency following the intracerebroventricular administration of the drug product. The composition of the flush solution is the same as the drug product, except the flush solution does not contain the active substance, and is supplied in the form of 5 mL of solution filled in a single 10 mL glass vial.

### Mechanism of Action of Cerliponase Alfa

Cerliponase alfa is a recombinant hTPP1 precursor consisting of 544 amino acids. The amino acid sequence is identical to that of hTPP1 precursor *in vivo.* N-linked oligosaccharides such as bis-phosphorylated high-mannose sugar chains are bound to the aspartic acid residues (Asn191, Asn203, Asn267, Asn294, and Asn424) of five N-linked oligosaccharide profiles and it is taken up into the target cell or lysosome by CI-M6PR via this oligosaccharides (J Biol Chem 2001; 276: 2249-55). After that, the pro-peptide fragment is cleaved by *in vivo* proteases in an acidic environment, yielding the active enzymes (e.g., J Biol Chem 2004; 279, 31058-67; J Bio Chem 2009; 284: 3985-97), and it cleaves tripeptides from the polypeptides which accumulate in the lysosome, preventing an increase in the storage materials, and thereby preventing CLN2 disease progression.

### Pharmacokinetics

In this study Brineura to non-Japanese CLN2 patients (target sample size: 5), Brineura was administered to the patients. The table below (Table 9) shows the CSF and plasma pharmacokinetic parameters in the individual patients who received intracerebroventricular doses of 200 or 300 mg.

**TABLE 9**

| **Pharmacokinetic Parameters in CSF and Plasma in Individual Patients Receiving Intracerebroventricular Doses of 200 or 300 mg^{a)}** | | | | | |
|---|---|---|---|---|---|
| Patient Number (Age at Baseline) | Dose (mg) | Measurement Time Point | Measured Substance | Cₘₐₓ (µg/mL) | AUC₀₋ₜ (µg·h/mL) |
| Patient 1 (2 years) | 300 | Week 97^{b)} | Plasma | 6.4 | 216 |
| Patient 2 (2 years) | 300 | Week 97^{b)} | Plasma | 3.19 | 66.9 |
| Patient 3 (2 years) | 300 | First dose | CSF | 508 | 2380 |
| | | | Plasma | 103 | 115 |
| | | Week 25^{c)} | CSF | 1540 | 6130 |
| | | | Plasma | 11 | 128 |
| Patient 4 (1 year) | 200 | First dose | CSF | 511 | 2720 |
| | | | Plasma | 9.46 | 727 |
| | 300 | Week 25^{c)} | CSF | 2.97 | 36 |
| | | | Plasma | 21.8 | 237 |
| Patient 5 (1 year) | 300 | Week 25^{c)} | CSF | 659 | 3720 |
| | | | Plasma | 15 | 148 |

| | | | | | |
|---|---|---|---|---|---|
| Cₘₐₓ: The maximum concentration in CSF or plasma AUCo-t: The area under the CSF or plasma concentration-time curve from administration to the last quantified time point t a) In this study, pharmacokinetic assessments were performed after the data cut-off date; the cerliponase alfa pharmacokinetic parameter data shown are those for the 5 patients who received Brineura between 2 dates. b) The 49th dose; c) The 13th dose | | | | | |

### Discussion of this study

The key inclusion criteria in this study was a decrease in TPP1 enzyme activity based on a blood test, age 1 year or more with a sibling enrolled in the study of Example 3, a total score on the motor and language subscales of 3 to 6 points,¹⁹ and no prior treatment with a stem cell therapy, gene therapy, or enzyme supplementation therapy.

This study consisted of a post-operative recovery period of 14 to 28 days following the procedure to implant the device for intracerebroventricular administration and a study drug treatment period of 96 weeks.

The dosage and administration was intracerebroventricular doses of TPP1 (Brineura) 300 mg once every 2 weeks over approximately 4 hours (infusion rate: 2.5 mL/h). The treatment period was 96 weeks.²⁰

Furthermore, the study protocol was revised later after data cut-off so that study participation would no longer be limited to the siblings of CLN2 patients enrolled in the study of Example 3. The eligible age for study participation was changed to birth to < 18 years, and the dosage and administration for patients younger than 2 years of age was changed to the following: for patients with age birth to < 0.5 years, 100 mg; for patients with age ≥ 0.5 year and < 1 year, 150 mg; and, for patients with age ≥ 1 year
¹⁹ This study (or portion thereof) was conducted in Germany and the US.
²⁰ The duration of exposure (mean ± SD) through the data cut-off date (month) was 30.5 ± 11.30 weeks.
and < 2 years, 200 mg for the first 4 doses, and then 300 mg once every 2 weeks thereafter. All doses were administered intracerebroventricularly at infusion rate of 2.5 mL/h.

Four treated patients (age at baseline: 2 to 5 years) were included in both the safety analysis set and the efficacy analysis set. For efficacy, Table 10 shows the baseline motor and language subscale scores, as well as the total thereof (the ML score), from the CLN2 clinical rating scale²¹ as well as the changes therein from baseline to the last assessment time point. No declines from baseline were found in any of the clinical rating scales; all were unchanged.

**TABLE 10**

| **Baseline Scores of Each Clinical Rating Scale and Changes Therein From Baseline to the Last Assessment Time Point (Study 109-203)** | | | | | | | | |
|---|---|---|---|---|---|---|---|---|
| | | Baseline Score | | | | | | |
| | | 0 | 1 | 2 | 3 | 4 | 5 | 6 |
| Baseline | Motor subscale (0 to 3 points) (4 patients) | 0 (0) | 2 (50) | 0 (0) | 2 (50) | - | - | - |
| | Language subscale (0 to 3 points) (3 patients)^{a)} | 0(0) | 1 (33) | 0(0) | 2 (67) | - | - | - |
| | ML subscale (0 to 6 points) (3 patients)^{a)} | 0 (0) | 0 (0) | 1 (33) | 0 (0) | 0 (0) | 0 (0) | 2 (67) |

| | | Change From Baseline to Last Assessment Time Point | | | | | | |
|---|---|---|---|---|---|---|---|---|
| | | -3 | -2 | -1 | 0 | 1 | 2 | 3 |
| Last Assessment Time Point | Motor subscale (4 patients) | 0 (0) | 0 (0) | 0 (0) | 4 (100) | 0 (0) | 0 (0) | 0 (0) |
| | Language subscale (3 patients)^{a)} | 0(0) | 0 (0) | 0 (0) | 3 (100) | 0(0) | 0(0) | 0(0) |
| | ML subscale (3 patients)) | 0 (0) | 0 (0) | 0 (0) | 3 (100) | 0 (0) | 0 (0) | 0 (0) |

| | | | | | | | | |
|---|---|---|---|---|---|---|---|---|
| Number (%) of patients a) Of the 4 patients who had been enrolled as of the data cut-off date, 1 had an ML subscale score at baseline of 1, and because this patient had concurrent autism, this patient was excluded from the language and ML subscale assessments. | | | | | | | | |

As far as safety was concerned, AEs 22 were reported in all 4 of the patients (upper respiratory tract infection, upper respiratory tract infection, malaise, seizure, seizure, partial seizures, partial seizures, constipation, and abdominal pain; pyrexia, sleep disorder, and pyrexia; influenza, pyrexia, and gastroenteritis; and vomiting, hypersensitivity, drop attacks, bronchitis, generalized tonic-clonic seizure, upper respiratory tract infection, upper respiratory tract infection, generalized tonic-clonic seizure, generalized tonic-clonic seizure, and gastroenteritis). Of these events, 3 (pyrexia in 2 patients and hypersensitivity in 1 patient) were considered ADRs.
²¹ The CLN2 clinical rating scale assessments were performed every 12 weeks.

No deaths were reported. Serious AEs were reported in 3 patients (pyrexia in 2 patients and hypersensitivity in 1 patient), and all of these events were considered ADRs. No AEs leading to treatment interruption were reported.

All 4 of the patients were positive for anti-cerliponase alfa antibodies in serum by 13 weeks after treatment initiation. Anti-cerliponase alfa antibodies were not detected in CSF in any of the patients by 37 weeks after treatment initiation.

### No clinically significant changes in vital signs or 12-lead ECG findings were reported.

### Efficacy

As far as the efficacy of Brineura in CLN2 patients younger than 3 years of age is concerned, in this study, after the data cut-off, the study protocol was revised to make the eligible study population patients with age from birth to < 18 years. According to the most recent data obtained,²⁷ Brineura has been administered to 11 patients, of whom 5 were younger than 3 years of age (3 were 2 years old and 2 were 1 year old). Table 11 shows the ML scores by age at baseline. From baseline to the last assessment time point, the ML scores did not change in 8 patients (4 patients maintained scores of 6 points, 3 patients maintained scores of 4 points, and 1 patient maintained a score of 2 points), the scores improved in 2 patients (1 patient's score improved from 5 points to 6 points, and 1 patient's score improved from 1 point to 2 points), and the score decline by 1 point in 1 patient (the patient's score declined from 4 points to 3 points). Of the 3 patients who were 2 years old, 2 patients maintained ML scores of 6 points, and the other patient's score improved by 1 point (from 5 points to 6 points). Both of the patients who were younger than 2 years old maintained ML scores of 6 points.

**TABLE 11.**

| Distribution of ML Scores at Baseline and the Final Assessment Time Point | | | | | | | | | |
|---|---|---|---|---|---|---|---|---|---|
| | Baseline Age Category ML Score | | | | | | | | |
| | | | 0 | 1 | 2 | 3 | 4 | 5 | 6 |
| Baseline | < 3 years (5 patients) | < 2 years (2 patients^{a)}) | 0 (0) | 0 (0) | 0 (0) | 0 (0) | 0 (0) | 0 (0) | 2 (100) |
| | | 2 years (3 patients) | 0 (0) | 0 (0) | 0 (0) | 0 (0) | 0 (0) | 1 (33.3) | 2 (66.7) |
| | ≥ 3 years (6 patients) | | 0 (0) | 1 (16.7)^{b)} | 1 (167) | 1 (167) | 3 (50.0) | 0 (0) | 0 (0) |
| | Overall (11 patients) | | 0 (0) | 1 (9.1)^{b)} | 1 (9.1) | 1 (9.1) | 3 (27.3) | 1 (9.1) | 4 (36.4) |
| Last Assessment Time Point | < 3 years (5 patients) | < 2 years (2 patients^{a)}) | 0 (0) | 0 (0) | 0 (0) | 0 (0) | 0 (0) | 0 (0) | 2 (100) |
| | | 2 years (3 patients) | 0 (0) | 0 (0) | 0 (0) | 0 (0) | 0 (0) | 0 (0) | 3 (100) |
| | > 3 years (6 patients) | | 0 (0) | 0 (0) | 2 (33.3)^{b)} | 1. (167) | 3 (50.0) | 0 (0) | 0 (0) |
| | Overall (11 patients) | | 0 (0) | 0 (0) | 2 (182)^{b)} | 1 (91) | 3 (27.3) | 0 (0) | 5 (45.5) |

| | | | | | | | | | |
|---|---|---|---|---|---|---|---|---|---|
| Number (%) of patients | | | | | | | | | |

²⁷ The duration of exposure (mean ± SD) through recent dateswas 67.2 ± 32.48 weeks. The duration of exposure in patients with age < 2 years and in patients with age ≥ 2 years was 43.2 ± 0.91 weeks and 72.6 ± 33.80 weeks, respectively.
a) Both of the patients who were younger than 2 years of age were 1 year old at baseline.
b) This data includes the MS Score of the patient with autism who was described in Table 29, a).

As far as the efficacy in patients younger than 3 years of age is concerned, the results confirmed that there was no worsening of symptoms during Brineura therapy.

In light of the above, it is believed that the use of Brineura can be expected to be effective at preventing disease progression in CLN2 in younger patients.

### Safety and Serious Adverse Events

Regarding safety in CLN2 patients aged <3years old: In this study, based on the latest data, Brineura was administered in 11 patients (6 patients aged >=3 years old, 3 patients aged 2 years old, and 2 patients aged 1 year old), and all 11 patients reported AEs. Out of them, the events in 3 patients aged >=3 years old (1 to 3 episodes of pyrexia occurred per patient) and 3 patients aged <3 years old (pyrexia and hypersensitivity in 2 patients aged 2 years old; drug hypersensitivity and drug hypersensitivity in 1 patient aged 1 year old) were assessed as ADR, and the outcome of all these events were "recovered". SAEs were reported in 6 patients aged >=3 years old (escherichia urinary tract infection, gastrointestinal fistula, and pneumonia; propionibacterium test positive; pyrexia, adenoidal hypertrophy and rhinitis; complication of device insertion; periorbital haematoma; and pyrexia, dental caries, and pyrexia) and 3 patients aged <3 years old (status epilepticus and infection; pyrexia, influenza and influenza; and hypersensitivity and medical device site haematoma, in patients aged 2 years old). Out of them, the events in 2 patients aged >=3 years old (pyrexia and pyrexia; and pyrexia) and the events in 2 patients aged <3 years old (pyrexia and hypersensitivity in patients aged 2 years old) were assessed as ADR, and the outcomes of all these events were "recovered". Based on the above, no new clinically significant AEs were observed in patients aged <3 years old in this study.

Based on the incidence of AEs in the clinical studies and overseas port-marketing experience, the safety in Brineura infusion is considered as acceptable.

In conclusion a certain level of efficacy can be expected for Brineura and safety of Brineura appears acceptable when intracerebroventricular administered based on prior studies in CLN2 patients aged ≥ 3 years and this study in CLN2 patients including those aged < 3 years..

### Dosage and Administration

The intracerebroventricular route was decided for the administration of Brineura by using a surgically placed implantable intracerebroventricular device to bypass the blood-brain barrier, directly and extensively deliver the enzymes to the central nervous system, thereby achieving the efficacy against neurological symptoms.

In a primary pharmacodynamic study in TPP1-deficient dogs (dachshund), intracerebroventricular administration of 16 mg cerliponase alfa every 2 weeks ³⁾ prolonged the time to onset of neurological symptoms with a trend of prolonged survival. Based on the fact that TPP1 activity in brain tissue is more closely related to accumulated lysosomal substances in the central nervous system, clinical doses were investigated based on the scaling factor for human dose by brain weight. Taking into account that human brain reaches 75% of the adult brain weight by the age of 2 years and nearly 100% by the age of 5 years (Cereb Cortex 1996; 6:551-60), brain weight aged 2 to 7 years can range from 1050 to 1400 g based on 1400 g of mean adult brain weight. Considering the possibility of progressive cerebral atrophy in CLN2 patients, the applicant assumed that the brain weight in CLN2 patients in this age group could be approximately 1000 g. Since the mean brain weight of dachshund was estimated to be 50 g (Peptide Drug Delivery to the Brain, Raven Press, 1991:112), the scaling factor for deciding the human dose was 20-fold, and the dose of 16 mg cerliponase alfa administered to TPP1-deficient dogs (dachshund) was considered to be 320 mg for those aged ≥ 2 years based on the brain weight ratio between the species. Then, the study described in Example 3 in CLN2 patients aged ≥ 3 years was initiated with e.g. a cohort starting at 30 mg and titrating to 300 mg from safety considerations. After confirming the tolerability, the efficacy and safety of 300 mg were evaluated, including the fixed-dose period. For this study, the protocol was revised (after data cut-off). The dose was selected based on the brain weight for patients aged < 2 years³⁹ for investigating the efficacy and safety of Brineura: < 100 mg for birth to patients aged 0.5, 150 mg for patients aged 0.5 to < 1 year, 200 mg for patients aged 1 year to < 2 years for the first four doses and 300 mg for the subsequent doses. In patients aged 1 year to < 2 years, the dose is estimated to be 300 mg based on their brain weights. However, the initial dose was set at 200 mg as an intermediate dose for patients aged 0.5 to < 1 year who started treatment with Brineura and increased the dose from 150 mg to 300 mg according to their age based on safety considerations. As for the dosing interval, Brineura was to be administered at a dose interval of once every two weeks in the clinical study, because the results of tissue distributions in non-clinical
39 Brain weights in female/male pediatric patients have been reported to be 360/380 g to 580/640 g in the age range of birth to < 0.5 year, 640/680 g to 940/970 g in the age range of 0.5 to < 1 year, and 940/970 g to 1040/1120 g in the age range of 1 year to < 2 years (AnnNeurol 1978; 4:345-56). pharmacokinetic studies, etc. suggest that exposure to drug actives in the CNS was expected to be maintained at a dose interval of once every two weeks.

For children below 2 years, decrease the dose referring the following table (Table 12).

**TABLE 12**

| **Age Groups** | **Each Dose** |
|---|---|
| Birth to < 6 months | 100 mg |
| 6 months to < 1 year | 150 mg |
| 1 year to < 2 years | Dose until first 4 doses: 200 mg |
| | Dose from 5^{th} doses: 300 mg |

Usually, this drug is infused at a rate of 2.5 mL/hour by using infusion pump, but decreases the infusion rate depending on patient's condition.

Hypersensitivities including anaphylaxis may occur when this drug administered. To alleviate the symptoms, consider pre-treatment of patients with antihistamines with or without antipyretics is 30 to 60 minutes prior to the start of infusion.

For infusion rate in the clinical studies, Brineura was intracerebroventricular administered at an infusion rate of 2.5 mL/h at all ages. If the infusion rate was estimated to be approximately 100 mL of CSF in patients aged 2 to 7 years and approximately 50 mL of CSF in patients aged < 1 year (Peptide Drug Delivery to the Brain, Raven Press 1991:112), the infusion volume of Brineura was estimated to be less than approximately 10% of the CSF. The CSF production rate in humans is generally about 20 mL/h (Am J Physiol 1962; 203:763-74), which is approximately 12% of the Brineura infusion rate (2.5 mL/h), and the CSF production rate at the age of 0.5 year (estimated to be approximately 2.5-4.7 mL/h)⁴⁰ is also assumed to be greater than the Brineura infusion rate (2.5 mL/h). Based on these data, the intracerebroventricular infusion rate of Brineura does not appear to have a significant effect on human CSF or CSF production rate.
40 A study in 100 pediatric hydrocephalus patients (61 males, 39 females, ages 0.02 year to 15.7 years) showed that CSF production (mean ± SD) calculated on the basis of ventricular drain output was 8.1±5.2 mL/h and correlated with age and body weight (Pediatr Neurosurg 2002;36:22-8), thus estimating the CSF production rate at 0.5 year based on the regression formula [CSF production per hour = 2.78-2.23 (males = 0, females = 1) ± 0.97 log (age [years old]) ± 2.26log (body weight [kg]).

Considering the above, it was demonstrated that Brineura was effective in inhibiting the progression of the disease and did not pose any major safety concerns based on the results of prior studies where 300 mg was intracerebroventricular administered to CLN2 patients aged ≥ 3 years once every for 2 weeks, and this study where 300 mg or less was intracerebroventricular administered once every for 2 weeks to CLN2 patients aged < 3 years.

### REFERENCES CITED IN EXAMPLES 4-9

The following references are cited herein according to the numbering below.
Arkin, LM, Sondhi, D, Worgall, S, Suh, LH et. al. Confronting the issues of therapeutic misconception, enrollment decisions, and personal motives in genetic medicine-based clinical research studies for fatal disorders. Hum Gene Ther 16[9], 1028-1036. 2005.
Awano, T, Katz, ML, O'Brien, DP, Sohar, I et. al. A frame shift mutation in canine TPP1 (the ortholog of human CLN2) in a juvenile Dachshund with neuronal ceroid lipofuscinosis. Mol Genet Metab 89[3], 254-260. 2006.
Brooks R. EuroQol: the current state of play. Health Policy 37[1], 53-72. 1996.
Chang, M. CLN2. The Nueronal Ceroid Lipofuscinoses. New York: Oxford Univ Press, 2011: 80¬109.
Crystal, RG, Sondhi, D, Hackett, NR, Kaminsky, SM et. al. Clinical protocol. Administration of a replication-deficient adeno-associated virus gene transfer vector expressing the human CLN2 cDNA to the brain of children with late infantile neuronal ceroid lipofuscinosis. Hum Gene Ther 15 [11], 1131-1154. 2004.
Dierenfeld, AD, McEntee, MF, Vogler, CA, Vite, CH et. al. Replacing the enzyme alpha-L-iduronidase at birth ameliorates symptoms in the brain and periphery of dogs with mucopolysaccharidosis type I. Sci Transl Med 2[60], 60ra89. 2010.
Dyke, JP, Sondhi, D, Voss, HU, Shungu, DC et. al. Assessment of Disease Severity in Late Infantile Neuronal Ceroid Lipofuscinosis Using Multiparametric MR Imaging. AJNR Am J Neuroradiol . 2012.
EuroQol Group. EuroQol - a new facility for the measurement of health-related quality of life. Health Policy 16[3], 199-208. 1990.
Giedd, JN, Snell, JW, Lange, N, Rajapakse, JC et. al. Quantitative magnetic resonance imaging of human brain development: ages 4-18. Cereb Cortex 6[4], 551-560. 1996.
Karavelis A, Foroglou G, Selviaridis P et al. Intraventricular administration of morphine for control for intractable cancer pain in 90 patients. Neurosurgery 39[1], 57-62. 1996.
Kronenberg MF, Laimer I, Rifici C et al. Epileptic seizure associated with intracerebroventricular and intrathecal morphine bolus. Pain 75[2], 383-387. 1998.
Kurachi, Y, Oka, A, Mizuguchi, M, Ohkoshi, Y et. al. Rapid immunologic diagnosis of classic late infantile neuronal ceroid lipofuscinosis. Neurology 54[8], 1676-1680. 2000.
Kwon JM, Adams H, Rothberg PG, et al. Quantifying physical decline in juvenile neuronal ceroid lipofuscinosis (Batten disease). Neurology 77[20], 1801-1807. 2011.
Lishner M, Perrin RG, Feld R et al. Complications Associated with Ommaya Reservoirs in Patients with Cancer: The Princess Margaret Hospital Experience and a Review of the Literature. Arch Intern Med 150[1], 173-176. 1990.
Msall ME. Measuring functional skills in preschool children at risk for neurodevelopmental disabilities. Ment. Retard. Dev. Disabil. Res. Rev. 11, 263-273. 2005.
Seitz, D, Grodd, W, Schwab, A, Seeger, U et. al. MR imaging and localized proton MR spectroscopy in late infantile neuronal ceroid lipofuscinosis. AJNR Am J Neuroradiol 19[7], 1373-1377. 1998.
Sleat, DE, El-Banna, M, Sohar, I, Kim, KH et. al. Residual levels of tripeptidyl-peptidase I activity dramatically ameliorate disease in late-infantile neuronal ceroid lipofuscinosis. Mol Genet Metab. 94, 222-233. 2008.
Sleat, DE, Wiseman, JA, El-Banna, M, Kim, KH et. al. A mouse model of classical late-infantile neuronal ceroid lipofuscinosis based on targeted disruption of the CLN2 gene results in a loss of tripeptidyl-peptidase I activity and progressive neurodegeneration. J Neurosci 24[41], 9117-9126. 2004.
Steinfeld, R, Heim, P, von Gregory, H, Meyer, K et. al. Late infantile neuronal ceroid lipofuscinosis: quantitative description of the clinical course in patients with CLN2 mutations. Am J Med Genet 112[4], 347-354. 2002.
Vuillemenot, BR, Katz, ML, Coates, JR, Kennedy, D et. al. Intrathecal tripeptidyl-peptidase 1 reduces lysosomal storage in a canine model of late infantile neuronal ceroid lipofuscinosis. Mol Genet Metab 104[3], 325-337. 2011.
Vuillemenot, BR, Kennedy, D, Reed, RP, Boyd, RB, et. al. Recombinant human tripeptidyl peptidase-1 infusion to the monkey CNS: safety, pharmacokinetics, and distribution. Toxicol Appl Pharmacol 277[1], 49-57. 2014.
Worgall, S, Kekatpure, MV, Heier, L, Ballon, D et. al. Neurological deterioration in late infantile neuronal ceroid lipofuscinosis. Neurology 69[6], 521-535. 2007.
Worgall, S, Sondhi, D, Hackett, NR, Kosofsky, B et. al. Treatment of late infantile neuronal ceroid lipofuscinosis by CNS administration of a serotype 2 adeno-associated virus expressing CLN2 cDNA. Hum Gene Ther 19[5], 463-474. 2008.
Xu, S, Wang, L, El-Banna, M, Sohar, I et. al. Large-volume intrathecal enzyme delivery increases survival of a mouse model of late infantile neuronal ceroid lipofuscinosis. Mol Ther 19[10], 1842¬1848. 2011.

### EXAMPLE 11

This Example describes additional pharmacokinetic and pharmacodynamic analysis carried out on patient samples from the Phase1/2 study of Example 3.

*Pharmacokinetic analysis:* CSF and blood (plasma) samples for pharmacokinetic analysis were collected following the initial dose, the first dose at each new dose level during the dose escalation phase, and Week 5 and Week 13 of the stable dose phase. Samples were collected pre-dose (within 0.25 hours prior to start of infusion), and 0.25, 4, 8, 20, 72, and 120 hours after end of infusion. Additional CSF and blood (plasma) samples were collected pre-dose at the start and every 4 weeks of the stable dose phase whenever serial samples were not collected. CSF samples were obtained from the lateral ventricle of the brain using the ICV port.

CSF and plasma samples were assayed for concentrations of cerliponase alfa by validated electrochemiluminescence immunoassay (ECLA) methods (BioMarin Pharmaceutical Inc., Novato, CA, US). The lower limit of quantitation (LLOQ) was 20 ng/mL in CSF and 16 ng/mL in plasma. Both inter-assay accuracy (absolute % relative error) and precision (% coefficient of variation) of quality controls were ≤13.2% in CSF and ≤17.2% in plasma throughout sample testing runs.

PK parameters were estimated based on concentration-time data in CSF and plasma by non-compartmental analysis (NCA) using Phoenix WinNonlin 6.4 (Pharsight Corporation, Cary, NC, USA). Maximum concentration (Cₘₐₓ) and time of maximum concentration (Tₘₐₓ) were recorded directly from the observed data. Other PK parameters estimated were elimination half-life (t_{1/2}); area under the concentration-time curve from time 0 to the time of last measurable concentration (AUC₀₋ₜ), estimated by the linear trapezoidal rule; area under the concentration-time curve extrapolated to infinity (AUC_{0-∞}); clearance of the absorbed fraction (CL); volume of distribution based on the terminal phase (V_{z}); and steady-state volume of distribution (Vₛₛ).

*Immunogenicity Analysis:* CSF and blood (serum) samples for immunogenicity were collected at baseline, every 4 weeks during the dose escalation phase, and at the start and every 4 weeks thereafter of the stable dose phase. CSF and serum samples were tested for total anti-drug antibodies (TAb) specific to cerliponase alfa by validated bridging electrochemiluminescence assays (BioMarin Pharmaceutical Inc., Novato, CA, US). TAb-positive samples in CSF were further characterized using a validated cell-based flow cytometry assay (BioMarin Pharmaceutical Inc., Novato, CA, US) for neutralizing antibodies (NAb) that block the uptake of cerliponase alfa into the lysosome. NAb testing was performed only in CSF samples as the target site of action is the CNS, and TAb-positive samples were tested for NAb response. Full immunogenicity methods and results from this study were previously reported [Cherukuri-2018].

*Statistical Analysis:* Demographic characteristics were summarized for the PK population. PK parameters were summarized descriptively by biological matrix, dose group, and study visit. The relationship of PK parameters to demographic characteristics, immunogenicity, safety, and efficacy parameters were assessed graphically since analyses of the PK population were not powered to assess statistical significance. For analyses without time as a covariate, mean PK parameters for each patient was used as the representative measure of an individual patient's exposure during treatment with 300 mg QOW. Mean PK parameters were derived by calculating the means of Cₘₐₓ and AUC₀₋ₜ values across study visits with 300 mg QOW dosing and intensive PK sampling (i.e., first dose at 300 mg, and Weeks 5 and 13 of the stable dose phase).

### Results

PK parameters were estimated in all patients, over various dose levels and study visits. At 300 mg QOW, there were 24 patients with evaluable PK data in CSF versus 15 patients with evaluable PK data in plasma.

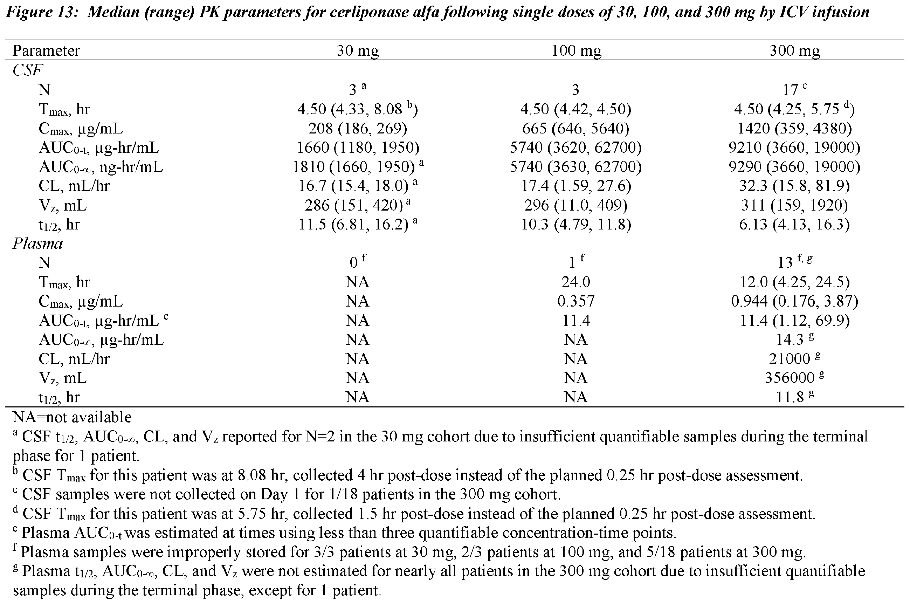

Single-dose PK data were available from patients who received an initial dose of 30 (n=3), 100 (n=3), or 300 mg (n=17); 4/4 from dose escalation phase and 13/14 enrolled directly into stable dose phase) of cerliponase alfa (Figure 13).

In CSF, peak concentrations were observed at the first sampling time point after the end of 4-hour infusion and appeared to decline in a biphasic manner. CSF exposure increased less than dose proportional with approximately 5 to 7-fold increase in median Cₘₐₓ and AUC versus the 10-fold increase in dose from 30 to 300 mg. One patient in the 100 mg group had high exposure following their initial dose, and accordingly, exposure parameters for the 100 mg dose level were highly variable due to small sample size. Cₘₐₓ and AUC for this patient (shown by the maximum value reported for the 100 mg group) were higher than median values for the 300 mg group. Although no conclusive findings were revealed to account for this outlier exposure, CSF exposures for this patient following subsequent infusions of 300 mg were less than their exposure following the initial 100 mg dose.

During the initial stages of study conduct, plasma PK samples were stored outside of the stability range, and thus, no data are available for the 30 mg group and available for only one patient in the 100 mg group. Based primarily on the 300 mg dose level, concentrations in plasma peaked between 8 to 20 hours after the end of 4-hour ICV infusion and appeared to decline in a biphasic manner, remaining above the lower limit of quantitation (LLOQ) through 72 hours.

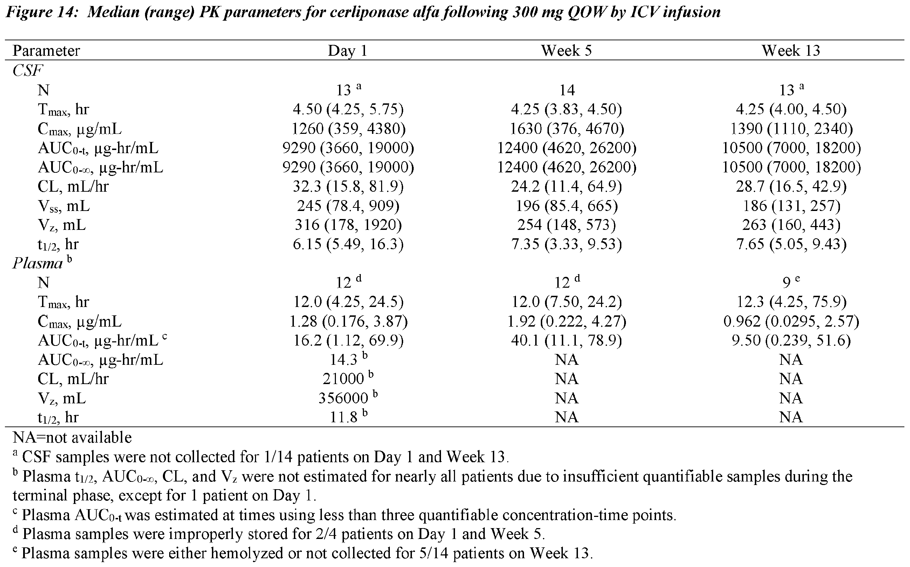

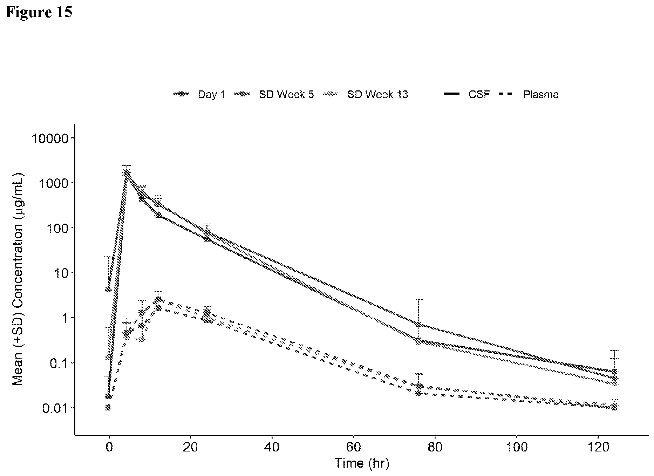

Multiple-dose PK data were evaluated from patients enrolled directly into the stable dose phase (n=14), who received 300 mg of cerliponase alfa QOW throughout the study (Figure 14-15).

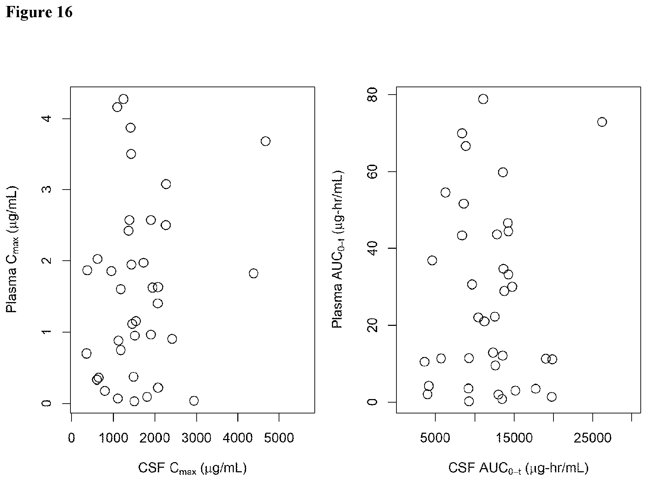

PK parameters in CSF were similar between Day 1, Week 5, and Week 13 visits. While variable, plasma Tₘₐₓ, Cₘₐₓ, and AUC₀₋ₜ were comparable with no discernible trends across visits. With ICV administration of 300 mg QOW, median Cₘₐₓ in plasma was approximately 1000-fold lower than in CSF and median AUC₀₋ₜ in plasma approximately 300 to 1000-fold lower than in CSF. There was no apparent correlation between the magnitude of either Cₘₐₓ or AUC₀₋ₜ in CSF versus in plasma based on patient- and visit-matched PK (Figure 16). Inter-individual variability of Cₘₐₓ and AUCo-t, respectively, were 26-73% and 31-49% in CSF versus 54-89% and 59-103% in plasma across visits. Intra-individual variability of Cₘₐₓ and AUC₀₋ₜ across visits, respectively, were 33% and 24% in CSF versus 69% and 80% in plasma.

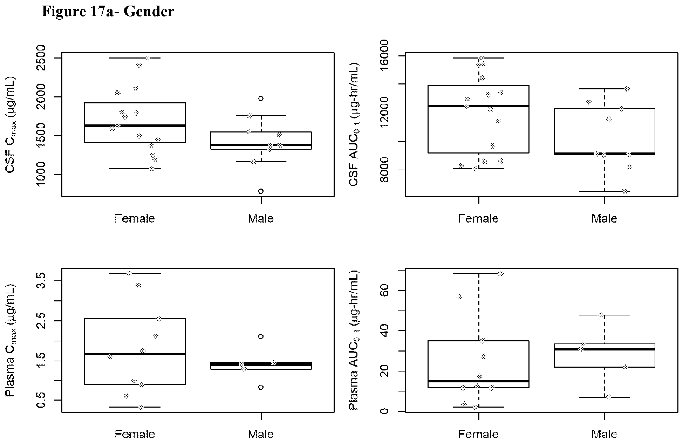

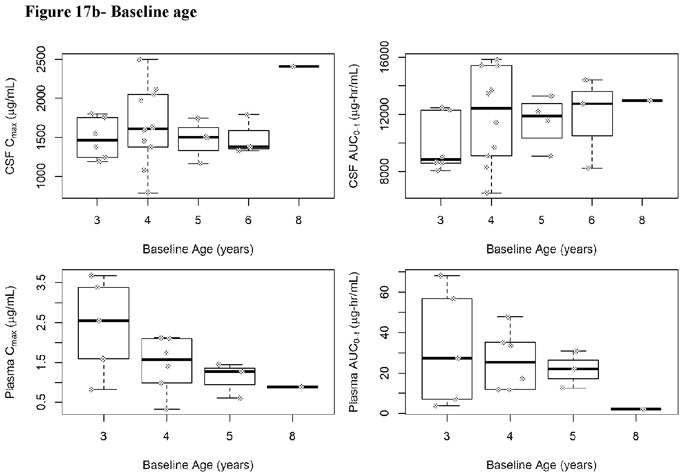

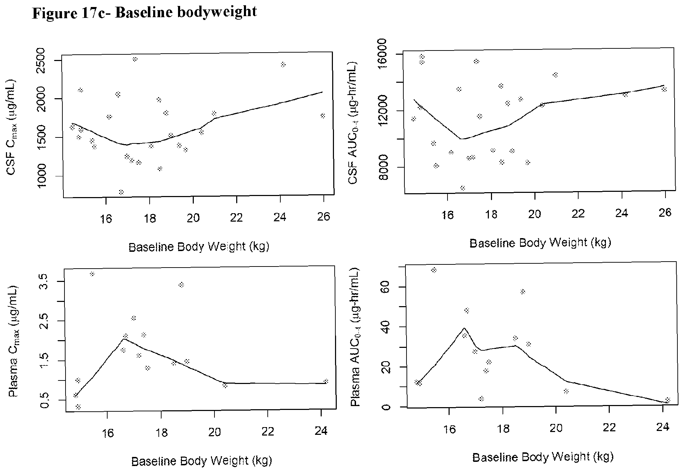

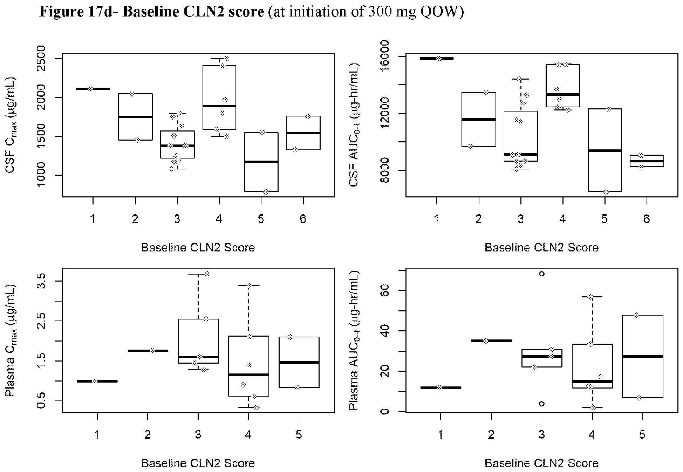

*Pharmacokinetics and Patient Characteristics:* The potential impact of baseline patient characteristics on cerliponase alfa PK was evaluated for the 300 mg QOW regimen. Mean estimates of Cₘₐₓ and AUC₀₋ₜ were used to represent an individual patient's exposure over the course of therapy and was deemed appropriate due to the lack of drug accumulation or time-dependent PK with 300 mg QOW. There was no apparent effect of baseline gender, age, bodyweight, or CLN2 score on the exposure of cerliponase alfa in CSF or plasma (Figure 17A-17D). There was a slight trend of increasing plasma Cₘₐₓ with decreasing age but was not exhibited of plasma AUC₀₋ₜ.

*Pharmacokinetics and Immunogenicity*: Total antibodies (TAbs) against cerliponase alfa were detected in CSF of 5/24 (21%) patients and in serum of 19/24 (79%) patients over the study duration. CSF TAb response was first detected at Week 13 of the stable dose phase, while serum TAb response was detected at the earliest time point sampled, Week 5 of the dose escalation phase. Neutralizing antibodies (NAbs) were not detected in CSF of any of the 5 patients with CSF TAb positivity, and thus, unavailable for further analysis.

To determine whether cerliponase alfa PK is affected by the development of anti-drug antibodies (ADA), Cₘₐₓ and AUC₀₋ₜ on visits with positive TAb response were compared to visits with negative TAb response. Visit-matched exposure parameters and ADA status (i.e., on Day 1, stable dose Week 5, and stable dose Week 13) were assessed from all patients who initiated treatment at 300 mg and had evaluable PK and ADA data. Eighteen patients with CSF data (4/4 from dose escalation phase and 14/14 enrolled directly into stable dose phase) and 14 patients with plasma/serum data (1/4 from dose escalation phase and 13/14 from stable dose phase) were included for analysis.

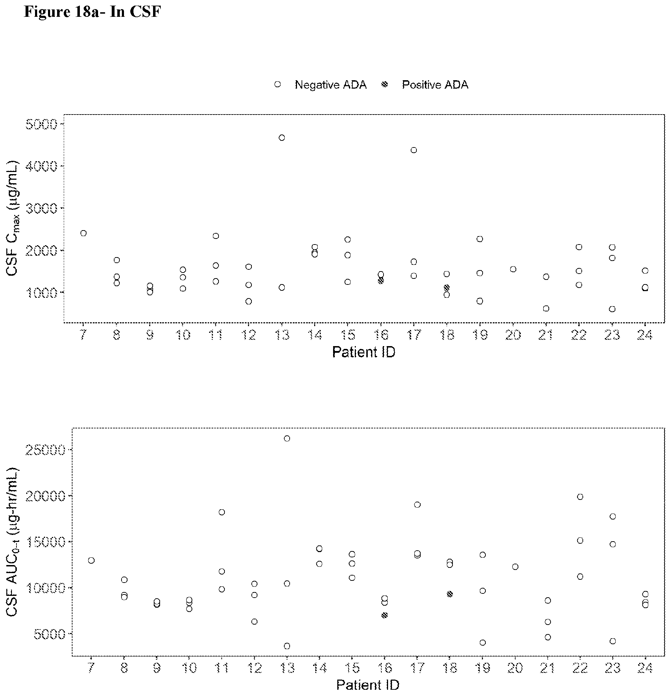

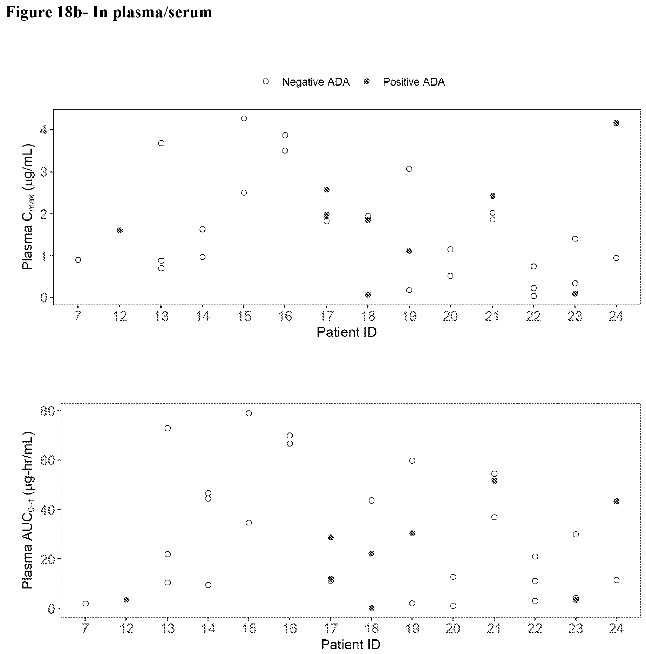

As displayed in Figure 18A, there was no discernible trend in CSF Cₘₐₓ and AUC₀₋ₜ by CSF ADA status across patients. CSF Cₘₐₓ and AUC₀₋ₜ values on visits with positive ADA response were well within the distribution of exposure values with negative ADA response. For the two patients with visit-matched PK and CSF ADA positivity, CSF AUC₀₋ₜ was lower by 17-27% on the ADA-positive visit compared to ADA-negative visits. No association was observed between plasma Cₘₐₓ and AUC₀₋ₜ with serum ADA status, across and within patients (Figure 18b). Plasma exposure on visits positive for serum ADA spanned the range of exposure on ADA-negative visits within an individual patient.

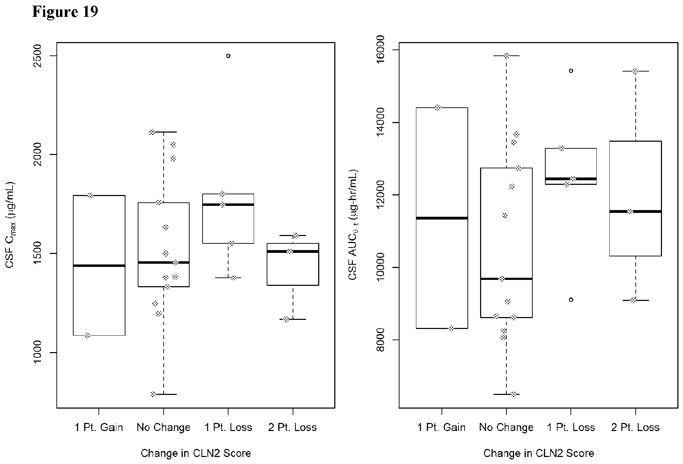

The relationship between cerliponase alfa PK and efficacy outcomes was evaluated using the change in motor-language score from the start of 300 mg QOW to the end of study. Of 23 patients in this analysis, 2 had a one-point gain, 13 had no change, 5 had a one-point loss, and 3 had a two-point loss after 48 weeks of treatment for an overall responder rate of 87% (20/23). A patient's change in motor-language score at Week 48 did not correlate with the individual mean Cₘₐₓ and AUC₀₋ₜ in CSF (Figure 19). Patients with a decline in score had CSF exposure parameters within the distribution of those with no change or gain in score. Similarly, no correlation was shown when assessed by the maximum decrease in a patient's score during 48 weeks of treatment.

The relationship between PK and adverse events was also analyzed. As noted above, study drug-related events that occurred in at least 10% of the study population were included for analysis: pyrexia 46% (11/24), hypersensitivity 33% (8/24), seizure 33% (8/24), epilepsy 17% (4/24), headache 13% (3/24), and vomiting 13% (3/24). Between patients with and without pyrexia, hypersensitivity, seizure, or epilepsy, Cmax and AUC0-t in CSF or plasma were not significantly different. There were slight trends of higher CSF exposure for patients with headache and higher exposure in both CSF and plasma for patients with vomiting compared to those without. Exposure in patients with headache or vomiting generally did not exceed the highest exposure observed in patients without either of the events.

The results above show that cerliponase alfa demonstrated less than dose proportional increase in CSF exposure following initial ICV infusions of 30, 100, and 300 mg. CSF Cₘₐₓ across the single-dose range (2.08×10⁵, 6.65×10⁵, and 1.42×10⁶ ng/mL respectively) were generally consistent with expected values for the amount of ICV dose administered to ~100 mL of CSF within a human brain (3.00×10⁵, 1.00×10⁶, and 3.00×10⁶ ng/mL respectively) (Pardridge et al., J. Cereb. Blood Flow Metab. 17, 713-731, 1997). At 300 mg QOW, there was no apparent accumulation or time-dependence in CSF or plasma PK based on comparable Cₘₐₓ, AUC, CL, and Vₛₛ across study visits. This is in line with the CSF half-life of 6.2-7.7 hours across patients and the calculable plasma half-life in one patient of 11.8 hours, given the biweekly dosing frequency. It should be emphasized that CSF half-life does not directly reflect the target site, for which the CNS tissue half-life (from assessment in monkeys) and lysosomal half-life (from ex vivo human fibroblasts) of days to weeks are most pertinent to the rationale for therapeutic biweekly dosing. In patients, CSF concentrations were greater than the lysosomal kᵤₚₜₐₖₑ for ~4 days, which based on animal data, suggest widespread distribution of the enzyme to CNS tissues. This was supported by estimates of CSF volume of distribution, which exceeded the typical CSF volume of ~100 mL. Direct administration of cerliponase alfa to the internal CSF spaces of the brain resulted in approximately three orders of magnitude greater exposure than in the periphery, with no correlation in the magnitude of Cₘₐₓ or AUC between CSF and plasma; indicating plasma PK is not a good surrogate for CSF PK. Plasma Tₘₐₓ was 8 hours after completion of 4-hour ICV infusion compared to CSF Tₘₐₓ, which occurred immediately after end of infusion. The blood-CSF barrier is leaky compared to the BBB, and thus, ICV-administered drug is transported out of the brain through CSF flow tracks and absorbed into the peripheral bloodstream across the arachnoid villi (Pardridge et al., Fluids Barriers CNS. 8:7, 2011).

Variability in CSF and plasma PK between patients was not explained by patient demographics, as intrinsic factors did not appear to correlate with cerliponase alfa exposure. The ICV dose of cerliponase alfa was designed according to brain mass and thus, CSF exposure would not be expected to change considerably for the range of age (3-8 years) and bodyweight (14.5-26.0 kg) in this study. The human brain on average achieves about 75% of adult mass by age 2 and 100% by age 5, with progressively decreasing brain-to-body weight ratio during development (Giedd et al., Cereb. Cortex. 6:551-560, 1996). Between ages 3 to 8-9 years, non-diseased human brains weigh on average 1.09-1.18 kg for females and 1.27-1.37 kg for males, compared to bodyweights of 14.1-26.0 kg and 15.6-27.5 kg, respectively (Dekaban et al., Ann. Neurol. 4, 345-356, 1978). The change in brain weight is thus only 8% across the age range in this study versus an 80% change in bodyweight. Of note, the slight trend of increasing plasma Cₘₐₓ with decreasing age is likely attributable to the disproportionate change in body versus brain weights during early childhood. While matched in age by brain weight, the amount of ICV dose is absorbed into a significantly smaller bodyweight and correspondingly smaller blood volume, resulting in more concentrated systemic exposure.

The between-patient variability in CSF exposure may rather be attributable to differences in disease severity than variability inherent to the ICV-administered enzyme, since within-patient variability was far less (33% for CSF Cₘₐₓ and 24% for CSF AUC₀₋ₜ) than between-patient variability. Although no association between CSF exposure and baseline CLN2 score was shown, there may be pathological effects to the CNS that are reflected in CSF PK but not translated to a change in clinical rating score. As systemic absorption occurs thereafter, inter- and intra-patient variability in plasma PK were both considerably higher than in CSF, contributed in part by the insufficient number of plasma samples with quantifiable concentrations.

Based on patient- and visit-matched analysis of 300 mg QOW, the presence of ADA in CSF and serum did not appear to have an impact on PK in CSF and plasma, respectively. The majority of treated patients developed ADA in serum, suggesting that plasma exposure of cerliponase alfa will likely lead to ADA positivity in serum, consistent with other ERTs as patients are deficient in endogenous protein (Long et al., Clin. Ther. 39:118-129, 2017). Development of an ADA response in this study was previously demonstrated not to be predictive of an adverse safety profile or poor treatment outcome (Cherukuri et al., Clin. Immunol. 197:68-76, 2018). For the most common adverse events related to cerliponase alfa, there were no apparent correlations between CSF or plasma exposure and the incidence of pyrexia, hypersensitivity, seizure, or epilepsy. Slight trends of increased exposure with incidences of headache and vomiting were limited for interpretation, given the low frequency amidst a small sample size (3/24 patients) for both events.

Response to treatment, as measured by change in CLN2 score after 48 weeks of 300 mg QOW, did not appear to correlate with magnitude of CSF exposure, indicating maximum benefit was obtained across the range of exposures with 300 mg QOW. Of note, CSF exposures in CLN2 patients exceeded those associated with the efficacious 16 mg dose in TPP1-null dogs, suggesting these exposures were within the plateau of an exposure-response relationship (Katz et al., J. Neurosci. Res. 92, 1591-1598, 2014; Vuillemenot et al., Mol. Genet. Metab. 114, 281-293, 2015). Despite the inter- and intra-patient PK variability, 91% (62/68) of visits with reportable CSF AUC₀₋ₜ at the clinical 300 mg dose had values above the mean CSF AUC₀₋ₜ in TPP1-null treated dogs (6.45×10⁶ ng-hr/mL) (Vuillemenot 2015, *supra*). Expression of low levels of TPP1 have been shown to dramatically attenuate disease in a CLN2-mutant mouse study, where just 6% of normal TPP1 activity in the brain increased the lifespan to almost that of a wild-type mouse (Sleat 2008, *supra*). Taken together, these nonclinical and clinical data indicate that cerliponase alfa delivered ICV at 300 mg QOW provides sufficient TPP1 exposure to the CNS for a meaningful therapeutic benefit. This is the first characterization of clinical CSF and plasma pharmacokinetics of an ICV administered protein.

## Claims

1. A composition comprising a formulation comprising recombinant human tripeptidyl peptidase-1 (rhTPP1) for use in a method of treating Neuronal Ceroid Lipofuscinosis (CLN2) disease in a subject less than 3 years old, wherein a dosage of about 300 mg or less is administered intracerebroventricularly, intrathecally, or intraocularly to the subject once every 2 weeks.

2. A composition comprising recombinant human tripeptidyl peptidase-1 (rhTPP1) for use in a method of delaying the onset of Neuronal Ceroid Lipofuscinosis (CLN2) disease, or a symptom thereof, in a subject less than 3 years old, wherein a dosage of about 300 mg or less is administered intracerebroventricularly, intrathecally, or intraocularly to the subject once every 2 weeks.

3. The composition for use according to any one of the preceding claims, wherein the formulation is administered by infusion at a rate of about 2.5 mL per hour.

4. The composition for use according to any one of the preceding claims, wherein the subject is greater than or about 2 years old, optionally wherein a dosage of about 300 mg rhTPP1 is administered to the subject.

5. The composition for use according to any one of claims 1 to 3, wherein the subject is greater than or about 1 year old and less than 2 years old.

6. The composition for use according to claim 5 wherein a dosage of about 200 mg rhTPP1 is administered to the subject.

7. The composition for use according to claim 6, wherein each of the 1^{st}, 2nd, 3^{rd} and 4^{th} dosages administered to the subject is about 200 mg rhTPP1 and each of the 5^{th} and subsequent dosages administered to the subject is greater than about 200 mg rhTPP1.

8. The composition for use according to claim 7, wherein each of the 5^{th} and subsequent dosages administered to the subject is about 300 mg rhTPP1.

9. The composition for use according to any one of claims 1 to 3, wherein the subject is greater than or about 6 months old and less than 1 year old, optionally wherein a dosage of about 150 mg rhTPP1 is administered to the subject.

10. The composition for use according to any one of claims 1 to 3, wherein the subject is less than 6 months old, optionally wherein a dosage of about 100 mg rhTPP1 is administered to the subject.

11. The composition for use according to any one of the preceding claims, wherein
i) the subject is a sibling of an individual diagnosed with CLN2
ii) the subject has a total score on the motor and language subscales of about 3 to about 6 points, and/or
iii) the subject has no prior treatment with a stem cell therapy, gene therapy, or enzyme supplementation therapy.

12. The composition for use according to any one of the preceding claims, comprising administering to the subject an antihistamine with or without an antipyretic before administration of the rhTPP1, optionally, about 30 to about 60 minutes before administration of the rhTPP1.

13. The composition for use according to any one of the preceding claims, wherein the formulation comprises the rhTPP1 and at least one pharmaceutically acceptably carrier, diluent or excipient.

14. The composition for use according to claim 13, wherein the formulation comprises disodium hydrogen phosphate pentahydrate, monosodium phosphate monohydrate, sodium chloride, potassium chloride, magnesium chloride, calcium chloride hydrate, water for injection, or a combination thereof.

15. The composition for use according to any one of the preceding claims, comprising administering to the subject a flush solution after administering the formulation, optionally wherein the flush solution comprises disodium hydrogen phosphate pentahydrate, monosodium phosphate monohydrate, sodium chloride, potassium chloride, magnesium chloride, calcium chloride hydrate, water for injection, or a combination thereof.

## Patentansprüche

1. Zusammensetzung, umfassend eine Formulierung, die rekombinante humane Tripeptidylpeptidase-1 (rhTPP1) umfasst, zur Verwendung in einem Verfahren zur Behandlung von neuronaler Ceroid-Lipofuszinose (CLN2)-Krankheit bei einem Subjekt, das weniger als 3 Jahre alt ist, wobei eine Dosis von etwa 300 mg oder weniger dem Subjekt einmal alle 2 Wochen intrazerebroventrikulär, intrathekal oder intraokular verabreicht wird.

2. Zusammensetzung, die rekombinante humane Tripeptidylpeptidase-1 (rhTPP1) umfasst, zur Verwendung in einem Verfahren zur Verzögerung des Ausbruchs von neuronaler Ceroid-Lipofuszinose (CLN2)-Krankheit oder eines Symptoms davon bei einem Subjekt, das weniger als 3 Jahre alt ist, wobei eine Dosis von etwa 300 mg oder weniger dem Subjekt einmal alle 2 Wochen intrazerebroventrikulär, intrathekal oder intraokular verabreicht wird.

3. Zusammensetzung zur Verwendung nach einem der vorhergehenden Ansprüche, wobei die Formulierung durch Infusion bei einer Geschwindigkeit von etwa 2,5 ml pro Stunde verabreicht wird.

4. Zusammensetzung zur Verwendung nach einem der vorhergehenden Ansprüche, wobei das Subjekt mehr als oder etwa 2 Jahre alt ist, wobei optional dem Subjekt eine Dosis von etwa 300 mg rhTPP1 verabreicht wird.

5. Zusammensetzung zur Verwendung nach einem der Ansprüche 1 bis 3, wobei das Subjekt mehr als oder etwa 1 Jahr alt und weniger als 2 Jahre alt ist.

6. Zusammensetzung zur Verwendung nach Anspruch 5, wobei dem Subjekt eine Dosis von etwa 200 mg rhTPP1 verabreicht wird.

7. Zusammensetzung zur Verwendung nach Anspruch 6, wobei jede der dem Subjekt verabreichten 1., 2., 3. und 4. Dosisgaben etwa 200 mg rhTPP1 beträgt und jede der 5. und nachfolgenden dem Subjekt verabreichten Dosisgaben mehr als etwa 200 mg rhTPP1 beträgt.

8. Zusammensetzung zur Verwendung nach Anspruch 7, wobei jede der 5. und nachfolgenden dem Subjekt verabreichten Dosisgaben etwa 300 mg rhTPP1 beträgt.

9. Zusammensetzung zur Verwendung nach einem der Ansprüche 1 bis 3, wobei das Subjekt mehr als oder etwa 6 Monate und weniger als 1 Jahr alt ist, wobei optional dem Subjekt eine Dosis von etwa 150 mg rhTPP1 verabreicht wird.

10. Zusammensetzung zur Verwendung nach einem der Ansprüche 1 bis 3, wobei das Subjekt weniger als 6 Monate alt ist, wobei optional dem Subjekt eine Dosis von etwa 100 mg rhTPP1 verabreicht wird.

11. Zusammensetzung zur Verwendung nach einem der vorhergehenden Ansprüche, wobei
i) das Subjekt ein Geschwister eines Individuums ist, bei dem CLN2 diagnostiziert wurde,
ii) das Subjekt auf den Motorik- und Sprachvermögen-Subskalen eine Gesamtpunktzahl von etwa 3 bis etwa 6 Punkten aufweist, und/oder
iii) das Subjekt keine vorherige Behandlung mit einer Stammzelltherapie, Gentherapie oder Enzymergänzungstherapie erhalten hat.

12. Zusammensetzung zur Verwendung nach einem der vorhergehenden Ansprüche, umfassend die Verabreichung eines Antihistaminikums mit oder ohne einem Antipyretikum an das Subjekt vor der Verabreichung des rhTPP1, optional etwa 30 bis etwa 60 Minuten vor der Verabreichung des rhTPP1.

13. Zusammensetzung zur Verwendung nach einem der vorhergehenden Ansprüche, wobei die Formulierung rhTPP1 und mindestens einen pharmazeutisch akzeptablen Träger, Verdünner oder Exzipienten umfasst.

14. Zusammensetzung zur Verwendung nach Anspruch 13, wobei die Formulierung Dinatriumhydrogenphosphat-Pentahydrat, Mononatriumphosphat-Monohydrat, Natriumchlorid, Kaliumchlorid, Magnesiumchlorid, Calciumchlorid-Hydrat, Wasser für Injektionszwecke oder eine Kombination davon umfasst.

15. Zusammensetzung zur Verwendung nach einem der vorhergehenden Ansprüche, umfassend die Verabreichung einer Spüllösung an das Subjekt nach der Verabreichung der Formulierung, wobei optional die Spüllösung Dinatriumhydrogenphosphat-Pentahydrat, Mononatriumphosphat-Monohydrat, Natriumchlorid, Kaliumchlorid, Magnesiumchlorid, Calciumchlorid-Hydrat, Wasser für Injektionszwecke oder eine Kombination davon umfasst.

## Revendications

1. Composition comprenant une formulation comprenant une tripeptidyl-peptidase-1 humaine (rhTPP1) recombinante pour une utilisation dans un procédé de traitement de la maladie lipofuscinose céroïde neuronale (CLN2) chez un sujet âgé de moins de 3 ans, un dosage d'environ 300 mg ou moins étant administré de manière intracérébroventriculaire, de manière intrathécale ou de manière intraoculaire au sujet une fois toutes les 2 semaines.

2. Composition comprenant une tripeptidyl-peptidase-1 humaine (rhTPP1) recombinante pour une utilisation dans un procédé de retardement de l'apparition de la maladie lipofuscinose céroïde neuronale (CLN2), ou d'un symptôme correspondant, chez un sujet âgé de moins de 3 ans, un dosage d'environ 300 mg ou moins étant administré de manière intracérébroventriculaire, de manière intrathécale ou de manière intraoculaire au sujet une fois toutes les 2 semaines.

3. Composition pour une utilisation selon l'une quelconque des revendications précédentes, la formulation étant administrée par perfusion à une vitesse d'environ 2,5 ml par heure.

4. Composition pour une utilisation selon l'une quelconque des revendications précédentes, le sujet étant âgé de plus de ou d'environ 2 ans, éventuellement un dosage d'environ 300 mg de rhTPP1 étant administré au sujet.

5. Composition pour une utilisation selon l'une quelconque des revendications 1 à 3, le sujet étant âgé de plus de ou d'environ 1 an et âgé de moins de 2 ans.

6. Composition pour une utilisation selon la revendication 5, un dosage d'environ 200 mg de rhTPP1 étant administré au sujet.

7. Composition pour une utilisation selon la revendication 6, chacun des 1^{er}, 2^{e}, 3^{e} et 4^{e} dosages administrés au sujet étant d'environ 200 mg de rhTPP1 et chacun du 5^{e} dosage et des dosages subséquents administrés au sujet étant supérieurs à environ 200 mg de rhTPP1.

8. Composition pour une utilisation selon la revendication 7, chacun du 5^{e} dosage et des dosages subséquents administrés au sujet étant d'environ 300 mg de rhTPP1.

9. Composition pour une utilisation selon l'une quelconque des revendications 1 à 3, le sujet étant âgé de plus de ou d'environ 6 mois et âgé de moins d'1 an, un dosage d'environ 150 mg de rhTPP1 étant administré au sujet.

10. Composition pour une utilisation selon l'une quelconque des revendications 1 à 3, le sujet étant âgé de moins de 6 mois, éventuellement un dosage d'environ 100 mg de rhTPP1 étant administré au sujet.

11. Composition pour une utilisation selon l'une quelconque des revendications précédentes,
i) le sujet étant un frère ou une soeur d'un individu ayant un diagnostic de CLN2
ii) le sujet ayant un score total sur les sous-échelles motrices et du langage d'environ 3 à environ 6 points, et/ou
iii) le sujet n'ayant pas de traitement précédent avec une thérapie à cellules souches, une thérapie génique ou une thérapie de supplémentation enzymatique.

12. Composition pour une utilisation selon l'une quelconque des revendications précédentes, comprenant une administration au sujet d'un antihistaminique avec ou sans antipyrétique avant administration du rhTPP1, éventuellement, environ 30 à environ 60 minutes avant administration du rhTPP1.

13. Composition pour une utilisation selon l'une quelconque des revendications précédentes, la formulation comprenant le rhTPP1 et au moins un support, diluant ou excipient pharmaceutiquement acceptable.

14. Composition pour une utilisation selon la revendication 13, la formulation comprenant du pentahydrate d'hydrogénophosphate disodique, du monohydrate de phosphate monosodique, du chlorure de sodium, du chlorure de potassium, du chlorure de magnésium, de l'hydrate de chlorure de calcium, de l'eau pour injection ou une combinaison correspondante.

15. Composition pour une utilisation selon l'une quelconque des revendications précédentes, comprenant une administration au sujet d'une solution de rinçage après administration de la formulation, éventuellement, la solution de rinçage comprenant du pentahydrate d'hydrogénophosphate disodique, du monohydrate de phosphate monosodique, du chlorure de sodium, du chlorure de potassium, du chlorure de magnésium, de l'hydrate de chlorure de calcium, de l'eau pour injection ou une combinaison correspondante.
